# EUROPEAN PATENT SPECIFICATION

(11) **EP 3 277 805 B1**
(45) Date of publication and mention of the grant of the patent: **07.05.2025**
(21) Application number: 16715977.1
(22) Date of filing: 31.03.2016
(51) Int. Cl.: C12N 9/22, C12N 15/62, C12N 9/12, C12N 15/90

(54) **CAS 9 RETROVIRAL INTEGRASE SYSTEMS FOR TARGETED INCORPORATION OF A DNA SEQUENCE INTO A GENOME OF A CELL**
CAS-9-RETROVIRALE INTEGRASESYSTEME ZUM GEZIELTEN EINSCHLUSS EINER DNA-SEQUENZ IN EIN GENOM EINER ZELLE
SYSTÈME CAS 9-INTÉGRASE RÉTROVIRALE POUR L'INCORPORATION CIBLÉE D'UNE SÉQUENCE D'ADN DANS UN GÉNOME D'UNE CELLULE

(30) Priority: 31.03.2015 US 201562140454 P; 27.08.2015 US 201562210451 P; 12.10.2015 US 201562240359 P
(43) Date of publication of application: 07.02.2018
(73) Proprietor: SOHM, Inc., Chino Hills, CA 91709 (US)
(72) Inventor: SHEIKH, Ferrukh, Westlake Village, CA 91362 (US); KAWAMURA, Tetsuya, San Diego, CA 92122 (US); MO, Gloria, San Diego, CA 92122 (US)
(74) Representative: J A Kemp LLP
(86) International application number: PCT/US2016/025426
(87) International publication number: WO 2016/161207

(56) References cited:
- US-A1- 2006 252 140
- US-A1- 2014 193 915
- US-A1- 2015 071 898
- J. A. DOUDNA ET AL: "The new frontier of genome engineering with CRISPR-Cas9", SCIENCE, vol. 346, no. 6213, 28 November 2014 (2014-11-28), pages 1258096 - 1258096, XP055162699, ISSN: 0036-8075, DOI: 10.1126/science.1258096
- BERND ZETSCHE ET AL: "Cpf1 Is a Single RNA-Guided Endonuclease of a Class 2 CRISPR-Cas System", CELL, vol. 163, no. 3, 22 October 2015 (2015-10-22), Amsterdam NL, pages 1 - 26, XP055553375, ISSN: 0092-8674, DOI: 10.1016/j.cell.2015.09.038
- KAI LI ET AL: "Optimization of Genome Engineering Approaches with the CRISPR/Cas9 System", PLOS ONE, vol. 9, no. 8, 28 August 2014 (2014-08-28), pages e105779, XP055243646, DOI: 10.1371/journal.pone.0105779
- TAN WENJIE ET AL: "Human immunodeficiency virus type 1 incorporated with fusion proteins consisting of integrase and the designed polydactyl zinc finger protein E2C can bias integration of viral DNA into a predetermined chromosomal region in human cells.", February 2006, JOURNAL OF VIROLOGY FEB 2006, VOL. 80, NR. 4, PAGE(S) 1939 - 1948, ISSN: 0022-538X
- RADOMSKA-LESNIEWSKA DOROTA ET AL: "DNA and RNA oligonucleotides as a new class of drugs", 2009, CENTRAL EUROPEAN JOURNAL OF IMMUNOLOGY, VOL. 34, NR. 4, PAGE(S) 280-283, ISSN: 1426-3912(print)
- DE SOULTRAIT V R ET AL: "DNA aptamers derived from HIV-1 RNase H inhibitors are strong anti-integrase agents.", JOURNAL OF MOLECULAR BIOLOGY, vol. 324, no. 2, 22 November 2002 (2002-11-22), pages 195 - 203, ISSN: 0022-2836
- MOUSCADET JEAN-FRANCOIS ET AL: "Triplex-mediated inhibition of HIV DNA integration in vitro", JOURNAL OF BIOLOGICAL CHEMISTRY, vol. 269, no. 34, 1994, pages 21635 - 21638, ISSN: 0021-9258
- SNASEL JAN ET AL: "Inhibition of HIV-1 integrase by modified oligonucleotides derived from U5' LTR", EUROPEAN JOURNAL OF BIOCHEMISTRY, vol. 268, no. 4, February 2001 (2001-02-01), pages 980 - 986, ISSN: 0014-2956
- BUSHMAN F D ET AL: "TETHERING HUMAN IMMUNODEFICIENCY VIRUS TYPE 1 PREINTEGRATION COMPLEXES TO TARGET DNA PROMOTES INTEGRATION AT NEARBY SITES", JOURNAL OF VIROLOGY, THE AMERICAN SOCIETY FOR MICROBIOLOGY, US, vol. 71, no. 1, 1 January 1997 (1997-01-01), pages 458 - 464, XP002914950, ISSN: 0022-538X
- SU K ET AL: "Site-specific integration of retroviral DNA in human cells using fusion proteins consisting of human immunodeficiency virus type 1 integrase and the designed polydactyl zinc-finger protein E2C", METHODS, ACADEMIC PRESS, US, vol. 47, no. 4, 1 April 2009 (2009-04-01), pages 269 - 276, XP026045893, ISSN: 1046-2023, [retrieved on 20090130], DOI: 10.1016/J.YMETH.2009.01.001
- CHARLES A. GERSBACH ET AL: "Synthetic Zinc Finger Proteins: The Advent of Targeted Gene Regulation and Genome Modification Technologies", ACCOUNTS OF CHEMICAL RESEARCH., vol. 47, no. 8, 30 May 2014 (2014-05-30), US, pages 2309 - 2318, XP055284026, ISSN: 0001-4842, DOI: 10.1021/ar500039w
- ZHANG WENLI ET AL: "PhiC31 integrase fused to designer DNA binding domains for sequence-specific genome engineering", HUMAN GENE THERAPY, vol. 25, no. 11, November 2014 (2014-11-01), & ESGCT AND NVGCT COLLABORATIVE CONGRESS; THE HAGUE, NETHERLANDS; OCTOBER 23 -26, 2014, pages A70 - A71, XP002759254

## Description

### CROSS REFERENCE TO RELATED APPLICATION

This application claims the benefit of United States Provisional Application No. 62,140,454, filed March 31, 2015, United States Provisional Application No. 62,210,451, filed August 27, 2015, and United States Provisional Application No. 62,240,359 filed October 12, 2015.

### INTRODUCTION

The instant disclosure relates to the use of engineered proteins with DNA binding proteins exhibiting genome specificity and in particular a catalytically inactive Cas9 (CRISPR (clustered regularly interspaced short palindromic repeats) protein) attached by a linker with a viral integrase (e.g. HIV or MMTV integrases) in order to deliver a DNA sequence of interest (or gene of interest) to a targeted site in a genome of a cell or organism. The use of a Cas9 that is inactive for its function in cutting DNA will allow us to use the Cas9 proteins ability to target DNA by the use of RNA guides (gRNA) without causing DNA breaks as intended in other systems for homologous recombination. The use of zinc finger proteins or TALE (engineered proteins that bind specific sequences of DNA) attached to the viral integrase is also disclosed. The system may be used for laboratory and therapeutic purposes. For example, donor DNA containing the gene(s) of interested can be easily introduced into host genome without the potential of off target cuts through conventional methods. Donor DNA can be engineered to facilitate "knock out" strategies as well. A new strategy for improving the specificity of Cas9 targeting is also discussed. This strategy uses surface bound dCas9 (Cas9 that is inactive for its DNA cutting ability) along with guide RNAs and genomic DNA in an assay to find which guide RNAs provide specific targeting of the Cas9. This will be especially important in in vivo applications of CRISPR/Cas9 and overcome limitations of the current in silico prediction models, although it may also be used in conjunction with in silico prediction models to make an educated determination of which gRNAs will be used in the assay.

### BACKGROUND

Current advances in genome sequencing techniques and analysis methods have significantly accelerated the ability to catalog and map genetic/genomic factors that are associated with a diverse range of biological functions and diseases. Precise genome targeting technologies are needed to enable systematic reverse engineering of causal genetic variations by allowing selective perturbation of individual genetic elements, as well as to advance synthetic biology, biotechnological, and medical applications. Genome-editing techniques such as designer zinc fingers, transcription activator-like effectors (TALEs), CRISPR/Cas9 or meganucleases are available for producing targeted genome perturbations, there remains a need for new genome engineering technologies that will allow the incorporation of DNA sequences (including full gene sequences) into a specific location in a given genome. This will allow for the production of cell lines or transgenic organisms that express an engineered gene or for the replacement of dysfunctional genes in a subject in need thereof.

Integrases are viral proteins that allow for the insertion of viral nucleic acids into a host genome (mammalian, human, mouse, rat, monkey, frog, fish, plant (including crop plants and experimental plants like Arabidopsis), laboratory or biomedical cell lines or primary cell cultures, C. elegans, fly (Drosophila), etc.). Integrases use DNA binding proteins of the host to bring the integrase in association with the host genome in order to incorporate the viral nucleic acid sequence into the host genome. Integrases are found in a retrovirus such as HIV (human immunodeficiency virus). Integrases depend on sequences on viral genes to insert their genome into host DNA. Leavitt et al (Journal of Biological Chemistry, 1993, volume 268, pages 2113-2119) examined the function of HIV1 integrase by using site directed mutagenesis and in vitro studies. Leavitt also indicates sequence of U5 and U3 HIV1 att sites that are important for the integration of HIV1 DNA (created after reverse transcription) into the host genome by the viral integrase. Su et al (Methods, 2009, volume 47, pages 269-276) disclose site-specific integration of retroviral DNA in human cells using fusion proteins consisting of HIV-1 integrase and the designed polydactyl zinc-finger protein E2C.

The instant disclosure improves current genome editing technology by allowing one to specifically insert desired nucleic acid (DNA) sequences into the genome at specified locations in the genome. The recombinant engineered integrase with DNA binding ability will bind a given DNA sequence in the genome and recognize a provided DNA sequence having integrase recognition domains (such as the HIV1 (or other retrovirus) att sites) and/or homology arms to insert the given nucleic acid sequence into the genome in a site specific manner. One aspect of the disclosure involves inserting DNA sequences of stop codons (UAA, UAG and/or UGA) just after the transcriptional start site of a gene. This will allow for effective inhibition of gene transcription in the genome of a cell.

### SUMMARY

The current disclosure links DNA targeting technologies, in particular CRISPR/Cas9, with retroviral integrases to form DNA targeting integrases. A gene of interest (GOI) may then be provided with the DNA targeting integrase so that it may be incorporated into the genome in a targeted manner. The GOI may be designed with homology arms to provide another level of specificity to its insertion in the genome.

The disclosure particularly relates to the use of a variant Cas9 that is inactive for cutting DNA for linking with a retroviral integrase.

Accordingly, the present invention provides a system for insertion of a donor nucleic acid into genomic DNA, comprising:
(a) a fusion protein comprising:
   i) a first protein comprising a catalytically inactive Cas9 protein;
   ii) a second protein comprising a retroviral integrase, and
   iii) a linker linking the first protein to the second protein;
(b) a guide RNA (gRNA); and
(c) a DNA vector comprising: the donor DNA, a first retroviral long terminal repeat (rLTR) and a second rLTR, wherein the donor DNA is located between the first rLTR and second rLTR. The . The fusion protein may be provided in an expression vector or as a purified protein. The donor DNA (a gene of interest (or DNA sequence of interest) may be provided with or without homology arms to be incorporated into the desired genome. The GOI or DNA sequence of interest may be modified to be recognized by the viral integrase as needed. Other reagents needed for polynucleotide transfection and/or introduction of protein into cells. Assaying for off-target integration of DNA sequences. In one aspect, using a marker sequence engineered into the inserted DNA sequence.

Disclosed herein are nucleic acid constructs comprising in operable linkage: a) a first polynucleotide sequence encoding an inactive Cas9: b) a second polynucleotide sequence encoding a retroviral integrase; and c) a third polynucleotide sequence encoding a nucleic acid linker; wherein the first polynucleotide sequence comprises a 5' and a 3' end and the second polynucleotide sequence comprises a 5' and a 3' end, and the 3' end of the first polynucleotide is connected to the 5' end of the second polynucleotide by the nucleic acid linker, and the first and second polynucleotide are able to be expressed as a fusion protein in a cell or an organism. In some embodiments, the first polynucleotide sequence comprises any one of SEQ ID NOS: 1, 3, 5, 7, 9, 11, 13, 27-46, 49, 56, or 68, or a sequence having at least 80%, at least 85%, at least 90%, at least 95%, or at least 99% identity thereto. In some embodiments, the inactive Cas9comprises any one of SEQ ID NOS: 2, 4, 6, 8, 10, 12, 14, 50, or 52, , or a sequence having at least 80%, at least 85%, at least 90%, at least 95%, or at least 99% identity thereto. In some embodiments, the second polynucleotide sequence comprises any one of SEQ ID NOS: 15, 17, 19, 21, 47, 55, 62, 70, or 79, or a sequence having at least 80%, at least 85%, at least 90%, at least 95%, or at least 99% identity thereto. In some embodiments, the integrase, comprises any one of SEQ ID NOS: 16, 18, 20, 22, 48, 63, 71, or 80, or a sequence having at least 80%, at least 85%, at least 90%, at least 95%, or at least 99% identity thereto. Also described herein are organisms comprising the nucleic acid construct. Also described herein is an organism comprising the fusion protein wherein the organism has a modified genome.

Disclosed herein are organisms comprising: a) a first polynucleotide sequence encoding an inactive Cas9: b) a second polynucleotide sequence encoding an integrase; and c) a third polynucleotide sequence encoding a nucleic acid linker; wherein the first polynucleotide sequence comprises a 5' and a 3' end and the second polynucleotide sequence comprises a 5' and a 3' end, and the 3' end of the first polynucleotide is connected to the 5' end of the second polynucleotide by the nucleic acid linker, and the first and second polynucleotide are able to be expressed as a fusion protein in a cell or an organism.

Also disclosed herein are fusion proteins, comprising: a) a first protein that is a catalytically inactive Cas9, wherein the first protein is targeted to a target DNA sequence; b) a second protein that is an integrase; and c) a linker linking the first protein to the second protein. In some embodiments, the second protein is an HIV1 integrase or a lentiviral integrase; the linker sequence is one or more amino acids in length; and the first protein is a catalytically inactive Cas9. Also disclosed are instances wherein the linker sequence is 4-8 amino acids in length; the first protein is a TALE protein; or the first protein is a Zinc finger protein. Also disclosed are instances wherein the fusion protein comprises a TALE or a Zinc finger protein, the target DNA sequence is about 16 to about 24 base pairs in length. In some embodiments, the first protein is catalytically inactive Cas9, wherein one or more guide RNAs are used for targeting of a target DNA sequence of from about 16 to about 24 base pairs.

Also provided herein is an in vitro method of inserting a DNA sequence into genomic DNA, comprising:
a) identifying a target sequence in the genomic DNA;
b) designing a fusion protein and guide RNA (gRNA) of the present invention to bind to the target sequence in the genomic DNA;
c) designing a DNA sequence of interest to incorporate into the genomic DNA; and
d) providing the fusion protein, the gRNA, and the DNA sequence of interest to a cell by techniques that allow for entry of the fusion protein, gRNA and DNA sequence of interest into the cell; wherein the DNA sequence of interest becomes integrated at the target sequence in the genomic DNA.

Also disclosed herein are nucleotide vectors, comprising: a) a first coding sequence for a first protein that is a catalytically inactive Cas9 protein; b) a second coding sequence for a second protein that is an retroviral integrase; c) a DNA sequence between the first and second coding sequences that forms an amino acid linker between the first and second proteins; d) optionally an expressed DNA sequence of interest surrounded by att sites recognized by an integrase, and optionally one or more guide RNAs, wherein the first protein is targeted to a determined DNA sequence, and wherein the first protein is linked to the second protein by the amino acid linker sequence.

Also provided is an in vitro method of inhibiting gene transcription in a cell, comprising:
a) identifying an ATG start codon in a gene;
b) designing a fusion protein system with a fusion protein as defined herein and a guide RNA (gRNA) to bind to a target sequence immediately after the ATG start codon of the gene;
c) designing a DNA sequence of interest that is one or more consecutive stop codons; and
d) providing the fusion protein, the gRNA, and the DNA sequence of interest to a cell by techniques that allow for entry of the fusion protein, gRNA and DNA sequence of interest into the cell; wherein the DNA sequence of interest becomes integrated at the target sequence in the genomic DNA; and wherein transcription of the gene is inhibited.

In one embodiment, the vector further comprising a reverse transcriptase gene to be expressed in a cell.

Also disclosed herein are compositions, comprising a purified protein of a DNA binding protein/integrase fusion and an RNA from about 15 to about 100 base pairs in length, wherein the DNA binding protein is a catalytically inactive Cas9 engineered to a targeted DNA sequence in a genome, and wherein the integrase is a HIV integrase, lentiviral integrase, adenoviral integrase, a retroviral integrase, or a MMTV integrase.

### BRIEF DESCRIPTION OF THE DRAWINGS

These and other features, aspects, and advantages of the present disclosure will become better understood with regard to the following description, appended claims and accompanying figures where:

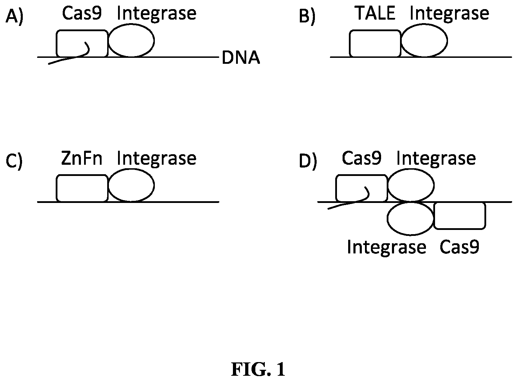

**FIG. 1** shows a) an exemplary catalytically inactive Cas9/HIV1 integrase fusion protein, b) an exemplary TALE/HIV1 integrase fusion protein, c) an exemplary zinc finger protein/HIV1 integrase fusion protein, and d) an exemplary Cas9/HIV1 integrase fusion protein designed to opposite sides of the DNA at the targeted site. Each of the fusion proteins binds to a specific target sequence of DNA. "ZnFn" is a Zinc finger protein. "Integrase" represents one integrase unit or two integrase units linked, for example, by a short amino acid linker. Cas9 is catalytically inactive.
**FIG. 2** shows a DNA plasmid system comprising, a vector comprising a catalytically inactive Cas9/integrase fusion protein, a vector comprising a DNA sequence of interest, and a vector comprising a reverse transcriptase. A guide RNA (gRNA) or RNAs may be provided separately. Another vector can be used to express a gRNA. "1 or 2" refers to one integrase or two integrases linked by, for example, an amino acid linker.
**FIG. 3** shows an exemplary DNA plasmid comprising a nucleotide sequence catalytically inactive Cas9/integrase fusion protein, guide RNAs, a DNA (gene) sequence of interest, and a reverse transcriptase. Viral att sites can be provided to the DNA sequence of interest, allowing for incorporation of the integrase into the cell's genomic DNA. A guide RNA (gRNA) or RNAs may be provided separately. Another vector can be used to express a gRNA. "1 or 2" refers to one integrase or two integrases linked by, for example, an amino acid linker.

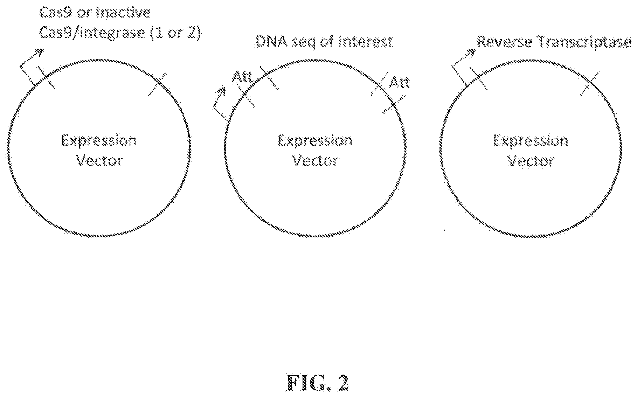

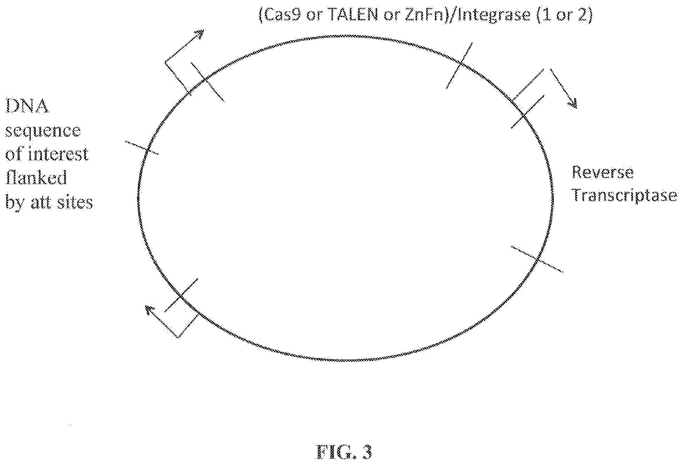

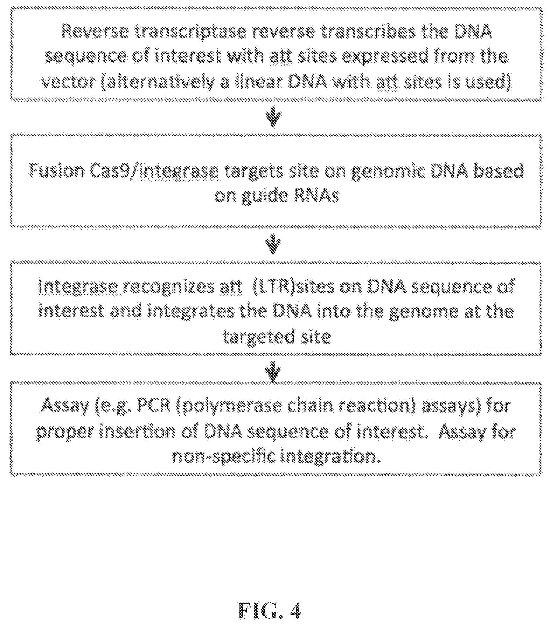

**FIG. 4** shows a flow diagram. One exemplary method of employing the vectors shown in **FIG. 2** and **FIG. 3** is shown in **FIG. 4****,** and is as follows: 1) reverse transcriptase reverse transcribes the DNA sequence of interest with att sites expressed from the vector (alternatively a linear DNA with att sites is used), 2) fusion Cas9/integrase targets site on genomic DNA based on guide RNAs, 3) integrase recognizes att (LTR) sites on DNA sequence of interest and integrates the DNA into the genome at the targeted site, and 4) an assay (e.g. PCR (polymerase chain reaction) is conducted to check for proper insertion of DNA sequence of interest. An assay can be conducted to check for non-specific integration.
**FIG. 5** shows Abbie1 Gene Editing Targeting Exon 2 of Nrf2 Using Guide NrF2-sgRNA2 and sgRNA3.
**FIG. 6** shows theoretical data generated by Abbie1 gene editing.
**FIG. 7** shows Abbie1 Gene Editing Targeting Exon 2 of Nrf2 Using Guide Nrf2-sgRNA 3.
**FIG. 8** shows Abbie1 Knock out of Nrf2 in pooled Hek293T Cells.
**FIG. 9** shows Abbie1 Knock out of Nrf2 in pooled Hek293T Cells.
**FIG. 10** shows Abbie1 Gene Editing Targeting CXCR4 Exon 2.

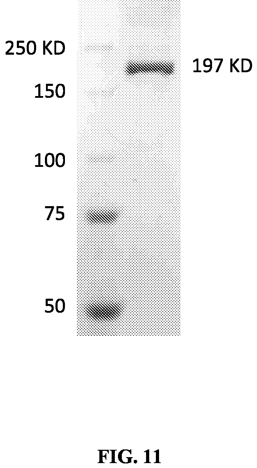

**FIG. 11** shows detection of ABBIE1 protein after isolation and purification from E coli. Coomassie stained gel.

### DETAILED DESCRIPTION

The following detailed description is provided to aid those skilled in the art in practicing the present disclosure.

As used in this disclosure and the appended claims, the singular forms "a", "an" and "the" include a plural reference unless the context clearly dictates otherwise. As used in this disclosure and the appended claims, the term "or" can be singular or inclusive. For example, A or B, can be A and B.

### ENDOGENOUS

An endogenous nucleic acid, nucleotide, polypeptide, or protein as described herein is defined in relationship to the host organism. An endogenous nucleic acid, nucleotide, polypeptide, or protein is one that naturally occurs in the host organism.

### EXOGENOUS

An exogenous nucleic acid, nucleotide, polypeptide, or protein as described herein is defined in relationship to the host organism. An exogenous nucleic acid, nucleotide, polypeptide, or protein is one that does not naturally occur in the host organism or is a different location in the host organism.

### KNOCKOUT

A gene is considered knocked out when an exogenous nucleic acid is transformed into a host organism (e.g. by random insertion or homologous recombination) resulting in the disruption (e.g. by deletion, insertion) of the gene.

Upon knocking out a gene, the activity of the corresponding protein can be decreased. For example, by at least 10%, by at least 20%, by at least 30%, by at least 40%, by at least 50%, by at least 60%, by at least 70%, by at least 80%, by at least 90%, or 100%, as compared to the activity of the same protein wherein the gene has not been knocked out.

Upon knockout out of a gene, the transcription of the gene can be decreased, as compared to a gene that has not been knocked out, by at least 20%, by at least 30%, by at least 40%, by at least 50%, by at least 60%, by at least 70%, by at least 80%, by at least 90%, or 100%.

### MODIFIED

A modified organism is an organism that is different than an unmodified organism. For example, a modified organism can comprise a fusion protein of the disclosure that results in a knockout of a targeted gene sequence. A modified organism can have a modified genome.

A modified nucleic acid sequence or amino acid sequence is different than the unmodified nucleic acid sequence or amino acid sequence. For example, a nucleic acid sequence can have one or more nucleic acids inserted, deleted, or added. For example, an amino acid sequence can have one or more amino acids inserted, deleted, or added.

### OPERABLY LINKED

In some embodiments, a vector comprises a polynucleotide operably linked to one or more control elements, such as a promoter and/or a transcription terminator. A nucleic acid sequence is operably linked when it is placed into a functional relationship with another nucleic acid sequence. For example, DNA for a presequence or secretory leader is operatively linked to DNA for a polypeptide if it is expressed as a preprotein which participates in the secretion of the polypeptide; a promoter is operably linked to a coding sequence if it affects the transcription of the sequence; or a ribosome binding site is operably linked to a coding sequence if it is positioned so as to facilitate translation. Operably linked sequences can be contiguous and, in the case of a secretory leader, contiguous and in reading phase.

### HOST CELL OR HOST ORGANISM

A host cell can contain a polynucleotide encoding a polypeptide of the present disclosure. In some embodiments, a host cell is part of a multicellular organism. In other embodiments, a host cell is cultured as a unicellular organism.

Host organisms can include any suitable host, for example, a microorganism. Microorganisms which are useful for the methods described herein include, for example, bacteria (e.g., *E. coli*), yeast (e.g., *Saccharomyces cerevisiae),* and plants. The organism can be prokaryotic or eukaryotic. The organism can be unicellular or multicellular.

The host cell can be prokaryotic. Suitable prokaryotic cells include, but are not limited to, any of a variety of laboratory strains of *Escherichia coli, Lactobacillus* sp., *Salmonella* sp., and *Shigella* sp. (for example, as described in Carrier et al. (1992) J. Immunol. 148:1176-1181; U.S. Pat. No. 6,447,784; and Sizemore et al. (1995) Science 270:299-302). Examples of *Salmonella* strains which can be employed in the present disclosure include, but are not limited to, *Salmonella typhi* and *S. typhimurium.* Suitable *Shigella* strains include, but are not limited to, *Shigella flexneri, Shigella sonnei,* and *Shigella disenteriae.* Typically, the laboratory strain is one that is non-pathogenic. Non-limiting examples of other suitable bacteria include, but are not limited to, *Pseudomonas pudita, Pseudomonas aeruginosa, Pseudomonas mevalonii, Rhodobacter sphaeroides, Rhodobacter capsulatus, Rhodospirillum rubrum,* and *Rhodococcus* sp.

In some embodiments, the host organism is eukaryotic. Suitable eukaryotic host cells include, but are not limited to, yeast cells, insect cells, plant cells, fungal cells, and algal cells.

### POLYNUCLEOTIDES AND POLYPEPTIDES [NUCLEIC ACIDS AND PROTEINS]

The proteins of the present disclosure can be made by any method known in the art. The protein may be synthesized using either solid-phase peptide synthesis or by classical solution peptide synthesis also known as liquid-phase peptide synthesis. Using Val-Pro-Pro, Enalapril and Lisinopril as starting templates, several series of peptide analogs such as X-Pro-Pro, X-Ala-Pro, and X-Lys-Pro, wherein X represents any amino acid residue, may be synthesized using solid-phase or liquid-phase peptide synthesis. Methods for carrying out liquid phase synthesis of libraries of peptides and oligonucleotides coupled to a soluble oligomeric support have also been described. Bayer, Ernst and Mutter, Manfred, Nature 237:512-513 (1972); Bayer, Ernst, et al., J. Am. Chem. Soc. 96:7333-7336 (1974); Bonora, Gian Maria, et al., Nucleic Acids Res. 18:3155-3159 (1990). Liquid phase synthetic methods have the advantage over solid phase synthetic methods in that liquid phase synthesis methods do not require a structure present on a first reactant which is suitable for attaching the reactant to the solid phase. Also, liquid phase synthesis methods do not require avoiding chemical conditions which may cleave the bond between the solid phase and the first reactant (or intermediate product). In addition, reactions in a homogeneous solution may give better yields and more complete reactions than those obtained in heterogeneous solid phase/liquid phase systems such as those present in solid phase synthesis.

In oligomer-supported liquid phase synthesis the growing product is attached to a large soluble polymeric group. The product from each step of the synthesis can then be separated from unreacted reactants based on the large difference in size between the relatively large polymer-attached product and the unreacted reactants. This permits reactions to take place in homogeneous solutions, and eliminates tedious purification steps associated with traditional liquid phase synthesis. Oligomer-supported liquid phase synthesis has also been adapted to automatic liquid phase synthesis of peptides. Bayer, Ernst, et al., Peptides: Chemistry, Structure, Biology, 426-432.

For solid-phase peptide synthesis, the procedure entails the sequential assembly of the appropriate amino acids into a peptide of a desired sequence while the end of the growing peptide is linked to an insoluble support. Usually, the carboxyl terminus of the peptide is linked to a polymer from which it can be liberated upon treatment with a cleavage reagent. In a common method, an amino acid is bound to a resin particle, and the peptide generated in a stepwise manner by successive additions of protected amino acids to produce a chain of amino acids. Modifications of the technique described by Merrifield are commonly used. See, e.g., Merrifield, J. Am. Chem. Soc. 96: 2989-93

(1964). In an automated solid-phase method, peptides are synthesized by loading the carboxy-terminal amino acid onto an organic linker (e.g., PAM, 4-oxymethylphenylacetamidomethyl), which is covalently attached to an insoluble polystyrene resin cross-linked with divinyl benzene. The terminal amine may be protected by blocking with t-butyloxycarbonyl. Hydroxyl- and carboxyl-groups are commonly protected by blocking with O-benzyl groups. Synthesis is accomplished in an automated peptide synthesizer, such as that available from Applied Biosystems (Foster City, Calif.). Following synthesis, the product may be removed from the resin. The blocking groups are removed by using hydrofluoric acid or trifluoromethyl sulfonic acid according to established methods. A routine synthesis may produce 0.5 mmole of peptide resin. Following cleavage and purification, a yield of approximately 60 to 70% is typically produced. Purification of the product peptides is accomplished by, for example, crystallizing the peptide from an organic solvent such as methyl-butyl ether, then dissolving in distilled water, and using dialysis (if the molecular weight of the subject peptide is greater than about 500 daltons) or reverse high pressure liquid chromatography (e.g., using a C¹⁸ column with 0.1% trifluoroacetic acid and acetonitrile as solvents) if the molecular weight of the peptide is less than 500 daltons. Purified peptide may be lyophilized and stored in a dry state until use. Analysis of the resulting peptides may be accomplished using the common methods of analytical high pressure liquid chromatography (HPLC) and electrospray mass spectrometry (ES-MS).

In other cases, a protein, for example, a protein is produced by recombinant methods. For production of any of the proteins described herein, host cells transformed with an expression vector containing the polynucleotide encoding such a protein can be used. The host cell can be a higher eukaryotic cell, such as a mammalian cell, or a lower eukaryotic cell such as a yeast, or the host can be a prokaryotic cell such as a bacterial cell. Introduction of the expression vector into the host cell can be accomplished by a variety of methods including calcium phosphate transfection, DEAE-dextran mediated transfection, polybrene, protoplast fusion, liposomes, direct microinjection into the nuclei, scrape loading, biolistic transformation and electroporation. Large scale production of proteins from recombinant organisms is a well established process practiced on a commercial scale and well within the capabilities of one skilled in the art.

### CODON OPTIMIZATION

One or more codons of an encoding polynucleotide can be "biased" or "optimized" to reflect the codon usage of the host organism. For example, one or more codons of an encoding polynucleotide can be "biased" or "optimized" to reflect chloroplast codon usage or nuclear codon usage. Most amino acids are encoded by two or more different (degenerate) codons, and it is well recognized that various organisms utilize certain codons in preference to others. "Biased" or codon "optimized" can be used interchangeably throughout the specification. Codon bias can be variously skewed in different plants, including, for example, in alga as compared to tobacco. Generally, the codon bias selected reflects codon usage of the plant (or organelle therein) which is being transformed with the nucleic acids of the present disclosure.

A polynucleotide that is biased for a particular codon usage can be synthesized de novo, or can be genetically modified using routine recombinant DNA techniques, for example, by a site directed mutagenesis method, to change one or more codons such that they are biased for chloroplast codon usage.

### PERCENT SEQUENCE IDENTITY

One example of an algorithm that is suitable for determining percent sequence identity or sequence similarity between nucleic acid or polypeptide sequences is the BLAST algorithm, which is described, e.g., in Altschul et al., J. Mol. Biol. 215:403-410 (1990). Software for performing BLAST analysis is publicly available through the National Center for Biotechnology Information. The BLAST algorithm parameters W, T, and X determine the sensitivity and speed of the alignment. The BLASTN program (for nucleotide sequences) uses as defaults a word length (W) of 11, an expectation (E) of 10, a cutoff of 100, M=5, N=4, and a comparison of both strands. For amino acid sequences, the BLASTP program uses as defaults a word length (W) of 3, an expectation (E) of 10, and the BLOSUM62 scoring matrix (as described, for example, in Henikoff & Henikoff (1989) Proc. Natl. Acad. Sci. USA, 89:10915). In addition to calculating percent sequence identity, the BLAST algorithm also can perform a statistical analysis of the similarity between two sequences (for example, as described in Karlin & Altschul, Proc. Nat'l. Acad. Sci. USA, 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, less than about 0.01, or less than about 0.001.

The instant disclosure comprises a system comprising: A) A viral integrase covalently linked to a catalytically inactive Cas9 protein that is, for example, inactive for DNA cutting ability. Also disclosed is the viral integrase (or a bacterial or phage recombinase) covalently linked to a TALE protein or zinc finger proteins where these proteins are designed to target a specific sequence of DNA in a genome.

This may be provided in an expression vector or as a purified protein. B) A gene of interest (or DNA sequence of interest) with or without homology arms to be incorporated into the desired genome. The GOI or DNA sequence of interest may be modified to be recognized by the viral integrase as needed. For example, the viral att sites can be added to the ends of the DNA sequence. C) Other reagents needed for polynucleotide transfection and/or introduction of protein into cells.

### NUCLEIC ACID

The terms "polynucleotide", "nucleotide", "nucleotide sequence", "nucleic acid" and "oligonucleotide" are used interchangeably in this disclosure. They refer to a polymeric form of nucleotides of any length, either deoxyribonucleotides or ribonucleotides, or analogs thereof. Polynucleotides may have any three dimensional structure, and may perform any function, known or unknown. The following are non limiting examples of polynucleotides: coding or non-coding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non nucleotide components. A polynucleotide may be further modified after polymerization, such as by conjugation with a labeling component.

### GUIDE RNA

In aspects of the disclosure the terms "chimeric RNA", "chimeric guide RNA", "guide RNA", "single guide RNA" and "synthetic guide RNA" are used interchangeably and refer to the polynucleotide sequence comprising the guide sequence, the tracr sequence and the tracr mate sequence. The term "guide sequence" refers to the about 20 bp (12-30 bp) sequence within the guide RNA that specifies the target site and may be used interchangeably with the terms "guide" or "spacer". The term "tracr mate sequence" may also be used interchangeably with the term "direct repeat(s)".

### WILD TYPE

As used herein the term "wild type" is a term of the art understood by skilled persons and means the typical form of an organism, strain, gene or characteristic as it occurs in nature as distinguished from mutant or variant forms.

### VARIANT

As used herein the terms "variant" or "mutant" should be taken to mean the exhibition of qualities that have a pattern that deviates from what occurs in nature. In relation to the genes, these terms indicate a number of changes in a gene that make it different from the wild-type gene including single nucleotide polymorphisms (SNPs), insertions, deletions, gene shifts among others.

### ENGINEERED

The terms "non-naturally occurring" or "engineered" are used interchangeably and indicate the involvement of man-made technology. The terms, when referring to nucleic acid molecules or polypeptides mean that the nucleic acid molecule or the polypeptide is at least substantially free from at least one other component with which they are naturally associated in nature and as found in nature.

### COMPLEMENTARY

"Complementarity" refers to the ability of a nucleic acid to form hydrogen bond(s) with another nucleic acid sequence by either traditional Watson-Crick or other non-traditional types. A percent complementarity indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 70%, 75%, 80%, 85%, 90%, 95%. 97%, 98%, 99%, or 100%, or percentages in between over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

### AMINO ACIDS

Full Name, Three-Letter Code, One-Letter Code
Aspartic Acid Asp D
Glutamic Acid Glu E
Lysine Lys K
Arginine Arg R
Histidine His H
Tyrosine Tyr Y
Cysteine Cys C
Asparagine Asn N
Glutamine Gln Q
Serine Ser S
Threonine Thr T
Glycine Gly G
Alanine Ala A
Valine Val V
Leucine Leu L
Isoleucine Ile I
Methionine Met M
Proline Pro P
Phenylalanine Phe F
Tryptophan Trp W

The expression "amino acid" as used herein is meant to include both natural and synthetic amino acids, and both D and L amino acids. "Standard amino acid" means any of the twenty standard L-amino acids commonly found in naturally occurring proteins/peptides. "Non-standard amino acid residue" means any amino acid, other than the standard amino acids, regardless of whether it is prepared synthetically or derived from a natural source. As used herein, "synthetic amino acid" encompasses chemically modified amino acids, including but not limited to salts, amino acid derivatives (such as amides), and substitutions. Amino acids contained within the peptides of the present disclosure, and particularly at the carboxy- or amino-terminus, can be modified by methylation, amidation, acetylation or substitution with other chemical groups which can change the peptide's circulating half-life without adversely affecting their activity. Additionally, a disulfide link may be present or absent in the peptides.

Amino acids may be classified into seven groups on the basis of the side chain R: (1) aliphatic side chains; (2) side chains containing a hydroxyl (OH) group; (3) side chains containing sulfur atoms; (4) side chains containing an acidic or amide group; (5) side chains containing a basic group; (6) side chains containing an aromatic ring; and (7) proline, an imino acid in which the side chain is fused to the amino group.

As used herein, the term "conservative amino acid substitution" is defined herein as exchanges within one of the following five groups:
I. Small aliphatic, nonpolar or slightly polar residues:
   Ala, Ser, Thr, Pro, Gly;
II. Polar, negatively charged residues and their amides:
   Asp, Asn, Glu, Gin;
III. Polar, positively charged residues:
   His, Arg, Lys;
IV. Large, aliphatic, nonpolar residues:
   Met Leu, He, Val, Cys (Ile; autocorrect is not literate)
V. Large, aromatic residues:
   Phe, Tyr, Tip (Trp, likewise)

The present disclosure utilizes, unless otherwise provided, conventional techniques of immunology, biochemistry, chemistry, molecular biology, microbiology, cell biology, genomics and recombinant DNA, which are within the skill of the art. See Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M. J. MacPherson, B. D. Hames and G. R. Taylor eds. (1995)), Harlow and Lane, eds. (1988) ANTIBODIES, A LABORATORY MANUAL, and ANIMAL CELL CULTURE (R. I. Freshney, ed. (1987)).

### VECTORS

Gene expression vectors (DNA-based or viral) will be used to express the fusion integrases in cells or tissues as well as to provide the DNA sequence (or gene) of interest with the appropriate sites needed for the integrase or recombinase to integrate that DNA (or gene) into the genome of the host species or cell. A number of gene expression vectors are known in the art. Vectors will be use for the gene of interest (or DNA sequence of interest). Vectors may be cut with a number of restriction enzymes known in the art.

### CRISPR/CAS9

CRISPR/Cas9 is described in US Patent 8697359, US 8889356 and Ran et al (Nature Protocols, 2013, volume 8, pages 2281-2308). Cas9 protein utilizes RNA guides in order to bind specific sequences of DNA in a genome. The RNA guides (guide RNAs) may be designed to be from 10 to 40, from 12 to 35, from 15 to 30, or for example, from 18 to 22, or 20 nucleotides in length. See Hsu et al, Nature Biotechnology, September 2013, volume 31, pages 827-832, which uses Cas9 from Streptococcus pyogenes. Another key Cas9 is from Staphylococcus Aureus (a smaller Cas9 than that of S pyogenes). The Cas9 protein utilizes guide RNAs to bind specific regions of a DNA sequence.

The present invention uses a catalytically inactive Cas9 protein as part of a fusion protein. A catalytically inactive form of Cas9 is described in Guilinger et al, Fusion of catalytically inactive Cas9 to FokI nuclease improves the specificity of genome modification, Nature Biotechnology, April 25, 2014, volume 32, pages 577-582. Guilinger et al attached the catalytically inactive Cas9 to a Fok1 enzyme to achieve greater specificity in making cuts in genomic DNA. This catalytically inactive Cas9 allows for Cas9 to use RNA guides for binding of genomic DNA, while not being able to cut the DNA.

Cas9 is also available in its natural wt form, and also a human optimized codon form for better expression of Cas9 constructs in cells. (see Mali et al, Science, 2013, volume 339, pages 823-826). Codon optimization of Cas9 may be conducted dependent on the species for its expression. Depending on whether one produces a protein form of the Integrase/Cas9 fusion protein (also known as ABBIE1) or a nucleotide expression vector form, the optimized or non-optimized (wt) form may be used.

RNA guides toward a specific DNA sequence can be designed by various computer-based tools.

### CRISPR/CPF1

Cpfl is another protein, which uses a guide RNA in order to bind a specific sequence in genomic DNA. Cpfl also cuts DNA making a staggered cut. Cpfl may be made to be catalytically inactive for cutting ability.

### OTHER CRISPR PROTEINS

These are proteins that utilize a guide RNA to target a specific DNA sequence and whether they have the ability to cut DNA or not. Some of these proteins may naturally have other enzymatic/catalytic functions.

### TALEN

Also disclosed are TALENs. Transcription Activator-Like Effector Nucleases (TALENs) are fusion proteins with restriction enzymes generated by fusing the TAL effector DNA binding domain to a DNA cleavage domain. These reagents enable efficient, programmable, and specific DNA cleavage and represent powerful tools for genome editing in situ. Transcription activator-like effectors (TALEs) can be quickly engineered to bind practically any DNA sequence. The term TALEN, as used herein, is broad and includes a monomeric TALEN that can cleave double stranded DNA without assistance from another TALEN. The term TALEN is also used to refer to one or both members of a pair of TALENs that are engineered to work together to cleave DNA at the same site. TALENs that work together may be referred to as a left-TALEN and a right-TALEN, which references the handedness of DNA. See US 8, 440,432.

TAL effectors are proteins secreted by Xanthomonas bacteria. The DNA binding domain contains a highly conserved 33-34 amino acid sequence with the exception of the 12th and 13th amino acids. These two locations are highly variable (Repeat Variable Diresidues (RVD)) and show a strong correlation with specific nucleotide recognition. This simple relationship between amino acid sequence and DNA recognition has allowed for the engineering of specific DNA binding domains by selecting a combination of repeat segments containing the appropriate RVDs.

The integrase can be used to construct hybrid integrase or recombinase that are active in a yeast or cell assay. These reagents are also active in plant cells and in animal cells. TALEN studies used the wild-type Fokl cleavage domain, but some subsequent TALEN studies also used Fokl cleavage domain variants with mutations designed to improve cleavage specificity and cleavage activity. Both the number of amino acid residues between the TALEN DNA binding domain and the integrase or recombinase domain and the number of bases between the two individual TALEN binding sites are parameters for achieving high levels of activity. The number of amino acid residues between the TALEN DNA binding domain and the integrase or recombinase domain may be modified by introduction of a spacer (distinct from the spacer sequence) between the plurality of TAL effector repeat sequences and the integrase or recombinase domain. The spacer sequence may be 6 to 102 or 9 to 30 nucleotides or 15 to 21 nucleotides. These spacers will usually not provide other activity to the hybrid protein besides providing a link between the DNA targeting protein (Cas9, TALE or zinc finger protein) and the integrase or recombinase. The amino acids for the spacers and for other uses in the instant disclosure are

The relationship between amino acid sequence and DNA recognition of the TALEN binding domain allows for designable proteins. In this case artificial gene synthesis is problematic because of improper annealing of the repetitive sequence found in the TALE binding domain. One solution to this is to use a publicly available software program named DNAWorks to find oligonucleotides suitable for assembly in a two step PCR; oligonucleotide assembly followed by whole gene amplification. A number of modular assembly methods for generating engineered TALE constructs have also been reported in the art.

Once the TALEN genes have been assembled together they are inserted into plasmids; the plasmids are then used to transfect the target cell where the gene products are expressed and enter the nucleus to access the genome. TALENs can be used to edit genomes by inducing double-strand breaks (DSB), which cells respond to with DNA repair, however, the instant disclosure seeks to use the power of viral integrases to insert DNA sequences of interest into targeted sites in the genome. See disclosure of WO 2014134412 and US Patent 8748134.

### ZINC FINGER PROTEINS

Also disclosed are Zinc finger proteins. Zinc finger proteins for binding DNA and their design are described in US 7928195, US 2009/0111188, and US 7951925. Zinc finger proteins utilize a number of linked zinc finger domains in a specified order to bind to a specific sequence of DNA. Zinc finger protein endonucleases have been well-established.

Zinc finger proteins (ZFPs) are proteins that can bind to DNA in a sequence-specific manner. Zinc fingers were first identified in the transcription factor TFIIIA from the oocytes of the African clawed toad, Xenopus laevis. A single zinc finger domain of this class of ZFPs is about 30 amino acids in length, and several structural studies have demonstrated that it contains a beta turn (containing two conserved cysteine residues) and an alpha helix (containing two conserved histidine residues), which are held in a particular conformation through coordination of a zinc atom by the two cysteines and the two histidines. This class of ZFPs is also known as C2H2 ZFPs. Additional classes of ZFPs have also been suggested. See, e.g., Jiang et al. (1996) J. Biol. Chem. 271:10723-10730 for a discussion of Cys-Cys-His-Cys (C3H) ZFPs. To date, over 10,000 zinc finger sequences have been identified in several thousand known or putative transcription factors. Zinc finger domains are involved not only in DNA recognition, but also in RNA binding and in protein-protein binding. Current estimates are that this class of molecules will constitute about 2% of all human genes.

Many zinc finger proteins have conserved cysteine and histidine residues that tetrahedrally-coordinate the single zinc atom in each finger domain. In particular, most ZFPs are characterized by finger components of the general sequence: -Cys-(X)2-4-Cys-(X)12-His-(X)3-5-His- (SEQ ID NO:49, in which X represents any amino acid (the C2H2 ZFPs). The zinc-coordinating sequences of this most widely represented class contain two cysteines and two histidines with particular spacings. The folded structure of each finger contains an antiparallel β-turn, a finger tip region and a short amphipathic α-helix. The metal coordinating ligands bind to the zinc ion and, in the case of zif268-type zinc fingers, the short amphipathic α-helix binds in the major groove of DNA. In addition, the structure of the zinc finger is stabilized by certain conserved hydrophobic amino acid residues (e.g., the residue directly preceding the first conserved Cys and the residue at position +4 of the helical segment of the finger) and by zinc coordination through the conserved cysteine and histidine residues.

### OTHER DNA BINDING PROTEINS THAT MAY BIND SPECIFIC TARGET SEQUENCES IN GENOMIC DNA

The proteins disclosed include those unrelated to the zinc finger proteins, TALEN and CRISPR proteins that may bind to specific sequences in genomic DNA of various organisms. These may include transcription factors, transcriptional repressors, meganucleases, endonuclease DNA binding domains and others.

### INTEGRASES

Integrases and endonuclease fusion proteins thereof are described in US 2009/0011509. Integrases introduced are lentiviral integrase and HIV1 (human immunodeficiency virus 1) integrase. The instant disclosure fuses a catalytically inactive Cas9 to an integrase via a linker to target the integrase to a specific region of DNA in the genome that is chosen by the user.

The HIV-1 integrase, like other retroviral integrases, is able to recognize special features at the ends of the viral DNA located in the U3 and U5 regions of the long terminal repeats (LTRs) (Brown, 1997). The LTR termini are the only viral sequences thought to be required in cis for recognition by the integration machinery of retroviruses. Short imperfect inverted repeats are present at the outer edges of the LTRs in both murine and avian retroviruses (reviewed by Reicin et al., 1995). Along with the subterminal CA located at the outermost positions 3 and 4 in retroviral DNA ends (positions 1 and 2 being the 3' end processed nucleotides, these sequences are both necessary and sufficient for correct proviral integration in vitro and in vivo. Sequences internal to the CA dinucleotide appear to be important for optimal integrase activity (Brin & Leis, 2002a; Brin & Leis, 2002b; Brown, 1997). The terminal 15 bp of the HIV-1 LTRs have been shown to be crucial for correct 3' end processing and strand transfer reactions in vitro (Reicin et al., 1995; Brown, 1997). Longer substrates are used more efficiently than shorter ones by HIV-1 IN which indicates that binding interactions extend at least 14-21 bp inward from the viral DNA end. Brin and Leis (2002a) analysed the specific features of the HIV-1 LTRs and concluded that both the U3 and U5 LTR recognition sequences are required for IN-catalysed concerted DNA integration, even though the U5 LTRs are more efficient substrates for IN processing in vitro (Bushman & Craigie, 1991; Sherman et al., 1992). The positions 17-20 of the INrecognition sequences are needed for a concerted DNA integration mechanism, but the HIV-1 IN tolerates considerable variation in both the U3 and U5 termini extending from the invariant subterminal CA dinucleotide (Brin & Leis, 2002b). The instant disclosure includes a DNA vector that contains viral (retroviral or HIV) LTR regions at the 5' and 3' ends of a location to house the DNA sequence or gene of interest to be integrated into the genome. The LTR regions do not have to be the full length LTRs as long as they function to interact with the integrase for proper integration. The LTR regions may be modified to contain detectable (e.g. fluorescent), PCR detection, or selectable markers (e.g. antibiotic resistance). The vector is designed to be cut and linearized so that the LTR regions are at the 5' and 3' ends of the DNA fragment (via designed restriction sites to restriction endonuclease).

Integrases consist of three domains connected by flexible linkers. These domains are an N-terminal HH-CC zinc-binding domain, a catalytic core domain and a C-terminal DNA binding domain (Lodi et al, Biochemistry, 1995, volume 34, pages 9826-9833). In some aspects of the disclosure the integrase bound to the Cas9 (or other DNA binding molecule) will not have the C-terminal binding domain. In one aspect of the disclosure, two different fusion proteins will be produced where one has catalytically inactive Cas9 fused with the N-terminal zinc binding domain of an integrase and the other has catalytically inactive Cas9 fused with the catalytic core domain of the integrase. The two different fusion proteins will be designed to bind to opposite strands of the genomic DNA as seen with TALE-Fok1 or Zinc finger-Fok1 systems. In this manner, when the N-terminal domain and the catalytic core come in contact, at the site on the genomic DNA, it will exhibit integrase activity. As full activity of integrase has also been observed to involve tetramers of integrase, fusion proteins may be designed with 1, 2, 3, 4 integrase proteins linked by flexible linkers that may be 1 to 20 amino acids in length or 4-12 amino acids in length.

### RECOMBINASES

Also disclosed are recombinases. Recombinases including Cre, Flp, R, Dre, Kw, and Gin recombinase are described in US 8816153 and US 2004/0003420. Recombinases such as Cre recombinase use LoxP sites in order to excise a sequence from the genome. Recombinases can be modified to become constitutively active for their recombination activity and also become less site specific. Thus, it is possible to target such constitutively active recombinase proteins with no sequence specificity to specific sequences of DNA in a genome by incorporating them into fusion proteins of the instant disclosure. In this manner, the CRISPR/Cas9 specifies the DNA sequence where the recombinase will contribute its recombination activity. Such recombinase proteins may be wild-type, constitutively active or dead for recombinase activity. A Cas9-recombinase such as Cas9-Gin or Cas9-Cre may be produced by use of a linker sequence or by direct fusion.

### NUCLEAR LOCALIZATION SIGNAL SEQUENCE (NLS) FOR FUSION PROTEINS

The signal peptide domain (also referred to as "NLS") is, for example, derived from yeast GAL4, SKI3, L29 or histone H2B proteins, polyoma virus large T protein, VP1 or VP2 capsid protein, SV40 VP1 or VP2 capsid protein, Adenovirus E1a or DBP protein, influenza virus NS1 protein, hepatitis virus core antigen or the mammalian lamin, c-myc, max, c-myb, p53, c-erbA, jun, Tax, steroid receptor or Mx proteins (see Boulikas, Crit. Rev. Eucar. Gene Expression, 3, 193-227 (1993)), simian virus 40 ("SV40") T-antigen (Kalderon et. al, Cell, 39, 499-509 (1984)) or other proteins with known nuclear localization. The NLS is, for example, derived from the SV40 T-antigen, but may be other NLS sequences known in the art. Tandem NLS sequences may be used.

### LINKER REGIONS

The various linkers used between fusion proteins/peptides being synthesized will be composed of amino acids. At the DNA level, these are represented by 3 base pair (bp) codons as known in the genetic code. Linkers may be from 1 to 1000 amino acids in length and any integer in between. For example, linkers are from 1 to 200 amino acids in length or linkers are from 1 to 20 amino acids in length.

### EXPRESSION VECTORS

Many nucleic acids may be introduced into cells to lead to expression of a gene. As used herein, the term nucleic acid includes DNA, RNA, and nucleic acid analogs, and nucleic acids that are double-stranded or single-stranded (i.e., a sense or an antisense single strand). Nucleic acid analogs can be modified at the base moiety, sugar moiety, or phosphate backbone to improve, for example, stability, hybridization, or solubility of the nucleic acid. Modifications at the base moiety include deoxyuridine for deoxythymidine, and 5-methyl-2'-deoxycytidine and 5- bromo-2'-doxycytidine for deoxycytidine. Modifications of the sugar moiety include modification of the 2' hydroxyl of the ribose sugar to form 2'-0-methyl or 2'-0-allyl sugars. The deoxyribose phosphate backbone can be modified to produce morpholino nucleic acids, in which each base moiety is linked to a six membered, morpholino ring, or peptide nucleic acids, in which the deoxyphosphate backbone is replaced by a pseudopeptide backbone and the four bases are retained. See, Summerton and Weller (1997) Antisense Nucleic Acid Drug Dev. 7(3): 187; and Hyrup et al. (1996) Bioorgan. Med. Chem. 4:5. In addition, the deoxyphosphate backbone can be replaced with, for example, a phosphorothioate or phosphorodithioate backbone, a phosphoroamidite, or an alkyl phosphotriester backbone. Nucleic acid sequences can be operably linked to a regulatory region such as a promoter. Regulatory regions can be from any species. As used herein, operably linked refers to positioning of a regulatory region relative to a nucleic acid sequence in such a way as to permit or facilitate transcription of the target nucleic acid. Any type of promoter can be operably linked to a nucleic acid sequence. Examples of promoters include, without limitation, tissue-specific promoters, constitutive promoters, and promoters responsive or unresponsive to a particular stimulus (e.g., inducible promoters).

Additional regions that may be useful in nucleic acid constructs, include, but are not limited to, polyadenylation sequences, translation control sequences (e.g., an internal ribosome entry segment, IRES), enhancers, inducible elements, or introns. Such regulatory regions may not be necessary, although they may increase expression by affecting transcription, stability of the mRNA, translational efficiency, or the like. Such regulatory regions can be included in a nucleic acid construct as desired to obtain optimal expression of the nucleic acids in the cell(s). Sufficient expression can sometimes be obtained without such additional elements.

A nucleic acid construct may be used that encodes signal peptides or selectable markers. Signaling (marker) peptides can be used such that an encoded polypeptide is directed to a particular cellular location (e.g., the cell surface). Non-limiting examples of such selectable markers include puromycin, ganciclovir, adenosine deaminase (ADA), aminoglycoside phosphotransferase (neo, G418, APH), dihydrofolate reductase (DHFR), hygromycin-B-phosphtransferase, thymidine kinase (TK), and xanthin-guanine phosphoribosyltransferase (XGPRT). These markers are useful for selecting stable transformants in culture. Other selectable markers include fluorescent polypeptides, such as green fluorescent protein, red fluorescent, or yellow fluorescent protein.

Nucleic acid constructs can be introduced into cells of any type using a variety of biological techniques known in the art. Non-limiting examples of these techniques would include the use of transposon systems, recombinant viruses that can infect cells, or liposomes or other non-viral methods such as electroporation, microinjection, or calcium phosphate precipitation, that are capable of delivering nucleic acids to cells. A system called Nucleofection.TM. may also be used.

Nucleic acids can be incorporated into vectors. A vector is a broad term that includes any specific DNA segment that is designed to move from a carrier into a target DNA. A vector may be referred to as an expression vector, or a vector system, which is a set of components needed to bring about DNA insertion into a genome or other targeted DNA sequence such as an episome, plasmid, or even virus/phage DNA segment. Vectors most often contain one or more expression cassettes that comprise one or more expression control sequences, wherein an expression control sequence is a DNA sequence that controls and regulates the transcription and/or translation of another DNA sequence or mRNA, respectively.

Many different types of vectors are known in the art. For example, plasmids and viral vectors, including retroviral vectors, are known. Mammalian expression plasmids typically have an origin of replication, a suitable promoter and optional enhancer, and also any necessary ribosome binding sites, a polyadenylation site, splice donor and acceptor sites, transcriptional termination sequences, and 5' flanking non- transcribed sequences. Such vectors include plasmids (which may also be a carrier of another type of vector), adenovirus, adeno-associated virus (AAV), lentivirus (e.g., modified HIV-1, SIV or FIV), retrovirus (e.g., ASV, ALV or MoMLV), and transposons (P-elements, Tol-2, Frog Prince, piggyBac or others).

Bacterial and viral genes and proteins for use in the disclosure are listed below in the section entitled "SEQUENCES OF THE DISCLOSURE". Other viral integrases, for example, those from mouse mammary tumor virus (MMTV) and adenovirus can also be used in the methods and compositions disclosed herein.

A pooled population of edited cells are considered a mixture of cells that have received a gene edit and cells that have not.

### EXEMPLARY ABBIE1 IN VITRO ASSAY

1) Incubate ABBIE1 protein with guide RNA;
2) Incubate ABBIE1/guide RNA with donor DNA having partial LTRs to form pre-initiation complex;
3) Incubate pre-initiation complex with plasmid containing gene to be edited (e.g. CXCR4); and
4) PCR and DNA sequencing confirmations for donor DNA integration.

Cas9protocols are described in, for example, Gagnon et al., 2014, http://labs.mcb.harvard.edu/schier/VertEmbryo/Cas9_Protocols.pdf.

Assays for integrase activity are described in, for example, Merkel et al., Methods, 2009, volume 47, pages 243-248.

### EXAMPLES

The following examples are intended to provide illustrations of the application of the present disclosure. The following examples are not intended to completely define or otherwise limit the scope of the disclosure.

### EXAMPLE 1: DNA VECTORS FOR EXPRESSION CAS9-INTEGRASE FUSION PROTEINS

The DNA sequence of catalytically inactive Cas9 is incorporated into an expression vector with a 12, 15, 18, 21, 24, 27 or 30 bp spacer (codes for 4, 5, 6, 7, 8, 9 or 10 amino acids as the linker between the Cas9 and the integrase) and the HIV1 integrase. In other experiments, recombinases of bacterial or phage origin are used rather than integrases. These include Hin recombinase (SEQ ID NO: 25) and Cre recombinase (SEQ ID NO: 26) with or without mutations that allow them to recombine DNA at any other sites. A His or cMyc tag (or other sequence useful for protein purification) may be included to isolate the fusion protein. The expression vector uses a promoter that will be activated in the cells that will be provided with the vector. The CMV (cytomegalovirus promoter) is commonly used for expression vectors for mammalian cells. The U6 promoter is also commonly used. A T7 promoter may be used for in vitro transcription in certain embodiments.

### EXAMPLE 2: DNA VECTOR FOR EXPRESSION OF THE DNA SEQUENCE OF INTEREST (GENE OF INTEREST)

The DNA sequence of interest will be inserted into the appropriate expression vector and sites will be appropriately added to the DNA sequence of interest so the HIV1 integrase will recognize the sequences for integration into the genome. These sites are termed att sites (U5 and U3 att sites) (see Masuda et al, Journal of Virology, 1998, volume 72, pages 8396-8402). Homology arms for the target site in the genome can be included in regions flanking the 5' and 3' ends of the DNA (gene) sequence of interest (see Ishii et al, PLOS ONE, September 24, 2014, DOI: 10.1371/journal.pone.0108236). When using a recombinase, the integrase recognition sites may not be included. Markers, such as drug resistance markers (e.g. blasticidin or puromycin), will be included in order to check for insertion of the DNA sequence of interest and to help assay for random insertions in the genome. These resistance markers can be engineered in such a way to remove them from the targeted genome landing pad For example flanking the puromycin resistance gene with a LoxP sites and introducing exogenously expressed CRE would remove the internal sequence leaving a scar containing a LoxP site.

### EXAMPLE 3: DNA VECTOR FOR REVERSE TRANSCRIPTASE EXPRESSION

A reverse transcriptase may also be co-expressed in such systems as the designed DNA sequence (Gene) of interest in the vector will become expressed as RNA and will have to be converted back to DNA for integration by the integrase enzyme. The reverse transcriptase may be viral in origin (e.g. a retrovirus such as HIV1). This may be incorporated within the same vector as the DNA sequence of interest.

### EXAMPLE 4: CO-EXPRESSION OF DNA TARGETING-INTEGRASES (OR RECOMBINASES) WITH DNA SEQUENCE OF INTEREST

Cells were electroporated for the vectors described above along with the Cas9 RNA guides required for the target site in the genome. In some experiments, vectors were created that expressed all of the components (fusion Cas9/integrase (or recombinase), the Cas9 RNA guides, and the DNA sequence of interest with integrase recognition sites and with or without homology arms). A reverse transcriptase may also be co-expressed in such systems as the designed DNA sequence (Gene) of interest in the vector will become expressed as RNA and will have to be converted back to DNA for integration by the integrase enzyme. The reverse transcriptase may be viral in origin (e.g. a retrovirus such as HIV1). In other experiments, the DNA sequence of interest in linearized before introduction to the cell. The Cas9 RNA guide sequences and DNA sequence of interest had to be designed and inserted into the vector before use by standard molecular biology protocols.

### EXAMPLE 5: TEST EXPERIMENTS AND ASSAYING FOR OFF-TARGET INSERTIONS

Cells missing expression of a particular gene, such as mouse embryonic fibroblasts from a knockout mouse model or cells genetically engineered to be knockouts for a given gene, are transfected or electroporated with the above vectors where the gene of interest is included. Chimeric primer sets designed to cover the inserted gene as well as flanking genomic sequence will be used to screen initial pools of edited cells. Limited dilution cloning (LDC) and or FACS analysis is then performed to ensure monoclonality. Next generation sequencing (NGS) or single nucleotide polymorphism (SNP) analysis is performed as a final quality control step to ensure isolated clones are homogenous for the designed edit. Other mechanisms for screening can include but are not limited to qRT-PCR and western blotting with appropriate antibodies. If the protein is associated with a certain phenotype of the cells, the cells may be examined for rescue of that phenotype. The genomes of the cells are assayed for the specificity of the DNA insertion and to find the relative number of off-target insertions, if any.

### EXAMPLE 6: CAS9 LINKED INTEGRASE PROTEIN EXPRESSION AND ISOLATION

Vectors designed for gene expression in E coli or insect cells will be incorporated into E coli or insect cells and allowed to express for a given period of time. Several designs will be utilized to generate Cas9 (or inactive Cas9) linked integrase protein. The vectors will also incorporate a tag that is not limited to a His or cMyc tag for eventual isolation of the protein with high purity and yield. Preparation of the chimeric protein will include but are not limited to standard chromatography techniques. The protein may also be designed with one or more NLS (nuclear localization signal sequence) and/or a TAT sequence. The nuclear localization signal allows the protein to enter the nucleus. The TAT sequence allows for easier entry of a protein into a cell (it is a cell-penetrating peptide). Other cell penetrating peptides in the art may be considered. After sufficient time for expression has occurred, protein lysate will be collected from the cells and purified in the appropriate column depending on the tag used. The purified protein will then be placed in the appropriate buffering solution and stored at either -20 or -80 degrees C.

### EXAMPLE 7: USING CAS9-INTEGRASE TO INCORPORATE STOP CODONS JUST UPSTREAM OF TRANSCRIPTION START SITE

The disclosure includes a method to create a knockout cell line or organism. The above system is used with the DNA sequence of interest being 1, 3, 6, 10, 15 or 20 consecutive stop codons to be placed just after the ATG start site for the target gene. This will create an effective gene knockout as transcription/translation will be stopped when reaching the immediate stop codon after the ATG start site. Additional stop codons will help prevent possible run through of the transcriptase (if transcriptase by-passes the first stop codon).

### EXAMPLE 8: USING ABBIE1 (OR OTHER VARIATIONS HAVING OTHER SPECIFIC DNA BINDING DOMAINS) AS A PURIFIED PROTEIN TO EDIT THE GENOMES OF CELLS

Incubate Abbie1 isolated protein (other specific DNA sequence binding protein linked to retroviral integrase) with insertable/integratable DNA having viral LTR regions in a suitable buffer. (for formation of tetramer or other multimer depending on the instance). Alternatively, a premade composition of isolated Abbie1 protein with guide RNA may be combined with the insertable DNA sequence. Include guide RNA and incubate to incorporate guide RNA. Transfect or electroporate (or other technique of providing protein to cells) Abbie1/DNA preparation into cells. Allow time for genome/DNA editing to take place. Check for insertion of designed insertable DNA sequence into the specific site of the genomic DNA of the cell. Check for non-specific insertions by PCR and DNA sequencing.

As currently planned, the bacterial expression vector will be the pMAL-c5e, which is a discontinued product from NEB and one of the in-house cloning choices for Genscript. Codon-optimized Spy Cas9 is cloned with the his-tag and the TEV protease cleavage site in frame with the maltose-binding protein (MBP) tag. The ORF is under the inducible Tac promoter, and the vector also codes for the lac repressor (LacI) for tighter regulation. MBP will be used only as a stabilization tag and not a purification tag, for the amylose resin is quite expensive. The soluble expressed material will be purified over the Ni-affinity chromatography, then Cas9 is released by the TEV protease from MBP, purified by cation exchange chromatography, and polished by gel filtration.

### EXAMPLE 9: DESIGN OF CONSTRUCTS FOR FUSION PROTEINS

Design sequence specific Zinc finger domain, TALE, or guide RNA for CRISPR based approach toward a target DNA sequence. Use on-line design software of choice.

Produce DNA construct with coding sequences for integrase, transposase or recombinase; a suitable amino acid linker; the appropriate zinc finger, TALE or CRISPR protein (e.g. Cas9, Cpfl); and a nuclear localization signal (or mitochondrial localization signal) to form the site specific fusion integrase protein. These are envisioned in multiple arrangements. A suitable tag may be included for protein isolation and purification if desired (e.g. maltose binding protein (MBP) or His tag).

DNA construct may utilize a mammalian cell promoter or a bacterial promoter common in the art (e.g. CMV, T7, etc.)

One may produce a recombinant fusion protein with E coli as the source. Isolate the protein by standard means in the art (e.g. MBP columns, nickel-sepharose columns, etc.).

Assemble the Donor-RNP complex (duplex the RNA oligos and mix with fusion protein of the invention (when fusion protein has an endonuclease inactive CRISPR related protein for its DNA binding ability, e.g. ABBIE1) - these steps of forming RNP are not necessary for Zinc finger domains and TALE.
1. Mix Donor DNA with appropriate LTR domains and insertable sequence, and fusion protein and incubate for 10 minutes. (alternatively add Donor DNA after the RNP complex formation)
2. Resuspend each RNA oligo (crRNA and tracrRNA) in Nuclease-Free IDTE Buffer. For example, use a final concentration of 100 µM.
3. Mix the two RNA oligos in equimolar concentrations in a sterile microcentrifuge tube. For example, create a final duplex concentration of 3 µM using the following table: Component Amount 100 µM crRNA 3 µL 100 µM tracrRNA 3 µL Nuclease-Free Duplex Buffer 94 µL Final volume 100 µL
4. Heat at 95° C for 5 min.
5. Remove from heat and allow to cool to room temperature (15-25°C) on your bench top.
6. If needed, dilute duplexed RNA to a working concentration (for example, 3 µM) in Nuclease-Free Duplex Buffer.
7. Dilute fusion protein to a working concentration (for example, 5 µM) in Working Buffer (20 mM HEPES, 150 mM KCI, 5% Glycerol, 1mM DTT, pH 7.5).
8. For each transfection, combine 1.5 pmol of duplexed RNA oligos (Step A5) with 1.5 pmol of fusion protein (Step A6) in Opti-MEM Media to a final volume of 12.5 µL.
9. Incubate at room temperature for 5 min to assemble the RNP complexes.

### EXAMPLE 10: REVERSE TRANSFECT GRNA-FUSION PROTEIN IN A 96-WELL PLATE

1. Incubate the following at room temperature for 20 min to form transfection complexes: Component Amount RNP (Step A8) 12.5 µL Lipofectamine^{®} RNAiMAX Transfection Reagent 1.2 µL Opti-MEM^{®} Media 11.3 µL Total volume 25.0 µL
2. During incubation (Step B1), dilute cultured cells to 400,000 cells/mL using complete media without antibiotics.
3. When incubation is complete, add 25 µL of transfection complexes (from Step B1) to a 96-well tissue culture plate.
4. Add 125 µL of diluted cells (from Step B2) to the 96-well tissure culture plate (50,000 cells/well; final concentration of RNP will be 10 nM).
5. Incubate the plate containing the transfection complexes and cells in a tissure culture incubator (37°C, 5% CO2) for 48 hr. To detect on-target mutations, use PCR with appropriate primers (primers within donor sequence and primers surrounding the target insertion site).

### EXAMPLE 11: PROTOCOL FOR TESTING THE SPECIFICITY OF CRISPR/CAS9

Produce dCas9 (DNA cutting inactive Cas9) linked to biotin (dCas9-biotin). Cas9 (s pyogenes, s aureus, etc.). Biotinylation methods are described below.

Biotinylation method #1: engineer the avi-tag (~15 residues) at the N- or C-terminus, express and purify as the WT (un-tagged) protein. Use the E. coli biotin ligase (BirA) and biotin to biotinylate the avi-tagged Cas9. We use this scheme to biotinylate chemokines. I believe the IP on the avi-tag technology expired a few years ago.

Biotinylation method #2.1: biotin functionalized with succinimidyl-ester can be incorporated at surface-exposed lysines residues (no enzymatic reaction required). For proteins as big as Cas9, this can be a viable option.

Biotinylation method #2.2: along the same line, biotin-maleimide is commercially available, and they can be conjugated at surface-exposed cysteines (no enzyme).

Testing will be accomplished to characterize the biotinylated Cas9 does in terms of DNA-binding and cleavage.

Streptavidin-coated 96-well plates are commercially available, but may also be produced in-house.

Bind dCas9-biotin to plastic plates (96-well, 24-well, 384-well, etc.).

Provide designed guide RNAs to each well. Allow time for guide RNAs to interact with Cas9 protein.

Provide genomic DNA to each well or DNA with targeted sequence. Allow time for Cas9 binding to DNA.

Wash wells with appropriate buffer.

Provide an adapter (DNA oligomer). Allow time to bind.

Restriction-digest the genomic DNA to make it more tractable and easier to ligate the adapter.

Wash wells.

Perform DNA sequencing to identify sites of binding (on target vs. off target).

### EXAMPLE 12: NRF2 EDITING VIA ABBIE 1

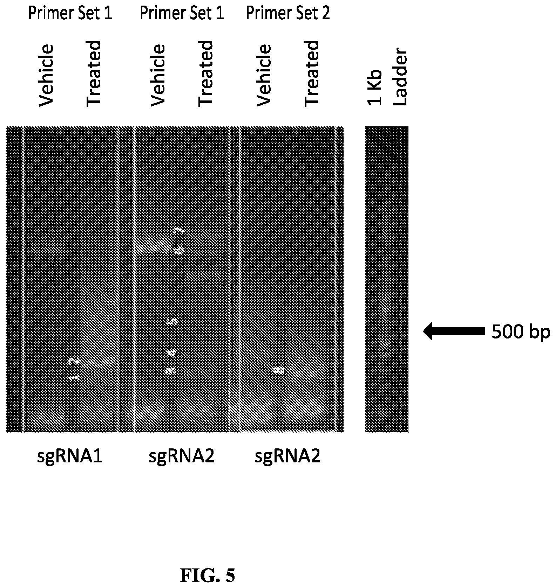

**FIG. 5** shows Abbie1 Gene Editing Targeting Exon 2 of Nrf2 Using Guide NrF2-sgRNA2 and sgRNA3. PCR screen against exon 2 targeting Nrf2 locus for knock-out via Abbie1 Editing. Abbie1 transfection targeting exon 2 of Nrf2 using guide NrF2-sgRNA 2 and 3 showed integration of donor at targeted region. Unique bands are identified as 1-8.

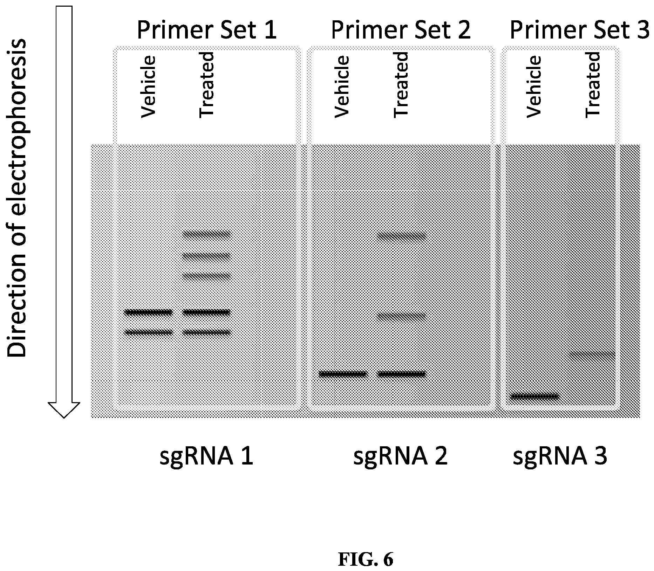

**FIG. 6** shows theoretical data generated by Abbie1 gene editing. Representation of DNA gel electrophoresis visualizing inserted donor DNA via the Abbie1 system to target genomic material using sgRNA 1-3. Black bands represent background product due to PCR methodology. Red bands represent unique products generated by amplifying insert and genetic material flanking the region of insert. Multiple bands represent possible multiple insertion in targeted region.

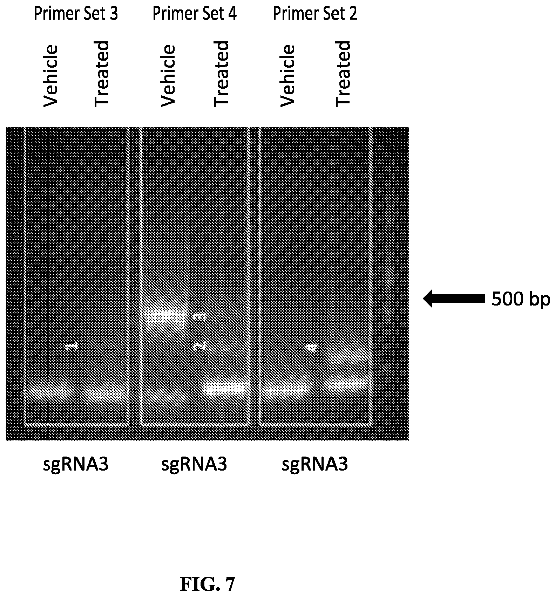

**FIG. 7** shows Abbie1 Gene Editing Targeting Exon 2 of Nrf2 Using Guide Nrf2-sgRNA 3. PCR screen against exon 2 targeting Nrf2 locus for knock-out via Abbie1 Editing. Targeting exon 2 of Nrf2 using guide NrF2-sgRNA 3 suggested donor insertions as indicated by PCR primers designed to donor sequence and adjacent site to expected insertion. Unique bands are identified as 1-4

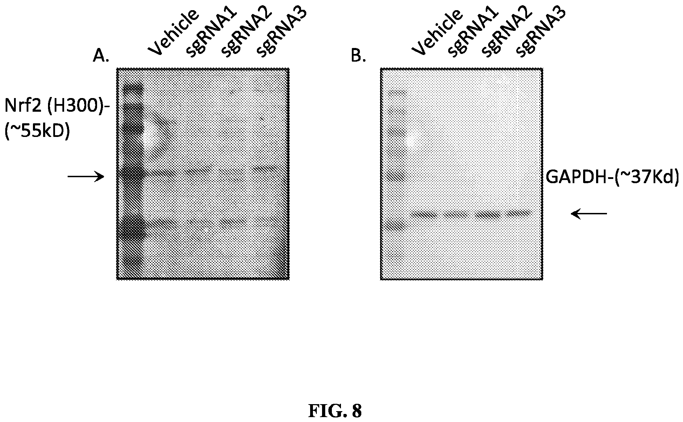

**FIG. 8** shows Abbie1 Knock out of Nrf2 in pooled Hek293T Cells. (A)Western blot analysis using polyclonal antibody against 55kD isoform (Santa Cruz Bio) showing knock out of Nrf2 in pooled HEK293T poplulations. (B) GAPDH (Santa Cruz Bio) loading control.

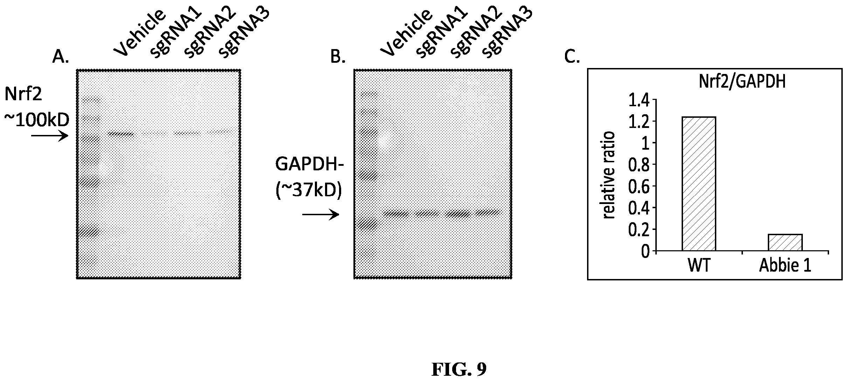

**FIG. 9** shows Abbie1 Knock out of Nrf2 in pooled Hek293T Cells. (A) Western blot analysis utilizing monoclonal antibody against Nrf2 (Abcam) showing knockout of Nrf2 pooled poplulations in HEK 293t cells. (B) GAPDH loading control. (C) Average of densitometric analysis showing decrease in expression ratios as compared to control.

Abbie1 treated cells generate a unique PCR band indicating integration of donor DNA. Phenotypic confirmation of knock out in a HEK293T pooled cell line was confirmed via western blot analysis probing for two isoforms with unique and different antibodies. ~80% knock out by integration was observed in pooled populations in under two weeks.

### EXAMPLE 13: CXCR4 EDITING VIA ABBIE1

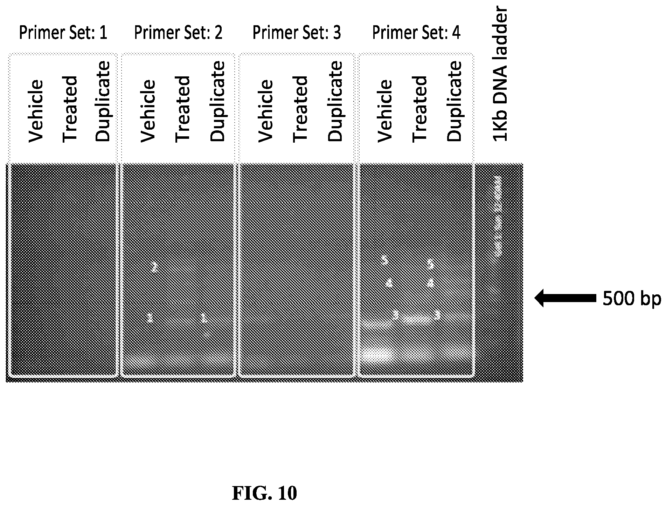

**FIG. 10** shows Abbie1 Gene Editing Targeting CXCR4 Exon 2. PCR screen targeting exon 2 of CXCR4 edited via Abbie1. Four sets of primers were designed against the region of interest. Set number 2 and 4 appears to have generated unique bands suggesting integration of donor DNA at the region of interest.

### EXAMPLE 14: TRANSFECTION FOR THE KNOCK-IN EXPERIMENT AT THE NRF2 LOCUS USING ABBIE1.

Note: 500 ng protein and 120ng sgRNA are used for a single reaction. The amount of DNA depends on the size of the donor constructs. Donor DNA (DNA with LTR sequences) may be incubated with ABBIE1 before, during, or after providing/transfecting/electroporating to the cells. All reactions are prepared in sterile biosafety cabinet.

Day 1: Human embryonic kidney (HEK 293T) Cells were seeded into 24-well culture plate (Corning) at 200,000 HEK293T cells (ATCC) per well in 500 µL DMEM (Gibco) supplemented with 10% fetal bovine serum (Omega Scientific). Cells were allowed to recover for 24 hours.

Day 2: ABBIE1 Preparation:
Tube 1:
   Purified ABBIE1 protein (SEQ ID NO: 58) and donor DNA (SEQ ID NO: 101) in a reduced-serum transfection medium (OptiMEM, Life Technologies) at 1:1 molar ratio for 10 minutes at room temperature. Add the sgRNA to the 1.3-fold molar excess (approximately 120 ng) to the protein/DNA complex and continue the incubation for additional 10 minutes at room temperature. The volume of this mixture is 25 µL.
Tube 2:
   2µL of transfection reagent (RNAiMAX, Life Technologies) was added to 23 µL of OptiMEM. And allowed to incubate for 10 minutes at room temperature.

Combined Tube 1 and Tube 2 (50µl final volume) and incubated for 15 minutes at room temperature.

Added the entire 50µL transfection mixture to the well.

Half of the pooled edited cells were harvested 48 hours after transfection for the verification of the genomic DNA editing in a pooled population. Verification of edited genome was performed by polymerase chain reaction (PCR). We performed PCR against the targeted region as described below (See PCR protocol) the remainder was seeded onto 6 cm culture dishes (Corning) and allowed to recover for 48 hours.

Day 5: Screening of phenotypic changes via western blotting.

Standard western blot analysis was performed for NrF2 isoforms using primary antibodies targeting 55kD isoform (Santa Cruz Biotechnology, sc-722) as well as 98kD isoform (Abcam, ab-62352). GAPDH (Santa Cruz Biotechnology, sc-51907)

### EXAMPLE 15: PCR CONDITIONS FOR DETECTION OF GENE EDITING USING ABBIE1 FOR NRF2 AND CXCR4 LOCUS.

Accession number for human Nrf2
Uniprot: Q16236
Ensembl gene ID: ENSG00000116044
Editing target sequences and PAMs for Nrf2 (exon 2): Used for sgRNA design 1-3.
GCGACGGAAAGAGTATGAGC TGG
TATTTGACTTCAGTCAGCGA CGG
TGGAGGCAAGATATAGATCT TGG
Primer Key for Detection of Integration at Nrf2 Target
Primer Set 1: Primer 1:5'-GTGTTAATTTCAAACATCAGCAGC-3', Primer 2: 5'- GACAAGACATCCTTGATTTG-3'
Primer Set 2: Primer 1:5'-GAGGTTGACTGTGTAAATG-3', Primer 2: 5'-GATACCAGAGTCACACAACAG-3'
Primer Set 3: Primer 1: 5'-TCTACATTAATTCTCTTGTGC-3', Primer 2:5'-GATACCAGAGTCACACAACAG-3'
Accession number for human CXCR4
Uniprot P61073
Ensembl gene ID: ENSG00000121966
Editing target sequence and PAM for CXCR4 (Exon 2): Used for sgRNA design1.
GGGCAATGGATTGGTCATCC TGG
Primer Key for Detection of Integration at CXCR4 Target
Primer Set 1: Primer 1: 5'- TCTACATTAATTCTCTTGTGC-3', Primer 2: 5'-GACAAGACATCCTTGATTTG-3'
Primer Set 2: Primer 1: 5'- TCTACATTAATTCTCTTGTGC-3', Primer 2: 5'-GATACCAGAGTCACACAACAG -3'
Primer Set 3: Primer 1: 5'- GAGGTTGACTGTGTAAATG -3', Primer 2: 5'-GACAAGACATCCTTGATTTG-3'
Primer Set 4: Primer 1: 5'- GAGGTTGACTGTGTAAATG -3', Primer 2: 5'-GATACCAGAGTCACACAACAG -3'
PCR Cycling conditions used for detection of integrated donor DNA
*Note annealing temperatures will vary depending on primer sequence

| | | |
|---|---|---|
| 1. Initial denaturation: | 4 min | 94°C |
| 2. denaturation: | 30 sec | 94°C |
| 3. annealing: | 30 sec | 55°C |
| 4. extension: | 30 sec | 72°C |
| 5. go to step 2: | 40 cycles | |
| 6. final extension: | 4 min | 72°C |
| 7. final hold: | ∞ | 4°C |

Avi-tagged Cas9 for biotinylation
Sequence of the avi-tag used for Cas9 biotinylation
Amino acid seqeunce:
   G G D L E G S G L N D I F E A Q K I E W H E *
Nucleic acid sequence: 
GGCGGCGACCTCGAGGGTAGCGGTCTGAACGATATTTTTGAAGCGCA
GAAAATTGAATGGCATGAATAA
First Underlined section = Cas9 C-terminus
Italicized section = restriction site/linker
Second underlined section = avi-tag (biotinylation site highlighted)

### EXAMPLE 16: EXPRESSION PROTOCOL FOR ABBIE1 FUSION PROTEIN.

Transformation of expression construct containing full-length fusion protein (SEQ ID NO: 57).

Take competent E. coli cells from -80°C freezer.

Turn on water bath to 42°C.

Put competent cells in a 1.5 ml tube (Eppendorf or similar). For transforming a DNA construct, use 50 ul of competent cells.

Keep tubes on ice.

Add 50 ng of circular DNA into E.coli cells. Incubate on ice for 10 min. to thaw competent cells.

Put tube(s) with DNA and E.coli into water bath at 42°C for 45 seconds. Put tubes back on ice for 2 minutes to reduce damage to the E.coli cells.

Add 1 ml of LB (with no antibiotic added). Incubate tubes for 1 hour at 37 oC. (Can incubate tubes for 30 minutes
Spread about 100 ul of the resulting culture on LB plates with appropriate antibiotic
Pick colonies about 12-16 hours later.

### INNOCULATION AND EXPANSION

Innoculate a 1 liter flask containing LB and antibiotic
Allow bacterial culture to grow until 0.6 OD is achieved then induce with Isopropyl β-D-1-thiogalactopyranoside (IPTG) at a 1mM final concentration
Allow the culture to expand for 6-8 hours and centrifuge the suspended bacterial culture at a minimum of two thousand G force for 10 minutes.

Freeze pellet at -80 C for further processing at a later time

### PROTEIN PREPARATION AND PURIFICATION

All steps are performed at room temperature.

Lyse the cells by 2 cycles of freeze-thaw in 20 mM Tris pH8.0, 300 mM NaCl, 0.1 mg/ml chicken egg white lysozyme. Centrifuge at 6,000 g for 15 minutes and retain the supernatant.

Load the supernatant onto a Ni-IDA agarose column equilibriated in 20 mM Tris pH8.0, 300 mM sodium chloride. Elute the protein with a 0-to-200 mM gradient of imidazole. Identify the fractions containing the fusion protein by a 7% SDS-PAGE.

Pool the fractions and dilute with 20 mM Tris pH8.0 so that the final NaCl concentration is 50 mM. Load onto a Q-sepharose column and elute with a 0-to-500 mM gradient of sodium chloride. Identify the fractions containing the fusion protein by a 7% SDS-PAGE.

Pool the fractions and dilute with 20 mM Tris pH8.0 so that the final NaCl concentration is 100 mM. Load onto an SP-sepharose column and elute with a 0-to-500 mM gradient of sodium chloride. Identify the fractions containing the fusion protein by a 7% SDS-PAGE.

Pool the fractions, measure the concentration by its UV absorbance at 280 nm, and concentrate by a centrifugal filter to the final concentration of 400 µg/ml. Add glycerol to the final concentration of 50%. Store at -20°C.

While certain embodiments have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the disclosure. It is intended that the following claims define the scope of the disclosure and that methods and structures within the scope of these claims and their equivalents be covered thereby.

### SEQUENCES OF THE DISCLOSURE

For each sequence provided below, the following information is provided: type of sequence (nucleic acid or amino acid), source (e.g. E. coli), length, and identification number (if available).

A first polynucleotide of the disclosure can encode, for example, a Cas9, Cpfl, TALE, or ZnFn protein. A second polynucleotide of the disclosure can encode, for example, an integrase, transposase, or recombinase. Listed below are exemplary first and second polynucleotide sequences and protein sequences, along with exemplary linker sequences, that can be used in the compositions (constructs, fusion proteins) and methods described herein. Other polynucleotide sequences, protein sequences, or linker sequences may be provided in the disclosure that are not listed in Table 1 below, but can be used in the compositions (constructs, fusion proteins) and methods described herein. For example, SEQ ID NO: 49, SEQ ID NO: 57, SEQ ID NO: 58, and/or portions thereof.

A linker can be any length, for example, 3 to 300 nucleotides in length, 6 to 60 nucleotides in length, or any length that will allow the first and second polynucleotide to be fused. A polypeptide can be made by an organism, e.g. E. coli or be made synthetically, or a combination of both.

Exemplary nucleic acid sequences: 1, 3, 5, 7, 9, 11, 13, 15, 17, 19, 21, 23, 27-47, 49, 55, 56, 57, 62, 64, 66, 68, 70, 79, 82, and 83.

Exemplary amino acid sequences: 2, 4, 6, 8, 10, 12, 14, 16, 18, 20, 22, 24, 25, 26, 48, 50, 52, 58, 63, 65, 67, 69, 71, 72-78, and 80.

**TABLE 1: FIRST PROTEIN, SECOND PROTEIN, OR LINKER**

| first polynucleotide or protein sequence SEQ ID NO: | second polynucleotide or protein sequence SEQ ID NO: | linker sequence SEQ ID NO: or sequence |
|---|---|---|
| 1-14, 27-46, 50, 52, 56, 57, 68, 69, 72-78, 86-92, 200-253 | 15-26, 47, 48, 55, 57, 62-67, 70, 71, 79, 80 | 51, 54, 61, GGS |

**TABLE 2: PARTIAL LIST OF SEQUENCES**

| **Gene (protein)** | **Bacteria/Virus** | **DNA sequence** | **Protein sequence** | **SEQ ID (DNA, protein)** |
|---|---|---|---|---|
| **Cas9** | S. thermophilus | HQ712120.1 | Q03JI6.1 | SEQ ID NOS: 1, 2 |
| | P. multocida | | Q9CLT2.1 | SEQ ID NOS: 3, 4 |
| | S. mutans | | Q8DTE3.1 | SEQ ID NOS: 5, 6 |
| | N. meningitides | | C9X1G5.1 | SEQ ID NOS: 7, 8 |
| | S. mitis | | KJQ69483.1 | SEQ ID NOS: 9, 10 |
| | S. macacae | | EHJ52063.1 | SEQ ID NOS: 11,12 |
| | Staphylococcus Aureus | | KKJ92487.1 | SEQ ID NOS: 49, 50 |
| | S. pyogenes | | AFV37892.1 | SEQ ID NOS: 13, 14 |
| **Integrase** | HIV1 | | ABR68182.1 | SEQ ID NOS: 15, 16 |
| | Simian T-lymphocyte virus | | AAA47841.1 | SEQ ID NOS: 17, 18 |
| | S. pneumonia | | CBW38769.1 | SEQ ID NOS: 19, 20 |
| | E. coli | | CAA41325.1 | SEQ ID NOS: 21, 22 |
| | Lentivirus | | | SEQ ID NOS: 47, 48 |
| **Recombinase** | Thermoanaerobacterium phage | | YP_006546326.1 | SEQ ID NOS: 23 24 |

### ADDITIONAL SEQUENCES

**SEQ ID NO: 1**
   NAME: S.thermophilus Csn1 cds HQ712120.1
   SEQUENCE: 
ATGACTAAGCCATACTCAATTGGACTTGATATTGGAACGAATAGTGTTGGAT
GGGCTGTAATAACTGATAATTACAAGGTTCCGTCTAAAAAAATGAAAGTCTT
AGGAAATACGAGTAAAAAGTATATCAAAAAGAACCTGTTAGGTGTATTACTC
TTTGACTCTGGAATCACAGCAGAAGGAAGAAGATTGAAGCGTACTGCAAGAA
GACGTTATACTAGACGCCGTAATCGTATCCTTTATTTGCAGGAAATTTTTAGC
ACGGAGATGGCTACATTAGATGATGCTTTCTTTCAAAGACTTGACGATTCGTT
TTTAGTTCCTGATGATAAACGTGATAGTAAGTATCCGATATTTGGAAACTTAG
TAGAAGAAAAAGTCTATCATGATGAATTTCCAACTATCTATCATTTAAGGAA
ATATTTAGCAGATAGTACTAAAAAAGCAGATTTGCGTCTAGTTTATCTTGCAT
TGGCTCATATGATTAAATATAGAGGTCACTTCTTAATTGAAGGAGAGTTTAAT
TCAAAAAATAATGATATTCAGAAGAATTTTCAAGACTTTTTGGACACTTATAA
TGCTATTTTTGAATCGGATTTATCACTTGAGAATAGTAAACAACTTGAGGAAA
TTGTTAAAGATAAGATTAGTAAATTAGAAAAGAAAGATCGTATTTTAAAACT
CTTCCCTGGGGAGAAGAATTCGGGGATTTTTTCAGAGTTTCTAAAGTTGATTG
TAGGAAATCAAGCTGATTTTAGGAAATGTTTTAATTTAGACGAAAAAGCCTC
CTTACATTTTTCCAAAGAAAGCTATGATGAAGATTTAGAGACTTTGTTAGGTT
ATATTGGAGATGATTACAGTGATGTCTTTCTCAAAGCAAAGAAACTTTATGAT
GCTATTCTTTTATCGGGTTTTCTGACTGTAACTGATAATGAGACAGAAGCACC
TCTCTCTTCTGCTATGATAAAGCGATATAATGAACACAAAGAAGATTTAGCGT
TACTAAAGGAATATATAAGAAATATTTCACTAAAAACGTATAATGAAGTATT
TAAAGATGACACCAAAAATGGTTATGCTGGTTATATTGATGGAAAAACAAAT
CAGGAAGATTTCTACGTATATCTAAAAAACCTATTGGCTGAATTTGAAGGTG
CGGATTATTTTCTTGAAAAAATTGATCGAGAAGATTTTTTGAGAAAGCAACGT
ACATTTGACAATGGTTCGATACCATATCAGATTCATCTTCAAGAAATGAGAG
 
CAATTCTTGATAAGCAAGCTAAATTTATCCTTTCTTGGCTAAAAATAAAGAA
AGAATCGAGAAGATTTAACCTTCCGAATTCCTTATTATGTAGGTCCACTTGC
GAGAGGGAATAGTGATTTGCCTGGTCAATAAGAAAACGAAATGAAAAATT
ACACCTTGGAATTTTGAGGACGTTATTGACAAAGAATCTTCGGCAGAGGCTTT
CATTAATCGAATGACTAGTTTGATTTGTATTTGCCAGAAGAGAAGGTACTTC
CAAAGCATAGTCTCTTATACGAAACTTTTAATGTATATAATGAATTAACAAAA
GTTAGATTTATTGCCGAAAGTATGAGAGATTATCAATTTTTAGATAGTAAGCA
GAAGAAAGATATTGTTAGACTTTATTTTAAAGATAAAAGGAAAGTTACTGAT
AAGGATATTATTGAATATTTACATGCAATTTATGGGTATGATGGAATTGAATT
AAAAGGCATAGAGAAACAGTTTAATTCTAGTTTATCTACTTATCACGATCTTT
TAAATATTATTAATGATAAAGAGTTTTTGGATGATAGTTCAAATGAAGCGATT
ATCGAAGAAATTATCCATACTTTGACAATTTTTGAAGATAGAGAGATGATAA
AACAACGTCTTTCAAAATTTGAGAATATATTCGATAAATCCGTTTTGAAAAG
TTATCTCGTAGACATTACACTGGCTGGGGTAAGTTATCTGCTAAGCTTATTAA
TGGTATTCGAGATGAAAAATCTGGTAATACTATTCTTGATTACTTAATTGATG
ATGGTATTTCTAACCGTAATTTCATGCAACTTATTCACGATGATGCTCTTTCTT
TTAAAAAGAAGATACAGAAAGCACAAATTATTGGTGACGAAGATAAAGGTA
ATATTAAAGAGGTCGTTAAGTCTTTGCCAGGTAGTCCTGCGATTAAAAAGG
TATTTTACAAAGCATAAAAATTGTAGATGAATTGGTCAAAGTAATGGGAGGA
AGAAAACCCGAGTCAATTGTTGTTGAGATGGCTCGTGAAAATCAATATACCA
ATCAAGGTAAGTCTAATTCCCAACAACGCTTGAAACGTTTAGAAAAATCTCT
CAAAGAGTTAGGTAGTAAGATACTTAAGGAAAATATTCCTGCAAAACTTTCT
AAAATAGACAATAACGCACTTCAAAATGATCGACTTTACTTATACTATCTTCA
AAATGGAAAAGATATGTATACCGGAGATGATTTAGATATTGATAGATTAAGT
AATTATGATATTGATCATATTATTCCTCAAGCTTTTTTGAAAGATAATTCTATT
GACAATAAAGTACTTGTTTCATCTGCTAGTAACCGTGGTAAATCAGATGATTT
TCCAAGTTTAGAGGTTGTCAAAAAAAGAAAGACATTTTGGTATCAATTATTG
AAATCAAAATTAATTTCTCAACGAAAATTTGATAATCTGACAAAAGCTGAAC
GGGGAGGATTGTTACCTGAGGACAAAGCTGGTTTTATTCAACGCCAGTTGGT
TGAAACACGTCAAATAACAAAACATGTAGCTCGTTTACTTGATGAGAAATTT
AATAATAAAAAAGATGAAAATAATAGAGCGGTACGAACAGTAAAAATTATT
 
ACCTTGAAATCTACCTTAGTTTCTCAATTTCGTAAGGATTTTGAACTTTATAA
AGTTCGTGAAATCAATGATTTTCATCATGCTCATGATGCTTACTTGAATGCCG
TTATAGCAAGTGCTTTACTTAAGAAATACCCTAAACTAGAGCCAGAATTTGTG
TACGGTGATTATCCAAAATACAATAGTTTTAGAGAAAGAAAGTCCGCTACAG
AAAAGGTATATTTCTATTCAAATATCATGAATATCTTTAAAAAATCTATTTCT
TTAGCTGATGGTAGAGTTATTGAAAGACCACTTATTGAGGTAAATGAGGAGA
CCGGCGAATCCGTTTGGAATAAAGAATCTGATTTAGCAACTGTAAGGAGAGT
ACTCTCTTATCCGCAAGTAAATGTTGTGAAAAAAGTTGAGGAACAGAATCAC
GGATTGGATAGAGGAAAACCAAAGGGATTGTTTAATGCAAATCTTTCCTCAA
AGCCAAAACCAAATAGTAATGAAAATTTAGTAGGTGCTAAAGAGTATCTTGA
CCCCAAAAAGTATGGGGGTATGCTGGAATTTCTAATTCTTTTGCTGTTCTTG
TTAAAGGGACAATTGAAAAAGGTGCTAAGAAAAAAATAACAAATGTACTAG
AATTTCAAGGTATTTCTATTTTAGATAGGATTAATTATAGAAAAGATAAACTT
AATTTTTTACTTGAAAAAGGTTATAAAGATATTGAGTTAATTATTGAACTACC
TAAATATAGTTTATTTGAACTTTCAGATGGTTCACGTCGTATGTTGGCTAGTA
TTTTGTCAACGAATAATAAGAGGGGAGAGATTCACAAAGGAAATCAGATTTT
TCTTTCACAGAAGTTTGTGAAATTACTTTATCATGCTAAGAGAATAAGTAACA
CAATTAATGAGAATCATAGAAAATATGTTGAGAACCATAAAAAAGAGTTTGA
AGAATTATTTTACTACATTCTTGAGTTTAATGAGAATTATGTTGGAGCTAAAA
AGAATGGTAAACTTTTAAACTCTGCCTTTCAATCTTGGCAAAATCATAGTATA
GATGAACTCTGTAGTAGTTTTATAGGACCTACCGGAAGTGAAAGAAAGGGGC
TATTTGAATTAACCTCTCGTGGAAGTGCTGCTGATTTTGAATTTTTAGGTGTTA
AAATTCCAAGGTATAGAGACTATACCCCATCATCCCTATTAAAAGATGCCAC
ACTTATTCATCAATCTGTTACAGGCCTCTATGAAACACGAATAGACCTTGCCA
AACTAGGAGAGGGTTAA
**SEQ ID NO: 2**
   SEQUENCE: 
MTKPYSIGLDIGTNSVGWAVITDNYKVPSKKMKVLGNTSKKYIKKNLLGVLLFD
SGITAEGRRLKRTARRRYTRRRNRILYLQEIFSTEMATLDDAFFQRLDDSFLVPDD
KRDSKYPIFGNLVEEKVYHDEFPTIYHLRKYLADSTKKADLRLVYLALAHMIKY
RGHFLIEGEFNSKNNDIQKNFQDFLDTYNAIFESDLSLENSKQLEEIVKDKISKLEK
 
KDRILKLFPGEKNSGIFSEFLKLIVGNQADFRKCFNLDEKASLHFSKESYDEDLET
LLGYIGDDYSDVFLKAKKLYDAILLSGFLTVTDNETEAPLSSAMIKRYNEHKEDL
ALLKEYIRNISLKTYNEVFKDDTKNGYAGYIDGKTNQEDFYVYLKNLLAEFEGA
DYFLEKIDREDFLRKQRTFDNGSIPYQIHLQEMRAILDKQAKFYPFLAKNKERIEK
ILTFRIPYYVGPLARGNSDFAWSIRKRNEKITPWNFEDVIDKESSAEAFINRMTSF
DLYLPEEKVLPKHSLLYETFNVYNELTKVRFIAESMRDYQFLDSKQKKDIVRLYF
KDKRKVTDKDIIEYLHAIYGYDGIELKGIEKQFNSSLSTYHDLLNIINDKEFLDDSS
NEAIIEEIIHTLTIFEDREMIKQRLSKFENIFDKSVLKKLSRRHYTGWGKLSAKLIN
GIRDEKSGNTILDYLIDDGISNRNFMQLIHDDALSFKKKIQKAQIIGDEDKGNIKEV
VKSLPGSPAIKKGILQSIKIVDELVKVMGGRKPESIVVEMARENQYTNQGKSNSQ
QRLKRLEKSLKELGSKILKENIPAKLSKIDNNALQNDRLYLYYLQNGKDMYTGD
DLDIDRLSNYDIDHIIPQAFLKDNSIDNKVLVSSASNRGKSDDFPSLEVVKKRKTF
WYQLLKSKLISQRKFDNLTKAERGGLLPEDKAGFIQRQLVETRQITKHVARLLDE
KFNNKKDENNRAVRTVKIITLKSTLVSQFRKDFELYKVREINDFHHAHDAYLNA
VIASALLKKYPKLEPEFVYGDYPKYNSFRERKSATEKVYFYSNIMNIFKKSISLAD
GRVIERPLIEVNEETGESVWNKESDLATVRRVLSYPQVNVVKKVEEQNHGLDRG
KPKGLFNANLSSKPKPNSNENLVGAKEYLDPKKYGGYAGISNSFAVLVKGTIEK
GAKKKITNVLEFQGISILDRINYRKDKLNFLLEKGYKDIELIIELPKYSLFELSDGSR
RMLASILSTNNKRGEIHKGNQIFLSQKFVKLLYHAKRISNTINENHRKYVENHKK
EFEELFYYILEFNENYVGAKKNGKLLNSAFQSWQNHSIDELCSSFIGPTGSERKGL
FELTSRGSAADFEFLGVKIPRYRDYTPSSLLKDATLIHQSVTGLYETRIDLAKLGE
G
**SEQ ID NO: 3**
   NAME: P.multocida Cas9
   SEQUENCE: 
ATGCAAACAACAAATTTAAGTTATATTTTAGGTTTAGATTTGGGGATCGCTTC
TGTAGGTTGGGCTGTCGTTGAAATCAATGAAAATGAAGACCCTATCGGCTTG
ATTGATGTAGGAGTAAGGATATTTGAGCGTGCTGAGGTACCCAAAACTGGAG
AATCTTTAGCACTCTCTCGCCGTCTTGCAAGAAGTACTCGCCGTTTGATACGC
CGTCGTGCACACCGTTTACTCCTCGCAAAACGCTTCTTAAAACGTGAAGGTAT
ACTTTCCACAATCGACTTAGAAAAAGGATTACCCAACCAAGCTTGGGAATTA
 
CGTGTCGCCGGTCTTGAACGTCGGTTATCCGCCATAGAATGGGGTGCGGTTCT
GCTACATTTAATCAAGCATCGAGGTTATCTTTCTAAACGTAAAAATGAATCCC
AAACAAACAACAAAGAATTAGGAGCCTTACTCTCTGGAGTGGCACAAAACCA
TCAATTATTACAATCAGATGACTACCGAACACCAGCAGAGCTCGCACTGAAA
AAATTTGCTAAAGAAGAAGGGCATATCCGTAATCAACGAGGTGCCTATACAC
ATACATTTAATCGATTAGACTTATTAGCTGAACTTAACTTGCTTTTTGCTCAAC
AACATCAGTTTGGTAACCCTCACTGTAAAGAGCATATTCAACAATATATGAC
AGAATTGCTTATGTGGCAAAAGCCAGCCTTATCTGGTGAGGCAATTTTAAAA
ATGTTGGGTAAATGTACGCATGAAAAAAATGAGTTTAAAGCAGCAAAACATA
CCTACAGTGCGGAGCGCTTTGTTTGGCTAACCAAACTCAATAACTTGCGCATT
TTAGAAGATGGGGCAGAACGAGCTCTTAATGAAGAAGAACGTCAACTATTGA
TAAATCATCCGTATGAGAAATCAAAATTAACCTATGCCCAAGTCAGAAAATT
GTTAGGGCTTTCCGAACAAGCGATTTTTAAGCATCTACGTTATAGTAAAGAA
AACGCAGAATCAGCTACTTTTATGGAGCTTAAAGCTTGGCATGCAATTCGTA
AAGCGTTAGAAAATCAAGGATTGAAGGATACTTGGCAAGATCTCGCTAAGAA
ACCTGACTTACTAGATGAAATTGGTACCGCATTTTCTCTTTATAAAACTGATG
AAGATATTCAGCAATATTTGACAAATAAGGTACCGAACTCAGTCATCAATGC
ATTATTAGTTTCTCTGAATTTCGATAAATTCATTGAGTTATCTTTGAAAAGTTT
ACGTAAAATCTTGCCCCTAATGGAGCAAGGTAAGCGTTATGATCAAGCTTGT
CGTGAAATTTATGGGCATCATTATGGTGAGGCAAATCAAAAAACTTCTCAGC
TACTACCAGCTATTCCAGCCCAAGAAATTCGTAATCCTGTTGTTTTACGTACA
CTTTCACAAGCACGTAAAGTGATCAATGCCATTATTCGTCAATATGGTTCCCC
TGCTCGAGTCCATATTGAAACAGGAAGAGAACTTGGGAAATCTTTTAAAGAA
CGTCGTGAAATTCAAAAACAACAGGAAGATAATCGAACTAAGCGAGAAAGT
GCGGTACAAAAATTCAAAGAATTATTTTCTGACTTTTCAAGTGAACCCAAAA
GTAAAGATATTTTAAAATTCCGCTTATACGAACAACAGCATGGTAAATGCTT
ATACTCTGGAAAAGAGATCAATATTCATCGCTTAAATGAAAAGGGTTATGTG
GAAATTGATCATGCTTTACCTTTCTCACGGACTTGGGATGATAGTTTTAATAA
TAAAGTATTAGTTCTTGCCAGCGAAAACCAAAACAAAGGGAATCAAACACCG
TATGAATGGCTACAAGGTAAAATAAATTCGGAACGTTGGAAAAACTTTGTTG
CTTTAGTACTGGGTAGCCAGTGCAGTGCAGCCAAGAAACAACGATTACTCAC
 
TCAAGTTATTGATGATAATAAATTTATTGATAGAAACTTAAATGATACTCGCT
ATATTGCCCGATTCCTATCCAACTATATTCAAGAAAATTTGCTTTTGGTGGGT
AAAAATAAGAAAAATGTCTTTACACCAAACGGTCAAATTACTGCATTATTAA
GAAGTCGCTGGGGATTAATTAAGGCTCGTGAGAATAATAACCGTCATCATGC
TTTAGATGCGATAGTTGTGGCTTGTGCAACACCTTCTATGCAACAAAAATTA
CCCGATTTATTCGATTTAAAGAAGTGCATCCATACAAAATAGAAAATAGGTA
TGAAATGGTGGATCAAGAAAGCGGAGAAATTATTTCACCTCATTTTCCTGAA
CCTTGGGCTTATTTTAGACAAGAGGTTAATATTCGTGTTTTTGATAATCATCC
AGATACTGTCTTAAAAGAGATGCTACCTGATCGCCCACAAGCAAATCACCAG
TTTGTACAGCCCCTTTTTGTTTCTCGTGCCCCAACTCGTAAAATGAGTGGTCA
AGGGCATATGGAAACAATTAAATCAGCTAAACGCTTAGCAGAAGGCATTAGC
GTTTTAAGAATTCCTCTCACGCAATTAAAACCTAATTTATTGGAAAATATGGT
GAATAAAGAACGTGAGCCAGCACTTTATGCAGGACTAAAAGCACGCTTGGCT
GAATTTAATCAAGATCCAGCAAAAGCGTTTGCTACGCCTTTTTATAAACAAG
GAGGGCAGCAGGTCAAAGCTATTCGTGTTGAACAGGTACAAAAATCAGGGGT
ATTAGTCAGAGAAAACAATGGGGTAGCAGATAATGCCTCTATCGTTCGAACA
GACGTATTTATCAAAAATAATAAATTTTTCCTTGTTCCTATCTATACTTGGCA
AGTTGCGAAAGGCATCTTGCCAAATAAAGCTATTGTTGCTCATAAAAATGAA
GATGAATGGGAAGAAATGGATGAAGGTGCTAAGTTTAAATTCAGCCTTTTCC
CGAATGATCTTGTCGAGCTAAAAACCAAAAAAGAATACTTTTTCGGCTATTA
CATCGGACTAGATCGTGCAACTGGAAACATTAGCCTAAAAGAACATGATGGT
GAGATATCAAAAGGTAAAGACGGTGTTTACCGTGTTGGTGTCAAGTTAGCTC
TTTCTTTTGAAAAATATCAAGTTGATGAGCTCGGTAAAAATAGACAAATTTGC
CGACCTCAGCAAAGACAACCTGTGCGTTAA
**SEQ ID NO: 4**
   SEQUENCE: 
MQTTNLSYILGLDLGIASVGWAVVEINENEDPIGLIDVGVRIFERAEVPKTGESLA
LSRRLARSTRRLIRRRAHRLLLAKRFLKREGILSTIDLEKGLPNQAWELRVAGLER
RLSAIEWGAVLLHLIKHRGYLSKRKNESQTNNKELGALLSGVAQNHQLLQSDDY
RTPAELALKKFAKEEGHIRNQRGAYTHTFNRLDLLAELNLLFAQQHQFGNPHCK
EHIQQYMTELLMWQKPALSGEAILKMLGKCTHEKNEFKAAKHTYSAERFVWLT
 
KLNNLRILEDGAERALNEEERQLLINHPYEKSKLTYAQVRKLLGLSEQAIFKHLR
YSKENAESATFMELKAWHAIRKALENQGLKDTWQDLAKKPDLLDEIGTAFSLY
KTDEDIQQYLTNKVPNSVINALLVSLNFDKFIELSLKSLRKILPLMEQGKRYDQAC
REIYGHHYGEANQKTSQLLPAIPAQEIRNPVVLRTLSQARKVINAIIRQYGSPARV
HIETGRELGKSFKERREIQKQQEDNRTKRESAVQKFKELFSDFSSEPKSKDILKFR
LYEQQHGKCLYSGKEINIHRLNEKGYVEIDHALPFSRTWDDSFNNKVLVLASEN
QNKGNQTPYEWLQGKINSERWKNFVALVLGSQCSAAKKQRLLTQVIDDNKFID
RNLNDTRYIARFLSNYIQENLLLVGKNKKNVFTPNGQITALLRSRWGLIKARENN
NRHHALDAIVVACATPSMQQKITRFIRFKEVHPYKIENRYEMVDQESGEIISPHFP
EPWAYFRQEVNIRVFDNHPDTVLKEMLPDRPQANHQFVQPLFVSRAPTRKMSG
QGHMETIKSAKRLAEGISVLRIPLTQLKPNLLENMVNKEREPALYAGLKARLAEF
NQDPAKAFATPFYKQGGQQVKAIRVEQVQKSGVLVRENNGVADNASIVRTDVFI
KNNKFFLVPIYTWQVAKGILPNKAIVAHKNEDEWEEMDEGAKFKFSLFPNDLVE
LKTKKEYFFGYYIGLDRATGNISLKEHDGEISKGKDGVYRVGVKLALSFEKYQV
DELGKNRQICRPQQRQPVR
**SEQ ID NO: 5**
   NAME: S.mutans Cas9
   SEQUENCE: 
ATGAAAAACCTTACTCTATTGGACTTGATATTGGAACCAATTCTGTTGGTTG
GGCTGTTGTGACAGATGACTACAAAGTTCCTGCTAAGAAGATGAAGGTTCTG
GGAAATACAGATAAAAGTCATATCGAGAAAAATTTGCTTGGCGCTTTATTAT
TTGATAGCGGGAATACTGCAGAAGACAGACGGTTAAAGAGAACTGCTCGCCG
TCGTTACACACGTCGCAGAAATCGTATTTATATTTGCAAGAGATTTTTCAG
AAGAAATGGGCAAGGTAGATGATAGTTTCTTTCATCGTTTAGAGGATTCTTTT
CTTGTTACTGAGGATAAACGAGGAGCGCCATCCCATTTTTGGGAATCTTG
AAGAAGAAGTTAAGTATCATGAAAATTTTCCAACCATTTATCATTTGCGGCA
ATATCTTGCGGATAATCCAGAAAAAGTTGATTTGCGTTTAGTTTATTTGGCTT
TGGCACATATAATTAAGTTTAGAGGTCATTTTTTAATTGAAGGAAAGTTTGAT
ACACGCAATAATGATGTACAAAGACTGTTTCAAGAATTTTTAGCAGTCTATG
ATAATACTTTTGAGAATAGTTCGCTTCAGGAGCAAAATGTTCAAGTTGAAGA
AATTCTGACTGATAAAATCAGTAAATCTGCTAAGAAAGATAGAGTTTTGAAA
 
CTTTTTCCTAATGAAAAGTCTAATGGCCGCTTTGCAGAATTTCTAAAACTAAT
TGTTGGTAATCAAGCTGATTTAAAAAGCATTTTGAATTAGAAGAGAAAGCA
CCATTGCAATTTCTAAAGATACTTATGAAGAAGAGTTAGAAGTACTATTAGC
TCAAATTGGAGATAATTACGCAGAGCTCTTTTTATCAGCAAAGAAACTGTAT
GATAGTATCCTTTTATCAGGGATTTTAACAGTTACTGATGTTGGTACCAAAGC
GCCTTTATCTGCTTCGATGATTCAGCGATATAATGAACATCAGATGGATTTAG
CTCAGCTTAAACAATTCATTCGTCAGAAATTATCAGATAAATATAACGAAGTT
TTTTCTGATGTTTCAAAAGACGGCTATGCGGGTTATATTGATGGAAAACAA
ATCAAGAAGCTTTTTATAAATACCTTAAAGGTCTATTAAATAAGATTGAGGG
AAGTGGCTATTTCCTTGATAAAATTGAGCGTGAAGATTTCTAAGAAAGCAA
CGTACCTTTGACAATGGCTCTATTCCACATCAGATTCATCTTCAAGAAATGCG
TGCTATCATTCGTAGACAGGCTGAATTTTATCCGTTTTTAGCAGACAATCAAG
ATAGGATTGAGAAATTATTGACTTTCCGTATTCCCTACTATGTTGGTCCATTA
GCGCGCGGAAAAAGTGATTTTGCTTGGTTAAGTCGGAAATCGGCTGATAAAA
TTACACCATGGAATTTTGATGAAATCGTTGATAAAGAATCCTCTGCAGAAGCT
TTTATCAATCGTATGACAAATTATGATTTGTACTTGCCAAATCAAAAAGTTCT
TCCTAAACATAGTTTATTATACGAAAAATTTACTGTTTACAATGAATTAACAA
AGGTTAAATATAAAACAGAGCAAGGAAAAACAGCATTTTTTGATGCCAATAT
GAAGCAAGAAATCTTTGATGGCGTATTTAAGGTTTATCGAAAAGTAACTAAA
GATAAATTAATGGATTTCCTTGAAAAAGAATTTGATGAATTTCGTATTGTTGA
TTTAACAGGTCTGGATAAAGAAAATAAAGTATTTAACGCTTCTTATGGAACTT
ATCATGATTTGTGTAAAATTTTAGATAAAGATTTTCTCGATAATTCAAAGAAT
GAAAAGATTTTAGAAGATATTGTGTTGACCTTAACGTTATTTGAAGATAGAG
AAATGATTAGAAAACGTCTAGAAAATTACAGTGATTTATTGACCAAAGAACA
AGTGAAAAAGCTGGAAAGACGTCATTATACTGGTTGGGGAAGATTATCAGCT
GAGTTAATTCATGGTATTCGCAATAAAGAAAGCAGAAAAACAATTCTTGATT
ATCTCATTGATGATGGCAATAGCAATCGGAACTTTATGCAACTGATTAACGAT
GATGCTCTTTCTTTCAAAGAAGAGATTGCTAAGGCACAAGTTATTGGAGAAA
CAGACAATCTAAATCAAGTTGTTAGTGATATTGCTGGCAGCCCTGCTATTAAA
AAAGGAATTTTACAAAGCTTGAAGATTGTTGATGAGCTTGTCAAAATTATGG
GACATCAACCTGAAAATATCGTCGTGGAGATGGCGCGTGAAAACCAGTTTAC
 
CAATCAGGGACGACGAAATTCACAGCAACGTTTGAAAGGTTTGACAGATTCT
ATTAAAGAATTTGGAAGTCAAATTCTTAAAGAACATCCGGTTGAGAATTCAC
AGTTACAAAATGATAGATTGTTTCTATATTATTTACAAAACGGCAGAGATATG
TATACTGGAGAAGAATTGGATATTGATTATCTAAGCCAGTATGATATAGACC
ATATTATCCCGCAAGCTTTTATAAAGGATAATTCTATTGATAATAGAGTATTG
ACTAGCTCAAAGGAAAATCGTGGAAAATCGGATGATGTACCAAGTAAAGAT
GTTGTTCGTAAAATGAAATCCTATTGGAGTAAGCTACTTTCGGCAAAGCTTAT
TACACAACGTAAATTTGATAATTTGACAAAAGCTGAACGAGGTGGATTGACC
GACGATGATAAAGCTGGATTCATCAAGCGTCAATTAGTAGAAACACGACAAA
TTACCAAACATGTAGCACGTATTCTGGACGAACGATTTAATACAGAAACAGA
TGAAAACAACAAGAAAATTCGTCAAGTAAAAATTGTGACCTTGAAATCAAAT
CTTGTTTCCAATTTCCGTAAAGAGTTTGAACTCTACAAAGTGCGTGAAATTAA
TGACTATCATCATGCACATGATGCCTATCTCAATGCTGTAATTGGAAAGGCTT
TACTAGGTGTTTACCCACAATTGGAACCTGAATTTGTTTATGGTGATTATCCT
CATTTTCATGGACATAAAGAAAATAAAGCAACTGCTAAGAAATTTTCTATTC
AAATATTATGAACTTCTTTAAAAAAGATGATGTCCGTACTGATAAAAATGGT
GAAATTATCTGGAAAAAAGATGAGCATATTTCTAATATTAAAAAAGTGCTTT
CTTATCCACAAGTTAATATTGTTAAGAAAGTAGAGGAGCAAACGGGAGGATT
TTCTAAAGAATCTATCTTGCCGAAAGGTAATTCTGACAAGCTTATTCCTCGAA
AAACGAAGAAATTTTATTGGGATACCAAGAAATATGGAGGATTTGATAGCCC
GATTGTTGCTTATTCTATTTTAGTTATTGCTGATATTGAAAAAGGTAAATCTA
AAAAATTGAAAACAGTCAAAGCCTTAGTTGGTGTCACTATTATGGAAAAGAT
GACTTTTGAAAGGGATCCAGTTGCTTTTCTTGAGCGAAAAGGCTATCGAAAT
GTTCAAGAAGAAAATATTATAAAGTTACCAAAATATAGTTTATTTAAACTAG
AAAACGGACGAAAAAGGCTATTGGCAAGTGCTAGGGAACTTCAAAAGGAA
ATGAAATCGTTTTGCCAAATCATTTAGGAACCTTGCTTTATCACGCTAAAAAT
ATTCATAAAGTTGATGAACCAAAGCATTTGGACTATGTTGATAAACATAAAG
ATGAATTTAAGGAGTTGCTAGATGTTGTGTCAAACTTTTCTAAAAAATATACT
TTAGCAGAAGGAAATTTAGAAAAAATCAAAGAATTATATGCACAAAATAATG
GTGAAGATCTTAAAGAATTAGCAAGTTCATTTATCAACTTATTAACATTTACT
GCTATAGGAGCACCGGCTACTTTTAAATTCTTTGATAAAAATATTGATCGAAA
 
ACGATATACTTCAACTACTGAAATTCTCAACGCTACCCTCATCCACCAATCCA
TCACCGGTCTTTATGAAACGCGGATTGATCTCAATAAGTTAGGAGGAGACTA
A
**SEQ ID NO: 6**
   SEQUENCE: 
MKKPYSIGLDIGTNSVGWAVVTDDYKVPAKKMKVLGNTDKSHIEKNLLGALLF
DSGNTAEDRRLKRTARRRYTRRRNRILYLQEIFSEEMGKVDDSFFHRLEDSFLVT
EDKRGERHPIFGNLEEEVKYHENFPTIYHLRQYLADNPEKVDLRLVYLALAHIIKF
RGHFLIEGKFDTRNNDVQRLFQEFLAVYDNTFENSSLQEQNVQVEEILTDKISKS
AKKDRVLKLFPNEKSNGRFAEFLKLIVGNQADFKKHFELEEKAPLQFSKDTYEEE
LEVLLAQIGDNYAELFLSAKKLYDSILLSGILTVTDVGTKAPLSASMIQRYNEHQ
MDLAQLKQFIRQKLSDKYNEVFSDVSKDGYAGYIDGKTNQEAFYKYLKGLLNKI
EGSGYFLDKIEREDFLRKQRTFDNGSIPHQIHLQEMRAIIRRQAEFYPFLADNQDR
IEKLLTFRIPYYVGPLARGKSDFAWLSRKSADKITPWNFDEIVDKESSAEAFINRM
TNYDLYLPNQKVLPKHSLLYEKFTVYNELTKVKYKTEQGKTAFFDANMKQEIFD
GVFKVYRKVTKDKLMDFLEKEFDEFRIVDLTGLDKENKVFNASYGTYHDLCKIL
DKDFLDNSKNEKILEDIVLTLTLFEDREMIRKRLENYSDLLTKEQVKKLERRHYT
GWGRLSAELIHGIRNKESRKTILDYLIDDGNSNRNFMQLINDDALSFKEEIAKAQ
VIGETDNLNQVVSDIAGSPAIKKGILQSLKIVDELVKIMGHQPENIVVEMARENQF
TNQGRRNSQQRLKGLTDSIKEFGSQILKEHPVENSQLQNDRLFLYYLQNGRDMY
TGEELDIDYLSQYDIDHIIPQAFIKDNSIDNRVLTSSKENRGKSDDVPSKDVVRKM
KSYWSKLLSAKLITQRKFDNLTKAERGGLTDDDKAGFIKRQLVETRQITKHVARI
LDERFNTETDENNKKIRQVKIVTLKSNLVSNFRKEFELYKVREINDYHHAHDAYL
NAVIGKALLGVYPQLEPEFVYGDYPHFHGHKENKATAKKFFYSNIMNFFKKDDV
RTDKNGEIIWKKDEHISNIKKVLSYPQVNIVKKVEEQTGGFSKESILPKGNSDKLIP
RKTKKFYWDTKKYGGFDSPIVAYSILVIADIEKGKSKKLKTVKALVGVTIMEKM
TFERDPVAFLERKGYRNVQEENIIKLPKYSLFKLENGRKRLLASARELQKGNEIVL
PNHLGTLLYHAKNIHKVDEPKHLDYVDKHKDEFKELLDVVSNFSKKYTLAEGN
LEKIKELYAQNNGEDLKELASSFINLLTFTAIGAPATFKFFDKNIDRKRYTSTTEIL
NATLIHQSITGLYETRIDLNKLGGD
**SEQ ID NO: 7**
   NAME: N.meningitides Cas9
   SEQUENCE: 
ATGGCTGCCTTCAAACCTAATTCAATCAACTACATCCTCGGCCTCGATATCGG
CATCGCATCCGTCGGCTGGGCGATGGTAGAAATTGACGAAGAAAACCCC
ATCCGCCTGATTGATTTGGGCGTGCGCGTATTTGAGCGTGCCGAAGTACCGA
AAACAGGCGACTCCCTTGCCATGGCAAGGCGTTTGGCGCGCAGTGTTCGCCG
CCTGACCCGCCGTCGCGCCCACCGCCTGCTTCGGACCCGCCGCCTATTGAAAC
GCGAAGGCGTATTACAAGCCGCCAATTTTGACGAAAACGGCTTGATTAAATC
CTTACCGAATACACCATGGCAACTTCGCGCAGCCGCATTAGACCGCAAACTG
ACGCCTTTAGAGTGGTCGGCAGTCTTGTTGCATTTAATCAAACATCGCGGCTA
TTTATCGCAACGGAAAAACGAGGGCGAAACTGCCGATAAGGAGCTTGGCGCT
TTGCTTAAAGGCGTAGCCGGCAATGCCCATGCCTTACAGACAGGCGATTTCC
GCACACCGGCCGAATTGGCTTTAAATAAATTTGAGAAAGAAAGCGGCCATAT
CCGCAATCAGCGCAGCGATTATTCGCATACGTTCAGCCGCAAAGATTTACAG
GCGGAGCTGATTTTGCTGTTTGAAAAACAAAAAGAATTTGGCAATCCGCATG
TTTCAGGCGGCCTTAAAGAAGGTATTGAAACCCTACTGATGACGCAACGCCC
TGCCCTGTCCGGCGATGCCGTTCAAAAAATGTTGGGGCATTGCACCTTCGAAC
CGGCAGAGCCGAAAGCCGCTAAAAACACCTACACAGCCGAACGTTTCATCTG
GCTGACCAAGCTGAACAACCTGCGTATTTTAGAGCAAGGCAGCGAGCGGCCA
TTGACCGATACCGAACGCGCCACGCTTATGGACGAGCCATACAGAAAATCCA
AACTGACTTACGCACAAGCCCGTAAGCTGCTGGGTTTAGAAGATACCGCCTT
TTTCAAAGGCTTGCGCTATGGTAAAGACAATGCCGAAGCCTCAACATTGATG
GAAATGAAGGCCTACCATGCCATCAGCCGTGCACTGGAAAAAGAAGGATTG
AAAGACAAAAAATCCCCATTAAACCTTTCTCCCGAATTACAAGACGAAATCG
GCACGGCATTCTCCCTGTTCAAAACCGATGAAGACATTACAGGCCGTCTGAA
AGACCGTATACAGCCCGAAATCTTAGAAGCGCTGTTGAAACACATCAGCTTC
GATAAGTTCGTCCAAATTTCCTTGAAAGCATTGCGCCGAATTGTGCCTCTAAT
GGAACAAGGCAAACGTTACGATGAAGCCTGCGCCGAAATCTACGGAGACCA
TTACGGCAAGAAGAATACGGAAGAAAAGATTTATCTGCCGCCGATTCCCGCC
GACGAAATCCGCAACCCCGTCGTCTTGCGCGCCTTATCTCAAGCACGTAAGG
TCATTAACGGCGTGGTACGCCGTTACGGCTCCCCAGCTCGTATCCATATTGAA
 
ACTGCAAGGGAAGTAGGTAAATCGTTTAAAGACCGCAAAGAAATTGAGAAA
CGCCAAGAAGAAAACCGCAAAGACCGGAAAAAGCCGCCGCCAAATTCCGA
GAGTATTTCCCCAATTTTGTCGGAGAACCCAAATCCAAAGATATTCTGAAACT
GCGCCTGTACGAGCAACAACACGGCAAATGCCTGTATTCGGGCAAAGAAATC
AACTTAGGCCGTCTGAACGAAAAAGGCTATGTCGAAATCGACCATGCCCTGC
CGTTCTCGCGCACATGGGACGACAGTTTCAACAATAAAGTACTGGTATTGGG
CAGCGAAAACCAAAACAAAGGCAATCAAACCCCTTACGAATACTTCAACGG
CAAAGACAACAGCCGCGAATGGCAGGAATTTAAAGCGCGTGTCGAAACCAG
CCGTTTCCCGCGCAGTAAAAAACAACGGATTCTGCTGCAAAAATTCGATGAA
GACGGCTTTAAAGAACGCAATCTGAACGACACGCGCTACGTCAACCGTTTCC
TGTGTCAATTTGTTGCCGACCGTATGCGGCTGACAGGTAAAGGCAAGAAACG
TGTCTTTGCATCCAACGGACAAATTACCAATCTGTTGCGCGGCTTTTGGGGAT
TGCGCAAAGTGCGTGCGGAAAACGACCGCCATCACGCCTTGGACGCCGTCGT
CGTTGCCTGCTCGACCGTTGCCATGCAGCAGAAAATTACCCGTTTTGTACGCT
ATAAAGAGATGAACGCGTTTGACGGTAAAACCATAGACAAAGAAACAGGAG
AAGTGCTGCATCAAAAAACACACTTCCCACAACCTTGGGAATTTTTCGCACA
AGAAGTCATGATTCGCGTCTTCGGCAAACCGGACGGCAAACCCGAATTCGAA
GAAGCCGATACCCTAGAAAAACTGCGCACGTTGCTTGCCGAAAAATTATCAT
CTCGCCCCGAAGCCGTACACGAATACGTTACGCCACTGTTTGTTTCACGCGCG
CCCAATCGGAAGATGAGCGGGCAAGGGCATATGGAGACCGTCAAATCCGCC
AAACGACTGGACGAAGGCGTCAGCGTGTTGCGCGTACCGCTGACACAGTTAA
AACTGAAAGACTTGGAAAAAATGGTCAATCGGGAGCGCGAACCTAAGCTAT
ACGAAGCACTGAAAGCACGGCTGGAAGCACATAAAGACGATCCTGCCAAAG
CCTTTGCCGAGCCGTTTTACAAATACGATAAAGCAGGCAACCGCACCCAACA
GGTAAAAGCCGTACGCGTAGAGCAAGTACAGAAAACCGGCGTATGGGTGCG
CAACCATAACGGTATTGCCGACAACGCAACCATGGTGCGCGTAGATGTGTTT
GAGAAAGGCGACAAGTATTATCTGGTACCGATTTACAGTTGGCAGGTAGCGA
AAGGGATTTTGCCGGATAGGGCTGTTGTACAAGGAAAAGATGAAGAAGATTG
GCAACTTATTGATGATAGTTTCAACTTTAAATTCTCATTACACCCTAATGATTT
AGTCGAGGTTATAACAAAAAAAGCTAGAATGTTTGGTTACTTTGCCAGCTGC
CATCGAGGCACAGGTAATATCAATATACGCATTCATGATCTTGATCATAAAA
 
TTGGCAAAAATGGAATACTGGAAGGTATCGGCGTCAAAACCGCCCTTTCATT
CCAAAAATACCAAATTGACGAACTGGGCAAAGAAATCAGACCATGCCGTCTG
AAAAAACGCCCGCCTGTCCGTTAA
**SEQ ID NO: 8**
   SEQUENCE: 
MAAFKPNSINYILGLDIGIASVGWAMVEIDEEENPIRLIDLGVRVFERAEVPKTGD
SLAMARRLARSVRRLTRRRAHRLLRTRRLLKREGVLQAANFDENGLIKSLPNTP
WQLRAAALDRKLTPLEWSAVLLHLIKHRGYLSQRKNEGETADKELGALLKGVA
GNAHALQTGDFRTPAELALNKFEKESGHIRNQRSDYSHTFSRKDLQAELILLFEK
QKEFGNPHVSGGLKEGIETLLMTQRPALSGDAVQKMLGHCTFEPAEPKAAKNTY
TAERFIWLTKLNNLRILEQGSERPLTDTERATLMDEPYRKSKLTYAQARKLLGLE
DTAFFKGLRYGKDNAEASTLMEMKAYHAISRALEKEGLKDKKSPLNLSPELQDE
IGTAFSLFKTDEDITGRLKDRIQPEILEALLKHISFDKFVQISLKALRRIVPLMEQGK
RYDEACAEIYGDHYGKKNTEEKIYLPPIPADEIRNPVVLRALSQARKVINGVVRR
YGSPARIHIETAREVGKSFKDRKEIEKRQEENRKDREKAAAKFREYFPNFVGEPK
SKDILKLRLYEQQHGKCLYSGKEINLGRLNEKGYVEIDHALPFSRTWDDSFNNK
VLVLGSENQNKGNQTPYEYFNGKDNSREWQEFKARVETSRFPRSKKQRILLQKF
DEDGFKERNLNDTRYVNRFLCQFVADRMRLTGKGKKRVFASNGQITNLLRGFW
GLRKVRAENDRHHALDAVVVACSTVAMQQKITRFVRYKEMNAFDGKTIDKETG
EVLHQKTHFPQPWEFFAQEVMIRVFGKPDGKPEFEEADTLEKLRTLLAEKLSSRP
EAVHEYVTPLFVSRAPNRKMSGQGHMETVKSAKRLDEGVSVLRVPLTQLKLKD
LEKMVNREREPKLYEALKARLEAHKDDPAKAFAEPFYKYDKAGNRTQQVKAV
RVEQVQKTGVWVRNHNGIADNATMVRVDVFEKGDKYYLVPIYSWQVAKGILP
DRAVVQGKDEEDWQLIDDSFNFKFSLHPNDLVEVITKKARMFGYFASCHRGTG
NINIRIHDLDHKIGKNGILEGIGVKTALSFQKYQIDELGKEIRPCRLKKRPPVR
**SEQ** ID NO: 9
   SEQUENCE: 
ATGAACAATAACAATTACTCTATCGGACTCGATATCGGAACAAACAGCGTCG
GATGGGCCGTCATTACGGATGACTATAAGGTGCCATCGAAAAAGATGAAAGT
TCTAGGCAATACAGATAAACACTTTATCAAGAAAAATCTAATTGGAGCTTTA
TTATTTGATGAAGGAGCTACTGCTGAAGATAGACGTTTCAAACGAACAGCAC
 
GCCGTCGCTATACTCGTCGAAAAAATCGTCTTCGCTATCTTCAAGAAATCTTT
TCTGAGGAAATGAGCAAAGTGGATAGTAGTTTCTTTCATCGATTAGATGACTC
ATTCTTAGTTCCTGAGGATAAAAGAGGAAGTAAATATCCTATTTTTGCTACCT
TGGCAGAAGAAAAAGAATATCACAAGAAATTTCCAACTATCTATCATTTGAG
AAAACACCTTGCGGACTCAAAAGAAAAACTGACTTGCGCTTGATCTATCTA
GCATTAGCGCATATGATTAAATACCGCGGACATTTTTTGTATGAAGAATCTTT
CGATATTAAAAACAATGATATCCAAAAAATCTTTAGCGAGTTTATAAGCATTT
ACGACAACACCTTTGAAGGAAGTTCACTTAGTGGACAAAATGCACAAGTAGA
AGCAATTTTTACTGATAAAATTAGTAAATCTGCTAAGAGAGAACGCATTCTA
AAACTCTTTGCTTATGAAAAATCCACTGATCTATTTTCAGAATTTCTCAAGCT
GATTGTAGGAAATCAAGCTGATTTTAAGAAACACTTTGACTTGGAAGAAAAA
GCTCCACTACAATTCTCTAAAGATACCTATGATGAGGATTTGGAAAACTTACT
CGGACAAATTGGAGATGACTTTGCAGACCTTTTCCTAGTTGCTAAAAAACTCT
ATGATGCCATTCTTTTATCAGGAATCTTAACTGTTACAGATTCTTCAACTAAG
GCCCCACTATCAGCATCTATGATTGAGCGCTATGAAAACCACCAAAAAGACT
TAGCGGCTTTAAAACAATTCATCCAAAACAATCTTCAAGAAAAATATGATGA
AGTTTTCTCTGACCAATCTAAAGATGGGTATGCTAGGTATATCAATGGCAAA
ACCACTCAAGAAGCATTTTACAAGTACATCAAAAATCTTCTCTCTAAATTCGA
AGGATCAGATTATTTCCTTGATAAAATTGAACGTGAAGATTTCTTGAGAAAA
CAACGCACCTTTGATAATGGTTCTATCCCTCATCAAATTCATCTTCAAGAAAT
GAATGCCATTATCCGTCGGCAAGGAGAACATTATCCATTTCTGAAGGAATAT
AAAGAAAAGATAGAGACAATCTTGACTTTCCGTATTCCTTATTATGTTGGCCC
ATTGGCTCGTGGAAATCGTAATTTTGCTTGGCTTACTCGAAACTCTGACCAAG
CAATCCGACCTTGGAATTTTGAAGAAATTGTTGATCAAGCAAGCTCTGCGGA
AGAATTCATCAATAAGATGACTAACTATGACTTGTATCTGCCAGAGGAAAAA
GTTTTGCCCAAGCATAGTCTCTTGTATGAAACATTTGCTGTCTACAATGAATT
AACAAAAGTAAAATTTATTTCAGAGGGATTGAGAGACTATCAATTCCTTGAT
AGTGGGCAAAAGAAGCAAATTGTCAATCAATTATTCAAAGAGAAAAGAAAA
GTAACTGAAAAAGACATCATTCAGTATCTACACAATGTTGATGGCTACGATG
GAATCGAACTAAAAGGAATTGAAAAACAATTTAACGCTAGTCTTTCTACTTA
TCATGATTTACTCAAAATAATCAAGGATAAAGAGTTTATGGATGATCCTAAA
 
AATGAAGAGATTCTTGAAAATATCGTCCACACACTAACTATCTTTGAAGATC
GTGAGATGATCAAGCAACGCCTTGCTCAATATGCCTCTATCTTTGATAAAAAA
GTGATCAAGGCACTGACTCGTCGACATTATACTGGTTGGGGAAAACTCTCTG
CTAAGCTAATCAACGGTATCTGTGATAAAAAAACTGGTAAAACAATTCTTGA
CTACTTGATTGATGACGGCTACAGCAATCGTAACTTTATGCAGTTAATCAATG
ATGACGGGCTTTCCTTCAAAGATATTATTCAAAAAGCACAAGTGGTTGGTAA
GACAAACGATGTGAAGCAAGTTGTCCAAGAACTCCCAGGTAGTCCTGCTATT
AAAAAGGGAATTTACAAAGTATCAAGCTTGTCGATGAGCTTGTCAAAGTTA
TGGGCCATGCTCCCGAGTCCATTGTGATTGAAATTGCACGAGAAAATCAGAC
AACTGCCAGAGGGAAAAAGAATTCTCAACAAAGATATAAGCGCATTGAAGA
TGCACTAAAAAATTTAGCACCTGGGCTTGATTCAAATATATTAAAAGAACAT
CCAACAGATAATATTCAACTTCAAAATGACCGTCTCTTCCTTTACTATCTCCA
AAATGGAAGGATATGTACACTGGAGAAGCTCTTGATATCAACCAACTGAGC
AGCTATGACATTGACCACATCGTCCCACAGGCCTTTATCAAGGATGATTCTCT
TGATAACCGTGTCTTGACTAGTTCAAAGGATAATCGTGGAAATCCGATAAT
GTTCCAAGTTTAGAAGTCGTTCAAAAAGAAAAGCTTTTTGGCAACAATTAC
TAGATTCCAAATTGATTTCAGAACATAAATTTAATAATTTAACCAAGGCTGAA
CGTGGTGGGCTAGATGAGCGAGATAAAGTTGGCTTTATCAGACGCCAACTAG
TTGAAACACGGCAAATCACAAAACATGTTGCTCAGATTTTGGATGCCCGTTTT
AATACAGAAGTGAATGAGAAAGATAAGAAGAACCGTACCGTCAAAATTATC
ACTTTGAAATCCAATCTAGTTTCCAACTTCCGTAAAGAATTTAAGTTATATAA
GGTACGCGAAATCAATGACTACCACCATGCACATGATGCCTATTTAAATGCA
GTGGTGGCTAAGGCTATCCTTAAGAAATATCCTAAACTAGAGCCTGAATTCG
TCTATGGTGACTATCAAAAGTACGATATTAAGAGATATATTTCCAGATCCAA
AGATCCTAAAGAAGTTGAAAAAGCAACTGAAAAGTATTTCTTCTACTCAAAC
TTGTTGAACTTCTTTAAAGAAGAGGTGCATTACGCAGACGGAACCATCGTAA
AACGAGAGAATATCGAATACTCTAAGGACACTGGAGAAATCGCTTGGAATAA
AGAAAAAGATTTCGCTACAATTAAAAAAGTTCTTTCACTTCCGCAGGTGAAT
ATTGTGAAGAAAACAGAGATTCAAACACATGGTCTAGATAGAGGTAAACCTA
GAGGATTGTTCAATTCCAATCCATCTCCTAAACCTTCAGAAGATCGTAAAGA
AAACCTTGTCCCAATTAAACAAGGGCTTGACCCACGAAAATACGGTGGTTAC
 
GCTGGTATTTCTAACTCATACGCGGTCTTAGTTAAAGCTATTATTGAAAAAGG
AGCGAAAAACAACAAAAGACCGTTCTTGAATTTCAAGGTATCTCTATTTTA
GATAAAATAAATTTTGAAAAGAACAAAGAAAACTATCTTCTTGAAAAAGGAT
ACATAAAAATTCTATCAACTATTACTTTACCTAAATATAGTTTGTTTGAGTTTC
CTGATGGTACAAGAAGAAGACTAGCAAGTATTCTATCGACAAACAATAAACG
AGGAGAAATTCATAAAGGTAATGAATTGGTCATCCCTGAAAAGTATACGACT
CTTTTGTATCATGCTAAGAATATTAATAAAACACTTGAACCAGAACACTTAGA
GTATGTTGAGAAACATCGAAATGATTTTGCTAAACTTTTAGAATATGTACTTA
ACTTTAACGATAAGTATGTAGGCGCATTAAAAAATGGAGAAAGAATCAGACA
AGCATTTATTGATTGGGAAACAGTTGATATTGAAAAGTTATGTTTCAGTTTCA
TTGGTCCAAGAAATAGTAAAAATGCTGGTTTATTCGAGTTAACTTCACAAGG
AAGTGCTTCTGACTTCGAGTTCTTGGGAGTAAAAATTCCACGATACAGAGAC
TATACACCTTCGTCACTCCTCAACGCCACCCTCATCCACCAATCCATCACTGG
TCTTTACGAGACTCGGATTGACTTAAGCAAACTGGGAGAAGACTGA
**SEQ ID NO: 10**
   NAME: gi|777888062|gb|KJQ69483.1| CRISPR-associated endonuclease Cas9
   [Streptococcus mitis]
   SEQUENCE: 
MNNNNYSIGLDIGTNSVGWAVITDDYKVPSKKMKVLGNTDKHFIKKNLIGALLF
DEGATAEDRRFKRTARRRYTRRKNRLRYLQEIFSEEMSKVDSSFFHRLDDSFLVP
EDKRGSKYPIFATLAEEKEYHKKFPTIYHLRKHLADSKEKTDLRLIYLALAHMIK
YRGHFLYEESFDIKNNDIQKIFSEFISIYDNTFEGSSLSGQNAQVEAIFTDKISKSAK
RERILKLFAYEKSTDLFSEFLKLIVGNQADFKKHFDLEEKAPLQFSKDTYDEDLEN
LLGQIGDDFADLFLVAKKLYDAILLSGILTVTDSSTKAPLSASMIERYENHQKDLA
ALKQFIQNNLQEKYDEVFSDQSKDGYARYINGKTTQEAFYKYIKNLLSKFEGSD
YFLDKIEREDFLRKQRTFDNGSIPHQIHLQEMNAIIRRQGEHYPFLKEYKEKIETIL
TFRIPYYVGPLARGNRNFAWLTRNSDQAIRPWNFEEIVDQASSAEEFINKMTNYD
LYLPEEKVLPKHSLLYETFAVYNELTKVKFISEGLRDYQFLDSGQKKQIVNQLFK
EKRKVTEKDIIQYLHNVDGYDGIELKGIEKQFNASLSTYHDLLKIIKDKEFMDDP
KNEEILENIVHTLTIFEDREMIKQRLAQYASIFDKKVIKALTRRHYTGWGKLSAKL
INGICDKKTGKTILDYLIDDGYSNRNFMQLINDDGLSFKDIIQKAQVVGKTNDVK
 
QVVQELPGSPAIKKGILQSIKLVDELVKVMGHAPESIVIEIARENQTTARGKKNSQ
QRYKRIEDALKNLAPGLDSNILKEHPTDNIQLQNDRLFLYYLQNGKDMYTGEAL
DINQLSSYDIDHIVPQAFIKDDSLDNRVLTSSKDNRGKSDNVPSLEVVQKRKAFW
QQLLDSKLISEHKFNNLTKAERGGLDERDKVGFIRRQLVETRQITKHVAQILDAR
FNTEVNEKDKKNRTVKIITLKSNLVSNFRKEFKLYKVREINDYHHAHDAYLNAV
VAKAILKKYPKLEPEFVYGDYQKYDIKRYISRSKDPKEVEKATEKYFFYSNLLNF
FKEEVHYADGTIVKRENIEYSKDTGEIAWNKEKDFATIKKVLSLPQVNIVKKTEIQ
THGLDRGKPRGLFNSNPSPKPSEDRKENLVPIKQGLDPRKYGGYAGISNSYAVLV
KAIIEKGAKKQQKTVLEFQGISILDKINFEKNKENYLLEKGYIKILSTITLPKYSLFE
FPDGTRRRLASILSTNNKRGEIHKGNELVIPEKYTTLLYHAKNINKTLEPEHLEYV
EKHRNDFAKLLEYVLNFNDKYVGALKNGERIRQAFIDWETVDIEKLCFSFIGPRN
SKNAGLFELTSQGSASDFEFLGVKIPRYRDYTPSSLLNATLIHQSITGLYETRIDLS
KLGED
**SEQ ID NO: 11**
   SEQUENCE: 
ATGACAAAACCTTATTCTATTGGACTTGATATTGGGACTAACTCTGTTGGTTG
GGCTGTTGTGACAGATGGCTACAAAGTTCCTGCTAAGAAGATGAAGGTTCTG
GGAAATACAGATAAAAGCCATATCAAGAAAAATTTACTTGGAGCTTTATTGT
TTGATAGCGGTAATACTGCAAAAGACAGACGTTTGAAGCGGACAGCTAGGCG
TCGATATACACGTCGTAGAAACCGTATTTTATATTTGCAGGAAATTTTTGCTG
AAGAAATGGCTAAAGCAGACGAAAGTTTCTTCCAGCGCTTAAACGAATCGTT
TTTAACAAATGATGACAAAGAATTTGATTCTCATCCAATCTTTGGGAATAAAG
CTGAAGAGGAGGCTCATCACCATAAATTTCCAACAATTTTTCATTTGCGAAAG
CATTTAGCAGACTCAACCGAGAAATCTGATTTGCGCTTAATTTATCTAGCTTT
AGCGCATATGATTAAATTCCGGGGACATTTCTTAATTGAAGGTCAGCTAAAA
GCTGAAAATACAAATGTTCAAACATTATTTGACGATTTTGTAGAAGTATATGA
TAAGACAGTTGAAGAAAGTCATTTATCAGAAATTAGTGTCTCCAGTATTCTGA
CAGAAAAAATTAGTAAATCGCGTCGCTTAGAAAATCTTATAAAATACTATCC
CACTGAGAAGAAAAACACTCTCTTCGGAAATCTTATCGCCTTGTCTTTAGGAT
TACAGCCAAACTTTAAAACAAATTTTAAATTATCCGAAGATGCTAAACTACA
GTTTTCTAAGGATACTTATGAAGAAGATTTAGGAGAATTACTTGGAAAAATC
 
GGAGATAATTATGCAGATTTATTTATATCAGCTAAAAATCTTTATGATGCTAT
TTTGCTATCAGGAATTTTAACAATAGATGACAACACGACAAAGGCTCCGTTG
TCTGCTTCAATGATTAAACGTTATGAGGAACATCAGGAAGATTTAGCACAAC
TTAAGAAATTTATCCGTCAGAATTTACCAGATCAATATAGTGAGGTTTTTCT
GATAAAACAAAGGATGGCTATGCTGGTTATATTGATGGAAAAACGAATCAGG
AGGCCTTTTATAAATACATCAAAAATATGCTGTCAAAAACAGAAGGTGCAGA
TTATTTTCTTGACAAAATTGATCGTGAAGACTTTTTGAGAAAACAGAGAACGT
TTGATAATGGTTCCGTTCCGCATCAGATTCATCTGCAAGAGATGCATGCTATT
TTACGACGTCAGGGTGAATACTATCCATTCTTGAAAGAAAATCAGGATAAAA
TTGAAAAAATCTTAACGTTTAGAATTCCTTACTACGTTGGTCCTTTGGCGCGA
AAAGGTAGCCGCTTTGCCTGGGCAGAATACAAGGCGGATAAAAAAGTTACGC
CATGGAATTTTGATGATATTCTTGATAAAGAAAAATCAGCAGAAGAATTCAT
CACACGCATGACTTTAAATGATTTGTATTTACCTGAAGAAAAAGTCTTACCAA
AGCATAGTCTTGTTTATGAAACGTTTAATGTTTACAATGAGTTAACTAAAGTT
AAGTATGTCAATGAGCAAGGGAAAGCCATTTTCTTTGATGCCAATATGAAGC
AAGAGATTTTTGATCATGTTTTTAAAGAAAATCGGAAAGTTACTAAAGATAA
ACTTTTAAATTATTTGAATAAAGAGTTTGAAGAATTTAGAATTGTTAACTTAA
CTGGACTGGATAAGGAAAATAAAGCCTTTAATTCCAGTCTTGGAACCTATCA
TGATTTGCGTAAAATTTTAGATAAATCATTCTTAGATGATAAAGTAAATGAAA
AGATAATTGAGGATATCATTCAAACACTAACTCTGTTTGAAGACAGAGAAAT
GATTCGTCAGCGTCTTCAAAAGTATAGTGATATTTTTACAACACAGCAATTGA
AAAAACTTGAACGCCGTCATTATACAGGTTGGGGAAGATTATCAGCGAAGTT
AATCAATGGTATTCGAGATAAACAGAGTAATAAGACTATTCTGGGTTATTTG
ATTGATGATGGTTATAGCAATCGTAACTTTATGCAGTTGATTAATGACGATTC
TCTTCCTTTTAAAGAAGAAATTGCTAGGGCACAAGTCATTGGAGAAACAGAT
GACTTAAATCAACTTGTTAGTGATATTGCTGGCAGTCCTGCTATTAAAAAGGG
AATTTTACAAAGTCTGAAAATTGTAGATGAGCTTGTTAAAGTCATGGGGCAT
AATCCTGCTAACATTGTTATCGAAATGGCGCGTGAAAATCAGACTACAGCCA
AAGGGCGTCGCAGTTCACAGCAACGTTATAAACGACTTGAGGAGGCAATAAA
AAATCTTGACCATGATTTAAATCATAAGATTTTAAAAGAACACCCAACAGAT
AATCAAGCTTTACAGAATGACCGTCTTTTCTTATATTATCTCCAAAATGGCCG
 
AGATATGTATACTGAAGATCCACTTGATATTAATCGTTTAAGTGATTATGATA
TCGACCATATTATTCCACAATCTTTATAAAAGATGACTCTATTGACAATAAG
GTTCTGGTTTCATCAGCTAAAAACCGTGGGAAATCGGATAATGTACCGAGTG
AAGATGTTGTCAATAGGATGAGACCGTTTTGGAATAAATTATTGAGCTGTGG
ATTGATTTCTCAACGGAAATACAGCAATCTAACCAAAAAGAATTAAAACCA
GATGATAAGGCTGGTTTCATCAAACGTCAATTGGTTGAGACAAGACAAATTA
CAAAGCATGTTGCACAAATTTTAGACGCTCGTTTTAATACAAAACGTGATGA
AAATAAAAAAGTAATTCGTGATGTCAAAATTATCACTTTAAAATCTAATTTAG
TTTCACAATTTCGTAAAGACTTTAAATTTTACAAAGTACGTGAGATTAATGAT
TACCATCATGCGCATGACGCTTATCTTAATGCAGTTATAGGAAAAGCTTATT
AGATGTTTATCCGCAGTTAGAGCCCGAATTTGTTTATGGTGAGTACCCTCATT
TTCATGGATATAAAGAAAATAAAGCAACTGCTAAGAAATTTTTCTATTCAAA
TATTATGAATTTTTTTAAGAAAGATGATATCCGTACCGATGAAAATGGTGAG
ATTGTTTGGAAAAAAGATGAGCATATTTCTAATATTAAAAGGGTGCTTTCCTA
TCCCCAAGTTAATATTGTTAAGAAAGTAGAAATACAGACTGTTGGACAAAAT
GGGGGACTTTTTGACGATAATCCTAAATCACCATTAGAGGTTACACCTAGTA
AACTTGTTCCACTAAAAAAAGAATTAAACCCTAAAAAATATGGAGGATATCA
AAAACCGACGACAGCTTATCCTGTTTTACTGATAACAGATACTAAACAGCTA
ATTCCAATCTCAGTAATGAATAAGAAGCAATTTGAACAAAATCCGGTTAAAT
TTTTAAGAGATAGAGGCTATCAACAGGTAGGAAAGAATGACTTTATTAAATT
ACCCAAATATACCCTAGTTGATATCGGTGATGGGATTAAACGCCTATGGGCT
AGTTCGAAAGAAATACATAAAGGAAATCAATTAGTTGTATCTAAAAAATCTC
AAATTTTGCTTTATCATGCACATCACTTAGATAGTGATTTGAGTAATGATTAT
CTTCAAAATCATAATCAACAATTCGATGTTTTATTTAATGAAATTATTTCTTTT
TCTAAAAAATGTAAATTGGGAAAAGAACATATTCAGAAAATTGAAAATGTTT
ACTCCAATAAGAAGAATAGTGCATCAATAGAAGAATTAGCAGAGAGTTTTAT
TAAATTATTAGGATTTACACAATTAGGTGCAACTTCCCCATTTAATTTTTTAG
GGGTAAAACTAAATCAAAAACAATATAAAGGTAAAAAAGATTATATTTTACC
GTGTACAGAGGGGACCCTTATCCGCCAATCTATCACTGGTCTTTACGAAACAC
GAGTTGATCTTAGTAAAATAGGAGAAGACTAA
**SEQ ID NO: 12**
   NAME: gi|357584860|gb|EHJ52063.1| CRISPR-associated protein Cas9/Csn1, subtype II/NMEMI [Streptococcus macacae NCTC 11558]
   SEQUENCE: 
MTKPYSIGLDIGTNSVGWAVVTDGYKVPAKKMKVLGNTDKSHIKKNLLGALLF
DSGNTAKDRRLKRTARRRYTRRRNRILYLQEIFAEEMAKADESFFQRLNESFLTN
DDKEFDSHPIFGNKAEEEAHHHKFPTIFHLRKHLADSTEKSDLRLIYLALAHMIKF
RGHFLIEGQLKAENTNVQTLFDDFVEVYDKTVEESHLSEISVSSILTEKISKSRRLE
NLIKYYPTEKKNTLFGNLIALSLGLQPNFKTNFKLSEDAKLQFSKDTYEEDLGELL
GKIGDNYADLFISAKNLYDAILLSGILTIDDNTTKAPLSASMIKRYEEHQEDLAQL
KKFIRQNLPDQYSEVFSDKTKDGYAGYIDGKTNQEAFYKYIKNMLSKTEGADYF
LDKIDREDFLRKQRTFDNGSVPHQIHLQEMHAILRRQGEYYPFLKENQDKIEKILT
FRIPYYVGPLARKGSRFAWAEYKADKKVTPWNFDDILDKEKSAEEFITRMTLND
LYLPEEKVLPKHSLVYETFNVYNELTKVKYVNEQGKAIFFDANMKQEIFDHVFK
ENRKVTKDKLLNYLNKEFEEFRIVNLTGLDKENKAFNSSLGTYHDLRKILDKSFL
DDKVNEKIIEDIIQTLTLFEDREMIRQRLQKYSDIFTTQQLKKLERRHYTGWGRLS
AKLINGIRDKQSNKTILGYLIDDGYSNRNFMQLINDDSLPFKEEIARAQVIGETDD
LNQLVSDIAGSPAIKKGILQSLKIVDELVKVMGHNPANIVIEMARENQTTAKGRR
SSQQRYKRLEEAIKNLDHDLNHKILKEHPTDNQALQNDRLFLYYLQNGRDMYTE
DPLDINRLSDYDIDHIIPQSFIKDDSIDNKVLVSSAKNRGKSDNVPSEDVVNRMRP
FWNKLLSCGLISQRKYSNLTKKELKPDDKAGFIKRQLVETRQITKHVAQILDARF
NTKRDENKKVIRDVKIITLKSNLVSQFRKDFKFYKVREINDYHHAHDAYLNAVIG
KALLDVYPQLEPEFVYGEYPHFHGYKENKATAKKFFYSNIMNFFKKDDIRTDEN
GEIVWKKDEHISNIKRVLSYPQVNIVKKVEIQTVGQNGGLFDDNPKSPLEVTPSK
LVPLKKELNPKKYGGYQKPTTAYPVLLITDTKQLIPISVMNKKQFEQNPVKFLRD
RGYQQVGKNDFIKLPKYTLVDIGDGIKRLWASSKEIHKGNQLVVSKKSQILLYHA
HHLDSDLSNDYLQNHNQQFDVLFNEIISFSKKCKLGKEHIQKIENVYSNKKNSASI
EELAESFIKLLGFTQLGATSPFNFLGVKLNQKQYKGKKDYILPCTEGTLIRQSITGL
YETRVDLSKIGED
**SEQ** ID NO: 13
   SEQUENCE: 
ATGGATAAGAAATACTCAATAGGCTTAGATATCGGCACAAATAGCGTCGGAT
GGGCGGTGATCACTGATGATTATAAGGTTCCGTCTAAAAAGTTCAAGGTTCT
GGGAAATACAGACCGCCACAGTATCAAAAAAAATCTTATAGGGGCTCTTTTA
TTTGACAGTGGAGAGACAGCGGAAGCGACTCGTCTCAAACGGACAGCTCGTA
GAAGGTATACACGTCGGAAGAATCGTATTTGTTATCTACAGGAGATTTTTTCA
AATGAGATGGCGAAAGTAGATGATAGTTTCTTTCATCGACTTGAAGAGTCTTT
TTTGGTGGAAGAAGACAAGAAGCATGAACGTCATCCTATTTTTGGAAATATA
GTAGATGAAGTTGCTTATCATGAGAAATATCCAACTATCTATCATCTGCGAAA
AAAATTGGTAGATTCTACTGATAAAGCGGATTTGCGCTTAATCTATTTGGCCT
TAGCGCATATGATTAAGTTTCGTGGTCATTTTTTGATTGAGGGAGATTTAAAT
CCTGATAATAGTGATGTGGACAAACTATTTATCCAGTTGGTACAAACCTACA
ATCAATTATTTGAAGAAAACCCTATTAACGCAAGTGGAGTAGATGCTAAAGC
GATTCTTTCTGCACGATTGAGTAAATCAAGACGATTAGAAAATCTCATTGCTC
AGCTCCCCGGTGAGAAGAAAAATGGCTTATTTGGGAATCTCATTGCTTTGTCA
TTGGGTTTGACCCCTAATTTTAAATCAAATTTTGATTTGGCAGAAGATGCTAA
ATTACAGCTTTCAAAAGATACTTACGATGATGATTTAGATAATTTATTGGCGC
AAATTGGAGATCAATATGCTGATTTGTTTTTGGCAGCTAAGAATTTATCAGAT
GCTATTTTACTTTCAGATATCCTAAGAGTAAATACTGAAATAACTAAGGCTCC
CCTATCAGCTTCAATGATTAAACGCTACGATGAACATCATCAAGACTTGACTC
TTTTAAAAGCTTTAGTTCGACAACAACTTCCAGAAAAGTATAAAGAAATCTTT
TTTGATCAATCAAAAAACGGATATGCAGGTTATATTGATGGGGGAGCTAGCC
AAGAAGAATTTTATAAATTTATCAAACCAATTTTAGAAAAAATGGATGGTAC
TGAGGAATTATTGGTGAAACTAAATCGTGAAGATTTGCTGCGCAAGCAACGG
ACCTTTGACAACGGCTCTATTCCCCATCAAATTCACTTGGGTGAGCTGCATGC
TATTTTGAGAAGACAAGAAGACTTTTATCCATTTTTAAAAGACAATCGTGAG
AAGATTGAAAAAATCTTGACTTTTCGAATTCCTTATTATGTTGGTCCATTGGC
GCGTGGCAATAGTCGTTTTGCATGGATGACTCGGAAGTCTGAAGAAACAATT
ACCCCATGGAATTTTGAAGAAGTTGTCGATAAAGGTGCTTCAGCTCAATCATT
TATTGAACGCATGACAAACTTTGATAAAAATCTTCCAAATGAAAAAGTACTA
CCAAAACATAGTTTGCTTTATGAGTATTTTACGGTTTATAACGAATTGACAAA
GGTCAAATATGTTACTGAAGGAATGCGAAAACCAGCATTTCTTTCAGGTGAA
 
CAGAAGAAAGCCATTGTTGATTTACTCTTCAAAACAAATCGAAAAGTAACCG
TTAAGCAATTAAAAGAAGATTATTCAAAAAAATAGAATGTTTTGATAGTGT
TGAAATTTCAGGAGTTGAAGATAGATTTAATGCTTCATTAGGTACCTACCATG
ATTTGCTAAAAATTATTAAAGATAAAGATTTTTTGGATAATGAAGAAAATGA
AGATATCTTAGAGGATATTGTTTTAACATTGACCTTATTTGAAGATAGGGAGA
TGATTGAGGAAAGACTTAAAACATATGCTCACCTCTTTGATGATAAGGTGAT
GAAACAGCTTAAACGTCGCCGTTATACTGGTTGGGGACGTTTGTCTCGAAAA
TTGATTAATGGTATTAGGGATAAGCAATCTGGCAAAACAATATTAGATTTTT
GAAATCAGATGGTTTTGCCAATCGCAATTTTATGCAGCTGATCCATGATGATA
GTTTGACATTTAAAGAAGACATTCAAAAAGCACAAGTGTCTGGACAAGGCGA
TAGTTTACATGAACATATTGCAAATTTAGCTGGTAGCCCTGCTATTAAAAAG
GTATTTTACAGACTGTAAAAGTTGTTGATGAATTGGTCAAAGTAATGGGGCG
GCATAAGCCAGAAAATATCGTTATTGAAATGGCACGTGAAAATCAGACAACT
CAAAAGGGCCAGAAAAATTCGCGAGAGCGTATGAAACGAATCGAAGAAGGT
ATCAAAGAATTAGGAAGTCAGATTCTTAAAGAGCATCCTGTTGAAAATACTC
AATTGCAAAATGAAAAGCTCTATCTCTATTATCTCCAAAATGGAAGAGACAT
GTATGTGGACCAAGAATTAGATATTAATCGTTTAAGTGATTATGATGTCGATC
ACATTGTTCCACAAAGTTTCCTTAAAGACGATTCAATAGACAATAAGGTCTTA
ACGCGTTCTGATAAAAATCGTGGTAAATCGGATAACGTTCCAAGTGAAGAAG
TAGTCAAAAAGATGAAAAACTATTGGAGACAACTTCTAAACGCCAAGTTAAT
CACTCAACGTAAGTTTGATAATTTAACGAAAGCTGAACGTGGAGGTTTGAGT
GAACTTGATAAAGCTGGTTTTATCAAACGCCAATTGGTTGAAACTCGCCAAA
TCACTAAGCATGTGGCACAAATTTTGGATAGTCGCATGAATACTAAATACGA
TGAAAATGATAAACTTATTCGAGAGGTTAAAGTGATTACCTTAAAATCTAAA
TTAGTTTCTGACTTCCGAAAAGATTTCCAATTCTATAAAGTACGTGAGATTAA
CAATTACCATCATGCCCATGATGCGTATCTAAATGCCGTCGTTGGAACTGCTT
TGATTAAGAAATATCCAAAACTTGAATCGGAGTTTGTCTATGGTGATTATAAA
GTTTATGATGTTCGTAAAATGATTGCTAAGTCTGAGCAAGAAATAGGCAAAG
CAACCGCAAAATATTTCTTTTACTCTAATATCATGAACTTCTTCAAAACAGAA
ATTACACTTGCAAATGGAGAGATTCGCAAACGCCCTCTAATCGAAACTAATG
GGGAAACTGGAGAAATTGTCTGGGATAAAGGGCGAGATTTTGCCACAGTGCG
 
CAAAGTATTGTCCATGCCCCAAGTCAATATTGTCAAGAAAACAGAAGTACAG
ACAGGCGGATTCTCCAAGGAGTCAATTTTACCAAAAAGAAATTCGGACAAGC
TTATTGCTCGTAAAAAAGACTGGGATCCAAAAAAATATGGTGGTTTTGATAG
TCCAACGGTAGCTTATTCAGTCCTAGTGGTTGCTAAGGTGGAAAAAGGGAAA
TCGAAGAAGTTAAAATCCGTTAAAGAGTTACTAGGGATCACAATTATGGAAA
GAAGTTCCTTTGAAAAAAATCCGATTGACTTTTTAGAAGCTAAAGGATATAA
GGAAGTTAAAAAAGACTTAATCATTAAACTACCTAAATATAGTCTTTTTGAGT
TAGAAAACGGTCGTAAACGGATGCTGGCTAGTGCCGGAGAATTACAAAAAG
GAAATGAGCTGGCTCTGCCAAGCAAATATGTGAATTTTTATATTTAGCTAGT
CATTATGAAAAGTTGAAGGGTAGTCCAGAAGATAACGAACAAAAACAATTGT
TTGTGGAGCAGCATAAGCATTATTTAGATGAGATTATTGAGCAAATCAGTGA
ATTTTCTAAGCGTGTTATTTTAGCAGATGCCAATTTAGATAAAGTTCTTAGTG
CATATAACAAACATAGAGACAAACCAATACGTGAACAAGCAGAAAATATTA
TTCATTTATTTACGTTGACGAATCTTGGAGCTCCCGCTGCTTTTAAATATTTTG
ATACAACAATTGATCGTAAACGATATACGTCTACAAAAGAAGTTTTAGATGC
CACTCTTATCCATCAATCCATCACTGGTCTTTATGAAACACGCATTGATTTGA
GTCAGCTAGGAGGTGACTGA
**SEQ ID NO: 14**
   NAME: gi|409693032|gb|AFV37892.1| CRISPR-associated protein, Csn1 family
   [Streptococcus pyogenes A20]
   SEQUENCE: 
MDKKYSIGLDIGTNSVGWAVITDDYKVPSKKFKVLGNTDRHSIKKNLIGALLFDS
GETAEATRLKRTARRRYTRRKNRICYLQEIFSNEMAKVDDSFFHRLEESFLVEED
KKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRR
LENLIAQLPGEKKNGLFGNLIALSLGLTPNFKSNFDLAEDAKLQLSKDTYDDDLD
NLLAQIGDQYADLFLAAKNLSDAILLSDILRVNTEITKAPLSASMIKRYDEHHQDL
TLLKALVRQQLPEKYKEIFFDQSKNGYAGYIDGGASQEEFYKFIKPILEKMDGTE
ELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFD
KNLPNEKVLPKHSLLYEYFTVYNELTKVKYVTEGMRKPAFLSGEQKKAIVDLLF
 
KTNRKVTVKQLKEDYFKKIECFDSVEISGVEDRFNASLGTYHDLLKIIKDKDFLD
NEENEDILEDIVLTLTLFEDREMIEERLKTYAHLFDDKVMKQLKRRRYTGWGRL
SRKLINGIRDKQSGKTILDFLKSDGFANRNFMQLIHDDSLTFKEDIQKAQVSGQG
DSLHEHIANLAGSPAIKKGILQTVKVVDELVKVMGRHKPENIVIEMARENQTTQK
GQKNSRERMKRIEEGIKELGSQILKEHPVENTQLQNEKLYLYYLQNGRDMYVDQ
ELDINRLSDYDVDHIVPQSFLKDDSIDNKVLTRSDKNRGKSDNVPSEEVVKKMK
NYWRQLLNAKLITQRKFDNLTKAERGGLSELDKAGFIKRQLVETRQITKHVAQIL
DSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIM
NFFKTEITLANGEIRKRPLIETNGETGEIVWDKGRDFATVRKVLSMPQVNIVKKTE
VQTGGFSKESILPKRNSDKLIARKKDWDPKKYGGFDSPTVAYSVLVVAKVEKGK
SKKLKSVKELLGITIMERSSFEKNPIDFLEAKGYKEVKKDLIIKLPKYSLFELENGR
KRMLASAGELQKGNELALPSKYVNFLYLASHYEKLKGSPEDNEQKQLFVEQHK
HYLDEIIEQISEFSKRVILADANLDKVLSAYNKHRDKPIREQAENIIHLFTLTNLGA
PAAFKYFDTTIDRKRYTSTKEVLDATLIHQSITGLYETRIDLSQLGGD
**SEQ ID NO: 15**
   NAME: gi|150381361|gb|EF472760.1| HIV-1 clone 39B from USA integrase (pol) gene, partial cds
   SEQUENCE: 
TTTTTGGATGGAATAGATAGGGCCCAAGAAGAGCATGAGAAATATCACAATA
ATTGGAGAGCAATGGCTAGTGATTTTAACCTGCCACCTNTAGTAGCAAAGGA
GATAGTAGCCAGCTGTGATAAATGTCAGCTAAAAGGAGAAGCCATGCATGGA
CAAGTAGACTGTAGTCCAGGAATATGGCAACTAGATTGTACACATNTAGAAG
GAAAAGTTATCCTGGTAGCAGTNCATGTAGCCAGTGGTTATATAGAAGCAGA
AGTTATTCCAGCAGAGACAGGGCAGGAAACAGCATACTTCCTCTTAAAATTA
GCAGGAAGATGGCCAGTAAAAACAGTACATACAGACAATGGCAGCAACTTC
ACCAGTGCTGCGNTGAAGGCCGCCTGTTGGTGGGCAGGGATCAAGCAGGAAT
TTGGCATTCCCTACAATCCCCAAAGTCAAGGAGTAGTAGAGTCTATGAATAA
TGAATTAAAGAAAATTGTAGGACAAGTAAGAGATCAGGCTGAGCATCTCAAG
ACAGCAGTACAAATGGCAGTATTCATCCACAATTTTAAAAGAAAAGGGGGA
TTGGGGGGTACAGTGCAGGAGAAAGAATAGTAGACATAATAGCCACAGACA
 
TACAAACTAAAGAACTACAAAAAAATATTACAAAAATGCAAAATTTTCGGGT
CTATTTCAGAGACAGCAGAGATCCACTTTGGAAAGGACCAGCAAAGCTTCTC
TGGAAAGGTGAAGGGGCAGTAGTAATACAAGATACCAATGACATAAARGTA
GTGCCARGAAGAAAAGCAAAGATCATTAGAGATTATGGAAAACAGATGGCA
GGTGATGATTGTGTGGCAAGTAGACAGGNTGAGGATTAG
**SEQ ID NO: 16**
   NAME: gi|150381362|gb|ABR68182.1| integrase, partial [Human immunodeficiency virus 1]
   SEQUENCE: 
FLDGIDRAQEEHEKYHNNWRAMASDFNLPPXVAKEIVASCDKCQLKGEAMHGQ
VDCSPGIWQLDCTHXEGKVILVAVHVASGYIEAEVIPAETGQETAYFLLKLAGR
WPVKTVHTDNGSNFTSAAXKAACWWAGIKQEFGIPYNPQSQGVVESMNNELKK
IVGQVRDQAEHLKTAVQMAVFIHNFKRKGGIGGYSAGERIVDIIATDIQTKELQK
NITKMQNFRVYFRDSRDPLWKGPAKLLWKGEGAVVIQDTNDIKVVPXRKAKIIR
DYGKQMAGDDCVASRQXED
**SEQ ID NO: 17**
   NAME: gi|459980|gb|L20651.1|STLKIAPOL Simian T-cell lymphotropic virus type I integrase (pol) gene, partial cds
   SEQUENCE: 
GACTTGTAGAACGCTCTAATGGCATTCTTAAAACCCTATTATATAAGTACTTT
ACTGACAAACCCGACCT
ACCTATGGATAATGCTCTATCCATAGCCCTATGGACGATCAACCACCTGAATG
TGTTAACCCACTGCCAC
**SEQ ID NO: 18**
   NAME: gi|459981|gb|AAA47841.1| integrase, partial [Simian T-lymphotropic virus 1]
   SEQUENCE:
      LVERSNGILKTLLYKYFTDKPDLPMDNALSIALWTINHLNVLTHCH
**SEQ ID NO: 19**
   NAME: gi|321156784:1-1509 Streptococcus pneumoniae integrative and conjugative element ICESpn11930, strain 11930
   SEQUENCE: 
GAGTTTTTTCCTTTCGTAGCAAGGGTTTAGAGCCCCTATTTTATTTTACTATT
GTCTAAACACCAAGCG
AACACCAAAACTACCATGCAATGGAAAAACCTCTGATTTGATTCTCACTTGAT
TTCACAATCTTTATATC
AAACTGTGGGTGGTATTTGACAATATCTTTTTTGATTTTTAATAGTAAATTCG
AAATAATATTTTTAGGT
GAGTAACGTGGACTAAGATGTAACAAGTCTTTGAACTCATCGACACTTAATT
CTACTTTATTGCTATTAT
CACTAGTTTCAATGAATTTTTCAATTATTCTGGAATATTTACAGGTATAACTTT
TCAATTCTTCAAAATG
GAAATTGTGATTTTCTACAAATTGATTTAAGGCTTTTACAGTATTTTCTTGTGA
ACGATTTATATTATGT
GTATAGCCCATTGTTGTCTCAAAGTTAGCGTGTCCTACTCTAGTCATAATATC
TTTCACTGCTATGTGCA
TCTCATTACTTTGAAGGTAACTAATATGCATATGCCTAAACGAATGGGGAGT
AACATGTTTTACCCACTT
AAAACCATAGTCACTTAAACAATTTGTCAATAATTTTCCTTCTATTCGTTTCA
AAATTTGACGAAAAGTG
CTTGATGTTATTGGAGAGCCGTATTCTGTTCTAAATACACTTTCAGAATGTGT
AAAAGCAGGACAGGGAT
GTTTCTCCATATAAGCATCAAACTCTTTATTTCTCTGTATTGTCCTTTTAATAG
CTTCGCTTGCAGCTTC
AGGCAAAGCTACTTCTCTAATTGAATTGAGTGTTTTAGTTGTATCAAAATGAA
ATTGTTTAACTTTTAAA
CAATGATATTGAAGTGCTTTATCAATATGCAAGATTCCTTTTTCAAAATCAAT
ATCTGATGGTAAAAATG
CTGCTTCACTAATTCGAATACCTGTAAGCAACAATACTATAGCAAGATCATA
ATAGTTTGCATTTCTGCA
TTGGCGTAACACATCAAAAAATGCATGTAATTCATGGATTTCTAGAAATTAG
AATCATGTCTTTCTTTT
 
GCTTTACGCCTTTTCTCTAGTGAAATATCTAGTTTTACCGCAGTCATTGGAGA
AAACTTAATGACATTAT
ATAACACACCATGATTAAAAATCTTATTACAAGTACTTTTTATATGAGTCATT
GTTGAAGGCGATGCATC
ATACATTTCTAAATATTTATTGAGACTATTTTTCATCAGAAGTGGAGTAATCC
TGTCTAACAAAAAATCA
TCTCCTATAATTTTCCCAAGACGCTTCATAACCAGTAGTTCTCTCTGAATTGTT
TGTGGTTTAACAGAGA
CACACCAAGTCTGAAACCAATTTTCTTTTAACTCTCCAAATGTTGTAATCAGT
TCAGGACTATACTGACT
TTCAAATGAAGTAGTTAGTCTATCTATTTTATCAAGAACCTCTCTTTCAGCTTG
TTTCCTCGCCCTACTA
GTATTCTTAGTATAACTTACAGTTACTGATTTCCACTTT
**SEQ ID NO: 20**
   NAME: gi|321156785|emb|CBW38769.1| Integrase [Streptococcus pneumoniae]
   SEQUENCE: 
MYYVTKTNSKGQPLYQVVEKYKDPLTGKWKSVTVSYTKNTSRARKQAEREVL
DKIDRLTTSFESQYSPEL
ITTFGELKENWFQTWCVSVKPQTIQRELLVMKRLGKIIGDDFLLDRITPLLMKNSL
NKYLEMYDASPSTM
THIKSTCNKIFNHGVLYNVIKFSPMTAVKLDISLEKRRKAKERHDSKFLEIHELHA
FFDVLRQCRNANYY
DLAIVLLLTGIRISEAAFLPSDIDFEKGILHIDKALQYHCLKVKQFHFDTTKTLNSIR
EVALPEAASEAI
KRTIQRNKEFDAYMEKHPCPAFTHSESVFRTEYGSPITSSTFRQILKRIEGKLLTNC
LSDYGFKWVKHVT
PHSFRHMHISYLQSNEMHIAVKDIMTRVGHANFETTMGYTHNINRSQENTVKAL
NQFVENHNFHFEELKS
YTCKYSRIIEKFIETSDNSNKVELSVDEFKDLLHLSPRYSPKNIISNLLLKIKKDIVK
YHPQFDIKIVKS
SENQIRGFSIAW
**SEQ ID NO: 21**
   NAME: gi|43090:1-436 E.coli (Tn5086) dhfrVII gene for dihydrofolate reductase type VII and sulI gene, 5' end (integrase)
   SEQUENCE: 
GCATGCCCGTTCCATACAGAAGCTGGGCGAACAAACGATGCTCGCCTTCCAG
AAAACCGAGGATGCGAAC
CACTTCATCCGGGGTCAGCACCACCGGCAAGCGCCGCGACGGCCGAGGTCTT
CCGATCTCCTGAAGCCAG
GGCAGATCCGTGCACAGCACCTTGCCGTAGAAGAACAGCAAGGCCGCCAAT
GCCTGACGATGCGTGGAGA
CCGAAACCTTGCGCTCGTTCGCCAGCCAGGACAGAAATGCCTCGACTTCGCT
GCTGCCCAAGGTTGCCGG
GTGACGCACACCGTGGAAACGGATGAAGGCACGAACCCAGTGGACATAAGC
CTGTTCGGTTCGTAAGCTG
TAATGCAAGTAGCGTATGCGCTCACGCAACTGGTCCAGAACCTTGACCGAAC
GCAGCGGTGGTAACGGCG
CAGTGGCGGTTTTCAT
**SEQ ID NO: 22**
   NAME: gi|43091|emb|CAA41325.1| integrase, partial (plasmid) [Escherichia coli]
   SEQUENCE: 
MKTATAPLPPLRSVKVLDQLRERIRYLHYSLRTEQAYVHWVRAFIRFHGVRHPA
TLGSSEVEAFLSWLAN
ERKVSVSTHRQALAALLFFYGKVLCTDLPWLQEIGRPRPSRRLPVVLTPDEVVRI
LGFLEGEHRLFAQLL
YGTGM
**SEQ ID NO: 23**
   >gi|397912605:40372-41898 Thermoanaerobacterium phage THSA-485A, complete genome - recombinase 
ATGAATCGTGTATGTATTTATCTTAGGAAGTCCCGAGCAGACGAAGAAATAG
AAAAAGAGCTTGGACAAG
 
GAGAAACACTCGCAAAACATCGTAAGGCCCTTCTTAAATTTGCAAAAGAGAA
AAATTTGAACATAGTAAA
AATCAGAGAGGAAATAGTATCAGGCGAAAGCCTTATCCATAGACCTGAAATG
TTGGAATTACTAAAAGAA
GTCGAACAAGGCATGTACGATGCTGTATTATGTATGGATCTACAGCGTTTAG
GGCGTGGCAACATGCAGG
AACAAGGTCTCATTTTAGAAGCCTTTAAAAAGTCAAACACTAAAATTATAAC
GCTTCAAAAAACTTATGA
TTTGAACAATGATTTTGACGAAGAATATAGCGAATTTGAAGCATTTATGAGC
CGAAAGGAACTTAAAATG
ATAAATAGAAGGCTACAAGGTGGCAGAGTACGCTCTATTCAGGAAGGTAATT
ATTTATCACCATTGCCAC
CTTATGGTTACTTAATACACGAAGAAAAATTTCGCGCACTCTTGTGCCTAAT
CCTGAGCAAGCTGATGT
AGTTAAAATGATTTTTGATATGTATGTCAATAAACAGATGGGGTCTAGTGCTA
TAGCGAACGAACTAAAC
AAAATGGGTTATAAGACGTATACTGGCAGGAATTGGGCTTCAAGCTCTGTAA
TAAACATACTCAAGAATC
CAGTTTACATCGGTAAAATAACGTGGAAGAAGAAGGATATAAAGAAGTCTGC
TGACCCAAATAAAAGCAA
AGATACACGTCAAAGACCACGCTCTGAATGGATTGTATCAGATGGCAAACAT
GAACCAATAGTGGGCAAA
GAGCTCTTTGCCAAGGCTCAAGAAATCATTAAAAACAAGTATCACATACCGT
ATCAGATCGTTAATGGTC
CACGTAACCCATTGGCAGGGCTTATTATATGCAAAATATGTGGCTCTAAAAT
GGTGTATAGACCCTACAA
AGATAAAGAAGCGCATATAATATGTCCAAACAAGTGCGGCAATAAAAGCAG
CAAATTTATCTATGTAGAA
AAAAGATTATTACAGGCTTTGGAGGAATGGATGCAAGGCTACGAGCTGGATC
TGCAAATAGAAGAAGATG
 
ACAGCTCTTTTGCAGAAGCACAAGAGAAACAAAAAGAAGCTCTTGAAAGAG
AATTGCACGAGCTGCAAAA
GCAAAAGAACAATTTACACGATTTGCTCGAGCGTGGCATATACGATATAGAT
ACATTTGTGGAAAGATCT
ACAATTGTAGCACAGAGAATAGAAGAAACACAGAAAAGTATAGATGTGCTT
GTGCAAAAAATAGAAGAAG
AAAAGAATAAAAGAGACAAAGAAAAAATACTTCCGGAAATTCGGCATGTGT
TGGATCTATATTGGAAAAC
AGACGACATTGCACAAAAAAATATGTTGTTAAAGAGCGTACTTGAAAAAGCA
GAATATCTAAAAGAAAAG
AAGCAGAGAGAAGACAACTTCGAACTTTGGATTTATCCAAAGCTGCCTGAAA
AATAG
**SEQ ID NO: 24**
   >gi|397912662|ref|YP_006546326.1| Recombinase [Thermoanaerobacterium phage THSA-485A] 
MNRVCIYLRKSRADEEIEKELGQGETLAKHRKALLKFAKEKNLNIVKIREEIVSG
ESLIHRPEMLELLKE
VEQGMYDAVLCMDLQRLGRGNMQEQGLILEAFKKSNTKIITLQKTYDLNNDFD
EEYSEFEAFMSRKELKM
INRRLQGGRVRSIQEGNYLSPLPPYGYLIHEEKFSRTLVPNPEQADVVKMIFDMY
VNKQMGSSAIANELN
KMGYKTYTGRNWASSSVINILKNPVYIGKITWKKKDIKKSADPNKSKDTRQRPR
SEWIVSDGKHEPIVGK
ELFAKAQEIIKNKYHIPYQIVNGPRNPLAGLIICKICGSKMVYRPYKDKEAHIICPN
KCGNKSSKFIYVE
KRLLQALEEWMQGYELDLQIEEDDSSFAEAQEKQKEALERELHELQKQKNNLH
DLLERGIYDIDTFVERS
TIVAQRIEETQKSIDVLVQKIEEEKNKRDKEKILPEIRHVLDLYWKTDDIAQKNML
LKSVLEKAEYLKEK
KQREDNFELWIYPKLPEK
**SEQ ID NO: 25**
   Gin recombinase
   >gi|657193240|sp|Q38199.2|GIN_BPD10 RecName: Full=Serine recombinase gin; AltName: Full=G-segment invertase; Short=Gin 
MLIGYVRVSTNDQNTDLQRNALVCAGCEQIFEDKLSGTRTDRPGLKRALKRLQK
GDTLVVWKLDRLGRSM
KHLISLVGELRERGINFRSLTDSIDTSSPMGRFFFHVMGALAEMERELIIERTMAG
LAAARNKGRIGGRP
PKLTKAEWEQAGRLLAQGIPRKQVALIYDVALSTLYKKHPAKRTHIENDDRINQI
DR
**SEQ ID NO: 26**
   Cre recombinase
   >gi|375331813|dbj|BAL61207.1| Cre recombinase [Cre-expression vector pHVX2-cre] 
MVQTSLLTVHQNLPALPVDATSDEVRKNLMDMFRDRQAFSEHTWKMLLSVCRS
WAAWCKLNNRKWFPAEP
EDVRDYLLYLQARGLAVKTIQQHLGQLNMLHRRSGLPRPSDSNAVSLVMRRIRK
ENVDAGERAKQALAFE
RTDFDQVRSLMENSDRCQDIRNLAFLGIAYNTLLRIAEIARIRVKDISRTDGGRML
IHIGRTKTLVSTAG
VEKALSLGVTKLVERWISVSGVADDPNNYLFCRVRKNGVAAPSATSQLSTRALE
GIFEATHRLIYGAKDD
SGQRYLAWSGHSARVGAARDMARAGVSIPEIMQAGGWTNVNIVMNYIRNLDSE
TGAMVRLLEDGD
**SEQ ID NOS: 27-46**
   These are exemplary sequences of polynucleotides encoding the TALE repeat modules for use in linking to integrases or recombinases as described in this invention.
**SEQ ID NO: 27**
   NAME: NI
   SEQUENCE: 
CTGACCCCAGAGCAGGTCGTGGCAATCGCCTCCAACATTGGCGG
GAAACAGGCACTCGAGACTGTCCAGCGCCTGCTTCCCGTGCTGTG
 CCAAGCGCACGGA
**SEQ ID NO: 28**
   NAME: NG
   SEQUENCE: 
CTGACCCCAGAGCAGGTCGTGGCCATTGCCTCGAATGGAGGGGG
CAAACAGGCGTTGGAAACCGTACAACGATTGCTGCCGGTGCTGT
GCCAAGCGCACGGC
**SEQ ID NO: 29**
   NAME: HD
   SEQUENCE: 
TTGACCCCAGAGCAGGTCGTGGCGATCGCAAGCCACGACGGAGG
AAAGCAAGCCTTGGAAACAGTACAGAGGCTGTTGCCTGTGCTGT
GCCAAGCGCACGGG
**SEQ ID NO: 30**
   NAME: NN
   SEQUENCE: 
CTTACCCCAGAGCAGGTCGTGGCAATCGCGAGCAATAACGGCGG
AAAACAGGCTTTGGAAACGGTGCAGAGGCTCCTTCCAGTGCTGT
GCCAAGCGCACGGG
**SEQ ID NO: 31**
   NAME: NI-NI 
SEQUENCE: CTGACCCCAGAGCAGGTCGTGGCAATCGCCTCCAACATTGGCGG
GAAACAGGCACTCGAGACTGTCCAGCGCCTGCTTCCCGTGCTTTG
TCAGGCACACGGCCTCACTCCGGAACAAGTGGTCGCAATCGCCTC
CAACATTGGCGGGAAACAGGCACTCGAGACTGTCCAGCGCCTGC
TTCCCGTGCTGTGCCAAGCGCACGGT
**SEQ ID NO: 32**
   NAME: NI-NG
   SEQUENCE: 
CTGACCCCAGAGCAGGTCGTGGCAATCGCCTCCAACATTGGCGG
GAAACAGGCACTCGAGACTGTCCAGCGCCTGCTTCCCGTGCTTTG
 
TCAGGCACACGGCCTCACTCCGGAACAAGTGGTCGCCATTGCCTC
GAATGGAGGGGGCAAACAGGCGTTGGAAACCGTACAACGATTG
CTGCCGGTGCTGTGCCAAGCGCACGGT
**SEQ ID NO: 33**
   NAME: NI-HD
   SEQUENCE: 
CTGACCCCAGAGCAGGTCGTGGCAATCGCCTCCAACATTGGCGG
GAAACAGGCACTCGAGACTGTCCAGCGCCTGCTTCCCGTGCTTTG
TCAGGCACACGGCCTCACTCCGGAACAAGTGGTCGCGATCGCAA
GCCACGACGGAGGAAAGCAAGCCTTGGAAACAGTACAGAGGCT
GTTGCCTGTGCTGTGCCAAGCGCACGGT
**SEQ ID NO: 34**
   NAME: NI-NN
   SEQUENCE: 
CTGACCCCAGAGCAGGTCGTGGCAATCGCCTCCAACATTGGCGG
GAAACAGGCACTCGAGACTGTCCAGCGCCTGCTTCCCGTGCTTTG
TCAGGCACACGGCCTCACTCCGGAACAAGTGGTCGCAATCGCGA
GCAATAACGGCGGAAAACAGGCTTTGGAAACGGTGCAGAGGCT
CCTTCCAGTGCTGTGCCAAGCGCACGGT
**SEQ ID NO: 35**
   NAME: NG-NI
   SEQUENCE: 
CTGACCCCAGAGCAGGTCGTGGCCATTGCCTCGAATGGAGGGGG
CAAACAGGCGTTGGAAACCGTACAACGATTGCTGCCGGTGCTTTG
TCAGGCACACGGCCTCACTCCGGAACAAGTGGTCGCAATCGCCTC
CAACATTGGCGGGAAACAGGCACTCGAGACTGTCCAGCGCCTGC
TTCCCGTGCTGTGCCAAGCGCACGGT
**SEQ ID NO: 36**
   NAME: NG-NG
   SEQUENCE: CTGACCCCAGAGCAGGTCGTGGCCATTGCCTCGAATGGAGGGGG 
CAAACAGGCGTTGGAAACCGTACAACGATTGCTGCCGGTGCTTTG
TCAGGCACACGGCCTCACTCCGGAACAAGTGGTCGCCATTGCCTC
GAATGGAGGGGGCAAACAGGCGTTGGAAACCGTACAACGATTG
CTGCCGGTGCTGTGCCAAGCGCACGGT
**SEQ ID NO: 37**
   NAME: NG-HD
   SEQUENCE: 
CAAACAGGCGTTGGAAACCGTACAACGATTGCTGCCGGTGCTTTG
TCAGGCACACGGCCTCACTCCGGAACAAGTGGTCGCGATCGCAA
GCCACGACGGAGGAAAGCAAGCCTTGGAAACAGTACAGAGGCT
GTTGCCTGTGCTGTGCCAAGCGCACGGT
**SEQ ID NO: 38**
   NAME: NG-NN
   SEQUENCE: 
CTGACCCCAGAGCAGGTCGTGGCCATTGCCTCGAATGGAGGGGG
CAAACAGGCGTTGGAAACCGTACAACGATTGCTGCCGGTGCTTTG
TCAGGCACACGGCCTCACTCCGGAACAAGTGGTCGCAATCGCGA
GCAATAACGGCGGAAAACAGGCTTTGGAAACGGTGCAGAGGCT
CCTTCCAGTGCTGTGCCAAGCGCACGGT
**SEQ ID NO: 39**
   NAME: HD-NI
   SEQUENCE: 
CTGACCCCAGAGCAGGTCGTGGCGATCGCAAGCCACGACGGAG
GAAAGCAAGCCTTGGAAACAGTACAGAGGCTGTTGCCTGTGCTTT
GTCAGGCACACGGCCTCACTCCGGAACAAGTGGTCGCAATCGCCT
CCAACATTGGCGGGAAACAGGCACTCGAGACTGTCCAGCGCCTG
CTTCCCGTGCTGTGCCAAGCGCACGGT
**SEQ ID NO: 40**
   NAME: HD-NG
   SEQUENCE: GAAAGCAAGCCTTGGAAACAGTACAGAGGCTGTTGCCTGTGCTTT 
GTCAGGCACACGGCCTCACTCCGGAACAAGTGGTCGCCATTGCCT
CGAATGGAGGGGGCAAACAGGCGTTGGAAACCGTACAACGATT
GCTGCCGGTGCTGTGCCAAGCGCACGGT
**SEQ ID NO: 41**
   NAME: HD-HD
   SEQUENCE: 
CTGACCCCAGAGCAGGTCGTGGCGATCGCAAGCCACGACGGAG
GAAAGCAAGCCTTGGAAACAGTACAGAGGCTGTTGCCTGTGCTTT
GTCAGGCACACGGCCTCACTCCGGAACAAGTGGTCGCGATCGCA
AGCCACGACGGAGGAAAGCAAGCCTTGGAAACAGTACAGAGGC
TGTTGCCTGTGCTGTGCCAAGCGCACGGT
**SEQ ID NO: 42**
   NAME: HD-NN
   SEQUENCE: 
CTCACCCCAGAGCAGGTCGTGGCGATCGCAAGCCACGACGGAGG
AAAGCAAGCCTTGGAAACAGTACAGAGGCTGTTGCCTGTGCTTTG
TCAGGCACACGGCCTCACTCCGGAACAAGTGGTCGCAATCGCGA
GCAATAACGGCGGAAAACAGGCTTTGGAAACGGTGCAGAGGCT
CCTTCCAGTGCTGTGCCAAGCGCACGGA
**SEQ ID NO: 43**
   NAME: NN-NI
   SEQUENCE: 
CTGACCCCAGAGCAGGTCGTGGCAATCGCGAGCAATAACGGCGG
AAAACAGGCTTTGGAAACGGTGCAGAGGCTCCTTCCAGTGCTTTG
TCAGGCACACGGCCTCACTCCGGAACAAGTGGTCGCAATCGCCTC
CAACATTGGCGGGAAACAGGCACTCGAGACTGTCCAGCGCCTGC
TTCCCGTGCTGTGCCAAGCGCACGGT
**SEQ ID NO: 44**
   NAME: NN-NG
   SEQUENCE: CTGACCCCAGAGCAGGTCGTGGCAATCGCGAGCAATAACGGCGG 
AAAACAGGCTTTGGAAACGGTGCAGAGGCTCCTTCCAGTGCTTTG
TCAGGCACACGGCCTCACTCCGGAACAAGTGGTCGCCATTGCCTC
GAATGGAGGGGGCAAACAGGCGTTGGAAACCGTACAACGATTG
CTGCCGGTGCTGTGCCAAGCGCACGGT
**SEQ ID NO: 45**
   NAME: NN-HD
   SEQUENCE: 
CTGACCCCAGAGCAGGTCGTGGCAATCGCGAGCAATAACGGCGG
AAAACAGGCTTTGGAAACGGTGCAGAGGCTCCTTCCAGTGCTTTG
TCAGGCACACGGCCTCACTCCGGAACAAGTGGTCGCGATCGCAA
GCCACGACGGAGGAAAGCAAGCCTTGGAAACAGTACAGAGGCT
GTTGCCTGTGCTGTGCCAAGCGCACGGT
**SEQ ID NO: 46**
   NAME: NN-NN
   SEQUENCE: 
CTGACCCCAGAGCAGGTCGTGGCAATCGCGAGCAATAACGGCGG
AAAACAGGCTTTGGAAACGGTGCAGAGGCTCCTTCCAGTGCTTTG
TCAGGCACACGGCCTCACTCCGGAACAAGTGGTCGCAATCGCGA
GCAATAACGGCGGAAAACAGGCTTTGGAAACGGTGCAGAGGCT
**SEQ ID NO: 47**
   NAME: gi|71796612|gb|DQ084353.1| Ovine lentivirus isolate Ov10 integrase (pol) gene, partial cds
   SEQUENCE: 
CATAGTAAATGGCATCAAGATGCTATGTCATTGCAGTTAGATTTTGGGATACC
GAAAGGTGCGGCAGAAG
ATATAGTACAACAATGTGAAGTATGTCAGGAAAATAAAATGCCTAGCACCAT
CAGAGGAAGTAACAAAAG
AGGGATAGATCATTGGCAGGTGGATTATACTCATTATAAAGACAAAATAATA
TTGGTATGGGTAGAAACA
AATTCGGGA
**SEQ ID NO: 48**
   NAME: gi|71796613|gb|AAZ41325.1| integrase, partial [Ovine lentivirus]
   SEQUENCE: 
HSKWHQDAMSLQLDFGIPKGAAEDIVQQCEVCQENKMPSTIRGSNKRGIDHWQ
VDYTHYKDKIILVWVET
NSG
**SEQ ID NO: 49**
   >gb|AYLT01000127.1|:11804-12046 Staphylococcus aureus subsp. aureus SK1585 contig000127, whole genome shotgun sequence 
TTATAGATAGGTTAGTGACAAAATACATTTTTCGTCTAGATTAACCGTGCCTC
TTAGATTATTAATATTT
TCGTTTAGATGTTTTTCAGAAACTTTAGCAACTTCATAATCGTTCATGTAAAG
TGTTTGGTTTTTATTG
TATAATTAAGTAATTCATAATCTTTGTATACTTCTTTTACTTTATCTATATCAA
CATTTTCAAGAACAAG
TTTTTTTATGTTATTATAATTAAAGTTTTCCAT
**SEQ ID NO: 50**
   >gi|669035130|gb|KFD30483.1| hypothetical protein D484_02234 [Staphylococcus aureus subsp. aureus SK1585] - s aureus cas9 
MENFNYNNIKKLVLENVDIDKVKEVYKDYELLNYTIKNQTLYMNDYEVAKVSE
KHLNENINNLRGTVNLD
EKCILSLTYL
**SEQ ID NO: 51**
   NAME: dna of linker2
   SEQUENCE:
      agcggcagcgaaaccccgggcaccagcgaaagcgcgaccccggaaagc
**SEQ ID NO: 52**
   NAME: dCas9 protein
   SEQUENCE: 
MDKKYSIGLAIGTNSVGWAVITDEYKVPSKKFKVLGNTDRHSIKKNLIGALLFDS
GETAEATR
LKRTARRRYTRRKNRICYLQEIFSNEMAKVDDSFFHRLEESFLVEEDKKHERHPIF
 
GNIVDEVA
YHEKYPTIYHLRKKLVDSTDKADLRLIYLALAHMIKFRGHFLIEGDLNPDNSDVD
KLFIQLVQ
TYNQLFEENPINASGVDAKAILSARLSKSRRLENLIAQLPGEKKNGLFGNLIALSL
GLTPNFKSN
FDLAEDAKLQLSKDTYDDDLDNLLAQIGDQYADLFLAAKNLSDAILLSDILRVNT
EITKAPLS
ASMIKRYDEHHQDLTLLKALVRQQLPEKYKEIFFDQSKNGYAGYIDGGASQEEF
YKFIKPILEK
MDGTEELLVKLNREDLLRKQRTFDNGSIPHQIHLGELHAILRRQEDFYPFLKDNR
EKIEKILTFR
IPYYVGPLARGNSRFAWMTRKSEETITPWNFEEVVDKGASAQSFIERMTNFDKN
LPNEKVLPK
HSLLYEYFTVYNELTKVKYVTEGMRKPAFLSGEQKKAIVDLLFKTNRKVTVKQL
KEDYFKKI
ECFDSVEISGVEDRFNASLGTYHDLLKIIKDKDFLDNEENEDILEDIVLTLTLFEDR
EMIEERLK
TYAHLFDDKVMKQLKRRRYTGWGRLSRKLINGIRDKQSGKTILDFLKSDGFANR
NFMQLIHD
DSLTFKEDIQKAQVSGQGDSLHEHIANLAGSPAIKKGILQTVKVVDELVKVMGR
HKPENIVIE
MARENQTTQKGQKNSRERMKRIEEGIKELGSQILKEHPVENTQLQNEKLYLYYL
QNGRDMY
VDQELDINRLSDYDVDAIVPQSFLKDDSIDNKVLTRSDKNRGKSDNVPSEEVVK
KMKNYWRQ
LLNAKLITQRKFDNLTKAERGGLSELDKAGFIKRQLVETRQITKHVAQILDSRMN
TKYDENDK
LIREVKVITLKSKLVSDFRKDFQFYKVREINNYHHAHDAYLNAVVGTALIKKYPK
LESEFVYG
DYKVYDVRKMIAKSEQEIGKATAKYFFYSNIMNFFKTEITLANGEIRKRPLIETNG
ETGEIVWD
 
KGRDFATVRKVLSMPQVNIVKKTEVQTGGFSKESILPKRNSDKLIARKKDWDPK
KYGGFDSP
TVAYSVLVVAKVEKGKSKKLKSVKELLGITIMERSSFEKNPIDFLEAKGYKEVKK
DLIIKLPKY
SLFELENGRKRMLASAGELQKGNELALPSKYVNFLYLASHYEKLKGSPEDNEQK
QLFVEQHK
HYLDEIIEQISEFSKRVILADANLDKVLSAYNKHRDKPIREQAENIIHLFTLTNLGA
PAAFKYFD TTIDRKRYTSTKEVLDATLIHQSITGLYETRIDLSQLGGD
**SEQ ID NO: 53**
   NAME: NLS nucleotide with ATG
   SEQUENCE: 
ATGGACTACAAAGACCATGACGGTGATTATAAAGATCATGACATCGATTACA
AGGATGAC
GATGACAAGATGGCCCCCAAGAAGAAGAGGAAGGTGGGCATTCACCGCGGG
GTACCT
**SEQ ID NO: 54**
   NAME: GGS linker nucleotide
   SEQUENCE:
      GGGGGAAGT
**SEQ ID NO: 55**
   NAME: Synthetic integrase
   SEQUENCE: 
ATGTTCCTGGACGGTATCGACAAAGCTCAGGACGAGCACGAAAAGTACCATT
CTAACTGGCGCGCCATGG
CCTCTGACTTCAATCTCCCGCCGGTTGTTGCCAAGGAGATCGTGGCTTCTTGC
GACAAGTGCCAATTGAA
GGGTGAGGCTATGCATGGTCAGGTCGATTGCTCTCCCGGTATCTGGCAGCTG
GACTGCACTCACCTCGAG
GGTAAGGTGATTCTCGTTGCTGTGCACGTGGCTTCCGGCTACATCGAGGCTGA
GGTCATCCCGGCTGAGA
 
CCGGTCAAGAGACTGCTTACTTCCTGCTCAAGCTGGCCGGCCGTTGGCCAGTT
AAGACTATTCACACTGA
TAACGGTTCTAACTTTACTTCCGCAACTGTGAAAGCTGCATGCTGGTGGGCCG
GCATTAAACAAGAGTTC
GGAATTCCGTATAACCCGCAGTCTCAGGGCGTTGTCGAGTCTATGAACAAGG
AGCTCAAAAAGATCATTG
GTCAAGTCCGTGACCAAGCTGAGCACCTTAAGACCGCTGTGCAGATGGCTGT
TTTTATTCATAACTTCAA
GCGTAAGGGTGGTATCGGTGGTTATAGCGCTGGTGAGCGTATCGTAGACATC
ATCGCTACTGATATCCAG
ACAAAGGAGCTGCAGAAGCAGATCACTAAGATCCAGAACTTCCGTGTGTACT
ATCGGGACTCTAGGAACC
CGCTCTGGAAGGGTCCTGCTAAACTGCTGTGGAAGGGAGAGGGTGCTGTTGT
TATCCAGGACAACTCTGA
TATCAAGGTGGTTCCGCGTCGTAAGGCTAAAATTATCCGCGACTACGGCAAG
CAAATGGCTGGAGACGAC
TGCGTTGCTAGCCGTCAAGACGAAGACTAA
**SEQ ID NO: 56**
   NAME: dCas9 nucleotide with ATG
   SEQUENCE: 
ATGGATAAAAAGTATTCTATTGGTTTAGCTATCGGCACTAATTCCGTTGGATG
GGCTGTCA
TAACCGATGAATACAAAGTACCTTCAAAGAAATTTAAGGTGTTGGGAACAC
AGACCGTC
ATTCGATTAAAAAGAATCTTATCGGTGCCCTCCTATTCGATAGTGGCGAAACG
GCAGAGG
CGACTCGCCTGAAACGAACCGCTCGGAGAAGGTATACACGTCGCAAGAACC
GAATATGTT
ACTTACAAGAAATTTTTAGCAATGAGATGGCCAAAGTTGACGATTCTTTCTTT
CACCGTTT
GGAAGAGTCCTTCCTTGTCGAAGAGGACAAGAAACATGAACGGCACCCCATC
 
TTTGGAAA
CATAGTAGATGAGGTGGCATATCATGAAAAGTACCCAACGATTTATCACCTC
AGAAAAAA
GCTAGTTGACTCAACTGATAAAGCGGACCTGAGGTTAATCTACTTGGCTCTTG
CCCATATG
ATAAAGTTCCGTGGGCACTTTCTCATTGAGGGTGATCTAAATCCGGACAACTC
GGATGTC
GACAAACTGTTCATCCAGTTAGTACAAACCTATAATCAGTTGTTTGAAGAGA
ACCCTATA
AATGCAAGTGGCGTGGATGCGAAGGCTATTCTTAGCGCCCGCCTCTCTAAAT
CCCGACGG
CTAGAAAACCTGATCGCACAATTACCCGGAGAGAAGAAAATGGGTTGTTCG
GTAACCTT
ATAGCGCTCTCACTAGGCCTGACACCAAATTTTAAGTCGAACTTCGACTTAGC
TGAAGAT
GCCAAATTGCAGCTTAGTAAGGACACGTACGATGACGATCTCGACAATCTAC
TGGCACAA
ATTGGAGATCAGTATGCGGACTTATTTTTGGCTGCCAAAAACCTTAGCGATGC
AATCCTCC
TATCTGACATACTGAGAGTTAATACTGAGATTACCAAGGCGCCGTTATCCGCT
TCAATGAT
CAAAAGGTACGATGAACATCACCAAGACTTGACACTTCTCAAGGCCCTAGTC
CGTCAGCA
ACTGCCTGAGAAATATAAGGAAATATTCTTTGATCAGTCGAAAAACGGGTAC
GCAGGTTA
TATTGACGGCGGAGCGAGTCAAGAGGAATTCTACAAGTTTATCAAACCCATA
TTAGAGAA
GATGGATGGGACGGAAGAGTTGCTTGTAAAACTCAATCGCGAAGATCTACTG
CGAAAGC
AGCGGACTTTCGACAACGGTAGCATTCCACATCAAATCCACTTAGGCGAATT
GCATGCTA
 
TACTTAGAAGGCAGGAGGATTTTATCCGTTCCTCAAAGACAATCGTGAAAA
GATTGAGA
AAATCCTAACCTTTCGCATACCTTACTATGTGGACCCCTGGCCCGAGGAA
CTCTCGGTT
CGCATGGATGACAAGAAAGTCCGAAGAAACGATTACTCCATGGAATTTTGAG
GAAGTTGT
CGATAAAGGTGCGTCAGCTCAATCGTTCATCGAGAGGATGACCAACTTTGAC
AAGAATTT
ACCGAACGAAAAGTATTGCCTAAGCACAGTTTACTTTACGAGTATTTCACA
GTGTACAA
TGAACTCACGAAAGTTAAGTATGTCACTGAGGGCATGCGTAAACCCGCCTTT
CTAAGCGG
AGAACAGAAGAAAGCAATAGTAGATCTGTTATTCAAGACCAACCGCAAAGT
GACAGTTA
AGCAATTGAAAGAGGACTACTTTAAGAAAATTGAATGCTTCGATTCTGTCGA
GATCTCCG
GGGTAGAAGATCGATTTAATGCGTCACTTGGTACGTATCATGACCTCCTAAA
GATAATTA
AAGATAAGGACTTCCTGGATAACGAAGAGAATGAAGATATCTTAGAAGATAT
AGTGTTGA
CTCTTACCCTCTTTGAAGATCGGGAAATGATTGAGGAAAGACTAAAAACATA
CGCTCACC
TGTTCGACGATAAGGTTATGAAACAGTTAAAGAGGCGTCGCTATACGGGCTG
GGGACGAT
TGTCGCGGAAACTTATCAACGGGATAAGAGACAAGCAAAGTGGTAAAACTAT
TCTCGATT
TTCTAAAGAGCGACGGCTTCGCCAATAGGAACTTTATGCAGCTGATCCATGA
TGACTCTTT
AACCTTCAAAGAGGATATACAAAAGGCACAGGTTTCCGGACAAGGGGACTC
ATTGCACG
AACATATTGCGAATCTTGCTGGTTCGCCAGCCATCAAAAAGGGCATACTCCA
 
GACAGTCA
AAGTAGTGGATGAGCTAGTTAAGGTCATGGGACGTCACAAACCGGAAAACAT
TGTAATCG
AGATGGCACGCGAAAATCAAACGACTCAGAAGGGGCAAAAAAACAGTCGAG
AGCGGAT
GAAGAGAATAGAAGAGGGTATTAAAGAACTGGGCAGCCAGATCTTAAAGGA
GCATCCTG
TGGAAAATACCCAATTGCAGAACGAGAAACTTTACCTCTATTACCTACAAAA
TGGAAGGG
ACATGTATGTTGATCAGGAACTGGACATAAACCGTTTATCTGATTACGACGTC
GATGCCAT
TGTACCCCAATCCTTTTTGAAGGACGATTCAATCGACAATAAAGTGCTTACAC
GCTCGGAT
AAGAACCGAGGGAAAAGTGACAATGTTCCAAGCGAGGAAGTCGTAAAGAAA
ATGAAGA
ACTATTGGCGGCAGCTCCTAAATGCGAAACTGATAACGCAAAGAAAGTTCGA
TAACTTAA
CTAAAGCTGAGAGGGGTGGCTTGTCTGAACTTGACAAGGCCGGATTTATTAA
ACGTCAGC
TCGTGGAAACCCGCCAAATCACAAAGCATGTTGCACAGATACTAGATTCCCG
AATGAATA
CGAAATACGACGAGAACGATAAGCTGATTCGGGAAGTCAAAGTAATCACTTT
AAAGTCA
AAATTGGTGTCGGACTTCAGAAAGGATTTTCAATTCTATAAAGTTAGGGAGA
TAAATAAC
TACCACCATGCGCACGACGCTTATCTTAATGCCGTCGTAGGGACCGCACTCAT
TAAGAAA
TACCCGAAGCTAGAAAGTGAGTTTGTGTATGGTGATTACAAAGTTTATGACG
TCCGTAAG
ATGATCGCGAAAAGCGAACAGGAGATAGGCAAGGCTACAGCCAAATACTTC
TTTTATTCT
 
AACATTATGAATTTCTTTAAGACGGAAATCACTCTGGCAAACGGAGAGATAC
GCAAACGA
CCTTTAATTGAAACCAATGGGGAGACAGGTGAAATCGTATGGATAAGGGCC
GGGACTTC
GCGACGGTGAGAAAAGTTTTGTCCATGCCCCAAGTCAACATAGTAAAGAAAA
CTGAGGTG
CAGACCGGAGGGTTTTCAAAGGAATCGATTCTTCCAAAAAGGAATAGTGATA
AGCTCATC
GCTCGTAAAAAGGACTGGGACCCGAAAAAGTACGGTGGCTTCGATAGCCCTA
CAGTTGCC
TATTCTGTCCTAGTAGTGGCAAAAGTTGAGAAGGGAAAATCCAAGAAACTGA
AGTCAGTC
AAAGAATTATTGGGGATAACGATTATGGAGCGCTCGTCTTTTGAAAAGAACC
CCATCGAC
TTCCTTGAGGCGAAAGGTTACAAGGAAGTAAAAAAGGATCTCATAATTAAAC
TACCAAAG
TATAGTCTGTTTGAGTTAGAAAATGGCCGAAAACGGATGTTGGCTAGCGCCG
GAGAGCTT
CAAAAGGGGAACGAACTCGCACTACCGTCTAAATACGTGAATTTCCTGTATT
TAGCGTCC
CATTACGAGAAGTTGAAAGGTTCACCTGAAGATAACGAACAGAAGCAACTTT
TTGTTGAG
CAGCACAAACATTATCTCGACGAAATCATAGAGCAAATTTCGGAATTCAGTA
AGAGAGTC
ATCCTAGCTGATGCCAATCTGGACAAAGTATTAAGCGCATACAACAAGCACA
GGGATAAA
CCCATACGTGAGCAGGCGGAAAATATTATCCATTTGTTTACTCTTACCAACCT
CGGCGCTC
CAGCCGCATTCAAGTATTTTGACACAACGATAGATCGCAAACGATACACTTC
TACCAAGG
 
AGGTGCTAGACGCGACACTGATTCACCAATCCATCACGGGATTATATGAAAC
TCGGATAG ATTTGTCACAGCTTGGGGGTGACTAA
**SEQ ID NO: 57**
   NAME: ABBIE1 (NLS-linker1-Integrase-linker2-dCas9) -DNA sequence
   SEQUENCE: 
ATGGACTACAAAGACCATGACGGTGATTATAAAGATCATGACATCGATTACA
AGGATGAC
GATGACAAGATGGCCCCCAAGAAGAAGAGGAAGGTGGGCATTCACCGCGGG
GTACCT
GGGGGAAGTATGTTCCTGGACGGTATCGACAAAGCTCAGGACGAGCACGAA
AAGTACCATTCTAACTGGCGCGCCATGGCCTCTGACTTCAATCTCCCGCCGGT
TGTTGCCAAGGAGATCGTGGCTTCTTGCGACAAGTGCCAATTGAA
GGGTGAGGCTATGCATGGTCAGGTCGATTGCTCTCCCGGTATCTGGCAGCTG
GACTGCACTCACCTCGAG
GGTAAGGTGATTCTCGTTGCTGTGCACGTGGCTTCCGGCTACATCGAGGCTGA
GGTCATCCCGGCTGAGA
CCGGTCAAGAGACTGCTTACTTCCTGCTCAAGCTGGCCGGCCGTTGGCCAGTT
AAGACTATTCACACTGA
TAACGGTTCTAACTTTACTTCCGCAACTGTGAAAGCTGCATGCTGGTGGGCCG
GCATTAAACAAGAGTTC
GGAATTCCGTATAACCCGCAGTCTCAGGGCGTTGTCGAGTCTATGAACAAGG
AGCTCAAAAAGATCATTG
GTCAAGTCCGTGACCAAGCTGAGCACCTTAAGACCGCTGTGCAGATGGCTGT
TTTTATTCATAACTTCAA
GCGTAAGGGTGGTATCGGTGGTTATAGCGCTGGTGAGCGTATCGTAGACATC
ATCGCTACTGATATCCAG
ACAAAGGAGCTGCAGAAGCAGATCACTAAGATCCAGAACTTCCGTGTGTACT
ATCGGGACTCTAGGAACC
CGCTCTGGAAGGGTCCTGCTAAACTGCTGTGGAAGGGAGAGGGTGCTGTTGT
TATCCAGGACAACTCTGA
 
TATCAAGGTGGTTCCGCGTCGTAAGGCTAAAATTATCCGCGACTACGGCAAG
CAAATGGCTGGAGACGAC
TGCGTTGCTAGCCGTCAAGACGAAGACagcggcagcgaaaccccgggcaccagcgaaagcgcga
ccccggaaagc
ATGGATAAAAAGTATTCTATTGGTTTAGCTATCGGCACTAATTCCGTTGGATG
GGCTGTCA
TAACCGATGAATACAAAGTACCTTCAAAGAAATTTAAGGTGTTGGGAACAC
AGACCGTC
ATTCGATTAAAAAGAATCTTATCGGTGCCCTCCTATTCGATAGTGGCGAAACG
GCAGAGG
CGACTCGCCTGAAACGAACCGCTCGGAGAAGGTATACACGTCGCAAGAACC
GAATATGTT
ACTTACAAGAAATTTTTAGCAATGAGATGGCCAAAGTTGACGATTCTTTCTTT
CACCGTTT
GGAAGAGTCCTTCCTTGTCGAAGAGGACAAGAAACATGAACGGCACCCCATC
TTTGGAAA
CATAGTAGATGAGGTGGCATATCATGAAAAGTACCCAACGATTTATCACCTC
AGAAAAAA
GCTAGTTGACTCAACTGATAAAGCGGACCTGAGGTTAATCTACTTGGCTCTTG
CCCATATG
ATAAAGTTCCGTGGGCACTTTCTCATTGAGGGTGATCTAAATCCGGACAACTC
GGATGTC
GACAAACTGTTCATCCAGTTAGTACAAACCTATAATCAGTTGTTTGAAGAGA
ACCCTATA
AATGCAAGTGGCGTGGATGCGAAGGCTATTCTTAGCGCCCGCCTCTCTAAAT
CCCGACGG
CTAGAAAACCTGATCGCACAATTACCCGGAGAGAAGAAAAATGGGTTGTTCG
GTAACCTT
ATAGCGCTCTCACTAGGCCTGACACCAAATTTTAAGTCGAACTTCGACTTAGC
TGAAGAT
GCCAAATTGCAGCTTAGTAAGGACACGTACGATGACGATCTCGACAATCTAC
 
TGGCACAA
ATTGGAGATCAGTATGCGGACTTATTTTGGCTGCCAAAAACCTTAGCGATGC
AATCCTCC
TATCTGACATACTGAGAGTTAATACTGAGATTACCAAGGCGCCGTTATCCGCT
TCAATGAT
CAAAAGGTACGATGAACATCACCAAGACTTGACACTTCTCAAGGCCCTAGTC
CGTCAGCA
ACTGCCTGAGAAATATAAGGAAATATTCTTTGATCAGTCGAAAAACGGGTAC
GCAGGTTA
TATTGACGGCGGAGCGAGTCAAGAGGAATTCTACAAGTTTATCAAACCCATA
TTAGAGAA
GATGGATGGGACGGAAGAGTTGCTTGTAAAACTCAATCGCGAAGATCTACTG
CGAAAGC
AGCGGACTTTCGACAACGGTAGCATTCCACATCAAATCCACTTAGGCGAATT
GCATGCTA
TACTTAGAAGGCAGGAGGATTTTTATCCGTTCCTCAAAGACAATCGTGAAAA
GATTGAGA
AAATCCTAACCTTTCGCATACCTTACTATGTGGGACCCCTGGCCCGAGGGAA
CTCTCGGTT
CGCATGGATGACAAGAAAGTCCGAAGAAACGATTACTCCATGGAATTTTGAG
GAAGTTGT
CGATAAAGGTGCGTCAGCTCAATCGTTCATCGAGAGGATGACCAACTTTGAC
AAGAATTT
ACCGAACGAAAAAGTATTGCCTAAGCACAGTTTACTTTACGAGTATTTCACA
GTGTACAA
TGAACTCACGAAAGTTAAGTATGTCACTGAGGGCATGCGTAAACCCGCCTTT
CTAAGCGG
AGAACAGAAGAAAGCAATAGTAGATCTGTTATTCAAGACCAACCGCAAAGT
GACAGTTA
AGCAATTGAAAGAGGACTACTTTAAGAAAATTGAATGCTTCGATTCTGTCGA
GATCTCCG
 
GGGTAGAAGATCGATTTAATGCGTCACTTGGTACGTATCATGACCTCCTAAA
GATAATTA
AAGATAAGGACTTCCTGGATAACGAAGAGAATGAAGATATCTTAGAAGATAT
AGTGTTGA
CTCTTACCCTCTTTGAAGATCGGAAATGATTGAGGAAAGACTAAAAACATA
CGCTCACC
TGTTCGACGATAAGGTTATGAAACAGTTAAAGAGGCGTCGCTATACGGGCTG
GGGACGAT
TGTCGCGGAAACTTATCAACGGGATAAGAGACAAGCAAAGTGGTAAAACTAT
TCTCGATT
TTCTAAAGAGCGACGGCTTCGCCAATAGGAACTTTATGCAGCTGATCCATGA
TGACTCTTT
AACCTTCAAAGAGGATATACAAAAGGCACAGGTTTCCGGACAAGGGGACTC
ATTGCACG
AACATATTGCGAATCTTGCTGGTTCGCCAGCCATCAAAAAGGGCATACTCCA
GACAGTCA
AAGTAGTGGATGAGCTAGTTAAGGTCATGGGACGTCACAAACCGGAAAACAT
TGTAATCG
AGATGGCACGCGAAAATCAAACGACTCAGAAGGGGCAAAAAAACAGTCGAG
AGCGGAT
GAAGAGAATAGAAGAGGGTATTAAAGAACTGGGCAGCCAGATCTTAAAGGA
GCATCCTG
TGGAAAATACCCAATTGCAGAACGAGAAACTTTACCTCTATTACCTACAAAA
TGGAAGGG
ACATGTATGTTGATCAGGAACTGGACATAAACCGTTTATCTGATTACGACGTC
GATGCCAT
TGTACCCCAATCCTTTTTGAAGGACGATTCAATCGACAATAAAGTGCTTACAC
GCTCGGAT
AAGAACCGAGGGAAAAGTGACAATGTTCCAAGCGAGGAAGTCGTAAAGAAA
ATGAAGA
ACTATTGGCGGCAGCTCCTAAATGCGAAACTGATAACGCAAAGAAAGTTCGA
 
TAACTTAA
CTAAAGCTGAGAGGGGTGGCTTGTCTGAACTTGACAAGGCCGGATTTATTAA
ACGTCAGC
TCGTGGAAACCCGCCAAATCACAAAGCATGTTGCACAGATACTAGATTCCCG
AATGAATA
CGAAATACGACGAGAACGATAAGCTGATTCGGGAAGTCAAAGTAATCACTTT
AAAGTCA
AAATTGGTGTCGGACTTCAGAAAGGATTTTCAATTCTATAAAGTTAGGGAGA
TAAATAAC
TACCACCATGCGCACGACGCTTATCTTAATGCCGTCGTAGGGACCGCACTCAT
TAAGAAA
TACCCGAAGCTAGAAAGTGAGTTTGTGTATGGTGATTACAAAGTTTATGACG
TCCGTAAG
ATGATCGCGAAAAGCGAACAGGAGATAGGCAAGGCTACAGCCAAATACTTC
TTTTATTCT
AACATTATGAATTTCTTTAAGACGGAAATCACTCTGGCAAACGGAGAGATAC
GCAAACGA
CCTTTAATTGAAACCAATGGGGAGACAGGTGAAATCGTATGGGATAAGGGCC
GGGACTTC
GCGACGGTGAGAAAAGTTTTGTCCATGCCCCAAGTCAACATAGTAAAGAAAA
CTGAGGTG
CAGACCGGAGGGTTTTCAAAGGAATCGATTCTTCCAAAAAGGAATAGTGATA
AGCTCATC
GCTCGTAAAAAGGACTGGGACCCGAAAAAGTACGGTGGCTTCGATAGCCCTA
CAGTTGCC
TATTCTGTCCTAGTAGTGGCAAAAGTTGAGAAGGGAAAATCCAAGAAACTGA
AGTCAGTC
AAAGAATTATTGGGGATAACGATTATGGAGCGCTCGTCTTTTGAAAAGAACC
CCATCGAC
TTCCTTGAGGCGAAAGGTTACAAGGAAGTAAAAAAGGATCTCATAATTAAAC
TACCAAAG
 
TATAGTCTGTTTGAGTTAGAAAATGGCCGAAAACGGATGTTGGCTAGCGCCG
GAGAGCTT
CAAAAGGGGAACGAACTCGCACTACCGTCTAAATACGTGAATTTCCTGTATT
TAGCGTCC
CATTACGAGAAGTTGAAAGGTTCACCTGAAGATAACGAACAGAAGCAACTTT
TTGTTGAG
CAGCACAAACATTATCTCGACGAAATCATAGAGCAAATTTCGGAATTCAGTA
AGAGAGTC
ATCCTAGCTGATGCCAATCTGGACAAAGTATTAAGCGCATACAACAAGCACA
GGGATAAA
CCCATACGTGAGCAGGCGGAAAATATTATCCATTTGTTTACTCTTACCAACCT
CGGCGCTC
CAGCCGCATTCAAGTATTTTGACACAACGATAGATCGCAAACGATACACTTC
TACCAAGG
AGGTGCTAGACGCGACACTGATTCACCAATCCATCACGGGATTATATGAAAC
TCGGATAG ATTTGTCACAGCTTGGGGGTGACTAA
**SEQ ID NO: 58**
   NAME: Translation of ABBIE1 (A Binding Based Integrase Editor)
   SEQUENCE: 
Met D Y K D H D G D Y K D H D I D Y K D D D D K Met A P K K K R K V G I H R
G V P G G S Met F L D G I D K A Q D E H E K Y H S N W R A Met A S D F N L P P
V V A K E I V A S C D K C Q L K G E A Met H G Q V D C S P G I W Q L D C T H L
E G K V I L V A V H V A S G Y I E A E V I P A E T G Q E T A Y F L L K L A G R W
P V K T I H T D N G S N F T S A T V K A A C W W A G I K Q E F G I P Y N P Q S Q
G V V E S Met N K E L K K I I G Q V R D Q A E H L K T A V Q Met A V F I H N F
K R K G G I G G Y S A G E R I V D I I A T D I Q T K E L Q K Q I T K I Q N F R V Y
Y R D S R N P L W K G P A K L L W K G E G A V V I Q D N S D I K V V P R R K A
K I I R D Y G K Q Met A G D D C V A S R Q D E D S G S E T P G T S E S A T P E S
Met D K K Y S I G L A I G T N S V G W A V I T D E Y K V P S K K F K V L G N T
D R H S I K K N L I G A L L F D S G E T A E A T R L K R T A R R R Y T R R K N R
I C Y L Q E I F S N E Met A K V D D S F F H R L E E S F L V E E D K K H E R H P I
 
F G N I V D E V A Y H E K Y P T I Y H L R K K L V D S T D K A D L R L I Y L A L
A H Met I K F R G H F L I E G D L N P D N S D V D K L F I Q L V Q T Y N Q L F E
E N P I N A S G V D A K A I L S A R L S K S R R L E N L I A Q L P G E K K N G L
F G N L I A L S L G L T P N F K S N F D L A E D A K L Q L S K D T Y D D D L D N
L L A Q I G D Q Y A D L F L A A K N L S D A I L L S D I L R V N T E I T K A P L S
A S Met I K R Y D E H H Q D L T L L K A L V R Q Q L P E K Y K E I F F D Q S K N
G Y A G Y I D G G A S Q E E F Y K F I K P I L E K Met D G T E E L L V K L N R E
D L L R K Q R T F D N G S I P H Q I H L G E L H A I L R R Q E D F Y P F L K D N
R E K I E K I L T F R I P Y Y V G P L A R G N S R F A W Met T R K S E E T I T P W
N F E E V V D K G A S A Q S F I E R Met T N F D K N L P N E K V L P K H S L L Y
E Y F T V Y N E L T K V K Y V T E G Met R K P A F L S G E Q K K A I V D L L F
K T N R K V T V K Q L K E D Y F K K I E C F D S V E I S G V E D R F N A S L G T
Y H D L L K I I K D K D F L D N E E N E D I L E D I V L T L T L F E D R E Met I E
E R L K T Y A H L F D D K V Met K Q L K R R R Y T G W G R L S R K L I N G I R
D K Q S G K T I L D F L K S D G F A N R N F Met Q L I H D D S L T F K E D I Q K
A Q V S G Q G D S L H E H I A N L A G S P A I K K G I L Q T V K V V D E L V K
V Met G R H K P E N I V I E Met A R E N Q T T Q K G Q K N S R E R Met K R I E E
G I K E L G S Q I L K E H P V E N T Q L Q N E K L Y L Y Y L Q N G R D Met Y V
D Q E L D I N R L S D Y D V D A I V P Q S F L K D D S I D N K V L T R S D K N R
G K S D N V P S E E V V K K Met K N Y W R Q L L N A K L I T Q R K F D N L T K
A E R G G L S E L D K A G F I K R Q L V E T R Q I T K H V A Q I L D S R Met N T
K Y D E N D K L I R E V K V I T L K S K L V S D F R K D F Q F Y K V R E I N N Y
H H A H D A Y L N A V V G T A L I K K Y P K L E S E F V Y G D Y K V Y D V R
K Met I A K S E Q E I G K A T A K Y F F Y S N I Met N F F K T E I T L A N G E I R
K R P L I E T N G E T G E I V W D K G R D F A T V R K V L S Met P Q V N I V K K
T E V Q T G G F S K E S I L P K R N S D K L I A R K K D W D P K K Y G G F D S P
T V A Y S V L V V A K V E K G K S K K L K S V K E L L G I T I Met E R S S F E K
N P I D F L E A K G Y K E V K K D L I I K L P K Y S L F E L E N G R K R Met L A
S A G E L Q K G N E L A L P S K Y V N F L Y L A S H Y E K L K G S P E D N E Q
K Q L F V E Q H K H Y L D E I I E Q I S E F S K R V I L A D A N L D K V L S A Y
 
N K H R D K P I R E Q A E N I I H L F T L T N L G A P A A F K Y F D T T I D R K R
Y T S T K E V L D A T L I H Q S I T G L Y E T R I D L S Q L G G D Stop
   For donor DNA (att sites of LTR regions for integrase recognition).
**SEQ ID NO: 59**
   NAME: U3att
   SEQUENCE:
      ACTGGAAGGGCTAATTCACTCCCAAAGAA
**SEQ ID NO: 60**
   NAME: U5att
   SEQUENCE:
      GACCCTTTTAGTCAGTGTGGAAAATCTCTAGCAGT
   NLS-linker1-Integrase-linker2-dCas9, or Integrase-linker1-NLS-linker2-dCas9 or Integrase-linker2-dCas9-linker1-NLS or Integrase-linker2-dCas9-NLS
   Linker 1 = GGS
**SEQ ID NO: 61**
   NAME: Linker 2
   SEQUENCE:
      SGSETPGTSESATPES
**SEQ ID NO: 62**
   NAME: MMTV integrase cDNA, gb|AF071010.1|:16-1113 Mouse mammary tumor virus putative integrase, env polyprotein, and superantigen mRNA, complete cds
   SEQUENCE: 
ATGACAGGAAAGTGGCCTTGTATTTACTCCACTAACTGCAGAGATGTGTTGC
ATGGGACGGGGGCACTG
CACCAGCCCTCGTGCTGAATTCGGCACGAGGAAATGCCTATGCAGATTCTTTA
ACAAGAATTCTGACCGC
TTTAGAGTCAGCTCAAGAAAGCCACGCACTGCACCATCAAAATGCCGCGGCG
CTTAGGTTTCAGTTTCAC
ATCACTCGTGAACAAGCACGAGAAATAGTAAAATTATGTCCAAATTGCCCCG
ACTGGGGACATGCACCAC
 
AACTAGGAGTAAACCCTAGGGGCCTTAAGCCCGGGGTTCTATGGCAAATGGA
TGTTACTCATGTCTCAGA
ATTTGGAAAATTAAAGTATGTACATGTGACAGTGGATACTTACTCTCATTTTA
CTTTCGCTACCGCCCGG
ACGGGCGAAGCAGCCAAAGATGTGTTACAACACTTGGCTCAAAGCTTTGCAT
ACATGGGCATTCCTCAAA
AAATAAAAACAGATAATGCCCCTGCCTATGTGTCTCGTTCAATACAAGAATTT
CTGGCCAGATGGAAAAT
ATCTCACGTCACGGGGATCCCTTACAATCCCCAAGGACAGGCCATTGTTGAA
CGAACGCACCAAAATATA
AAGGCACAGATTAATAAACTTCAAAAGGCTGGAAAATACTATACACCCCACC
ATCTATTGGCACATGCTC
TTTTTGTGCTGAATCATGTAAATATGGACAATCAAGGCCATACAGCGGCCGA
AAGACATTGGGGTCCAAT
CTCAGCCGATCCAAAACCTATGGTCATGTGGAAAGACCTTCTCACAGGGTCC
TGGAAAGGACCCGATGTC
CTAATAACAGCCGGACGAGGCTATGCTTGTGTTTTCCACAGGATGCCGAATC
ACCAATCTGGGTCCCCG
ACCGGTTCATCCGACCTTTTACTGAGCGGAAAGAAGCAACGCCCACACCTGG
CACTGCGGAGAAACGCC
GCCGCGAGATGAGAAAGATCAACAGGAAAGTCCGGAGGATGAATCTTGCCC
CCATCAAAGAGAAGACGGC
TTGGCAACATCTGCAGGCGTTAATCTCCGAAGCGGAGGAGGTTCTTAA
**SEQ ID NO: 63**
   NAME: gi|3273866|gb|AAC24859.1| putative integrase [Mouse mammary tumor virus]
   SEQUENCE: 
MTGKWPCIYSTNCRDVLHGTGGTAPALVLNSARGNAYADSLTRILTALESAQES
HALHHQNAAALRFQFH
ITREQAREIVKLCPNCPDWGHAPQLGVNPRGLKPGVLWQMDVTHVSEFGKLKY
VHVTVDTYSHFTFATAR
 
TGEAAKDVLQHLAQSFAYMGIPQKIKTDNAPAYVSRSIQEFLARWKISHVTGIPY
NPQGQAIVERTHQNI
KAQINKLQKAGKYYTPHHLLAHALFVLNHVNMDNQGHTAAERHWGPISADPKP
MVMWKDLLTGSWKGPDV
LITAGRGYACVFPQDAESPIWVPDRFIRPFTERKEATPTPGTAEKTPPRDEKDQQE
SPEDESCPHQREDG
LATSAGVNLRSGGGS
**SEQ ID NO: 64**
   NAME: gb|AXUN02000059.1|:5116-8850 Youngiibacter fragilis 232.1 contig_151, whole genome shotgun sequence - recombinase
   SEQUENCE: 
TTGAAAGATAACGATAAAAGGATGTGGGTTCAGACTTTATGGAATCCCATCA
ATGAAAGACATAAAAGTC
CACTGGATAGCCCAGAACCAGGGATTAAAGTAGCGGCCTACTGCAGAGTAAG
CATGAAAGAGGAGGAACA
ACTCCGGTCATTGGAAAACCAGGTGCATCACTATACTCATTTTATCAAAAGTA
AGCCGAATTGGAGATTT
GTAGGGGTTTATTACGATGATGGCATAAGTGCAGCCATGGCAAGTGGGAGAA
GAGGGTTCCAGCGGATTA
TCCGTCATGCTGAAGAAGGTAAGGTTGATCTGATTCTAACAAAGAATATTTC
ACGGTTTTCCAGAAATTC
CAAGGAGTTACTGGATATAATCAATCAACTGAAAGCTATCGGTGTGGGCATC
TATTTTGAGAAAGAGAAT
ATTGATACTTCAAGAGAGTACAATAAATTCCTCTTAAGCACTTATGCTGCGCT
GGCACAGGAAGAGATAG
AAACTATTTCAAACTCTACGATGTGGGGTTATGAGAAAAGGTTTCTAAAGGG
TATCCCAAAGTTCAACCG
CTTATATGGATACAAAGTCATCCATGCAGGGGATGATTCCCAATTGATTGTTC
TTGAAGATGAAGCAAAA
ATCGTAAGAATGATGTATGAACAGTACCTTCAAGGGAAGACGTTCACTGATA
TTGCAAGGGCGCTAACAG
 
AAGCTGGAGTGAAAACAGCCAAAGGGAAGGATGTCTGGATAGGCGGCATGA
TAAAGCATATTTATCCAA
CGTCACCTACACCGGTAACAAGCTTACACGAGAACTGAAAAGAGATTTATTT
ACGAACAAAGTTAATAGC
GGTGAACGGGATCAGGTTTTTATAGGAAACACTCACGAACCGATCATCAGCA
ATGATATTTTCAATCTTG
TTCAAAAGAAGCTTGAGGCCAATACGAAGGAAAGAAAGCCCAGTGAGAAGC
GAGAGAAGAACCACATGTC
TGGTCGGCTACTTTGCGGAAGATGTGGATACAGTTTTACCATAATTCACAATA
GAGCTTCTCATCACTTT
AAGTGTAGCCCTAAAATCATGGGGGTCTGTGATTCTGAACTTTATCGGGATGC
GGATATTCGAGAAATGA
TGATGAGGGCAATGTATATAAAATATGACTTCACCGATGAAGACATAGTACT
AAAACTGCTGAAGGAACT
CCAGGTCATCAATCAAAATGATCACTTTGAGTTTCATAGGCTAAAGTTTATCA
CTGAAATTGAAATCGTA
AAAAGGCAGCAGGCCATTTCAGATAGATATTCAGCTATTAGCATAGAAAAAA
TGGAAGAAGAATACCGCA
CTTTTGAAAGCAAGATTGCGAAAATTGAGGATGACAGGTACATCAGAATCGA
TGCAGTGGAGTGGTTAAA
GAAAAACAAGACGCTGGATTCTTTTATCGCTCAGGTCACCACTAAAATATTG
CGAGCTTGGGTTTCCGAG
ATGACTGTTTATACACGAGATGACTTTTTAGTGCAGTGGATTGACGGAACTCA
AACTGAGATAGGAAGCT
GCGAGCATCATCTTGTGAAGGATAGAAATAGTAAGAGTTACGAGTCCGGTGA
AGAAACGAGCAGGAGGGC
CAAATTTGAAGTCAACCACATTAGTGAAACCACCGAAGGACAAGGAGAACTT
GATCTCTTAAGCAAGAGT
GCAAGTTCAAACAATGAAGATAGTAATCAACCAGAAAATAATTCTACGGGAA
AGGAGGAGCTTGAATTGA
 
ACTTAAACAGTAATGCAGAAATTATCAAAATTGAGCCCGGGCAAAGGACTA
TATTATGAAGAATTTGCA
CAAGAGCCTGAGTGCAAATATGATGATGCAAAATGCTTCAGTACACACGGCA
AGTATTAACAAACCTAGA
CTTAAGACTGCTGCTTACTGCAGAATCTCAACAGATTCAGAAGAACAAAAGG
TAAGCTTGAAAACCCAAG
TAGCCTATTACACTTATCTGATTCTAAAGGATCCCCAATATGAATATGCAGGC
ATCTATGCCGATGAAGG
TATATCAGGGCGTTCTATGAAAAACCGTACAGAATTTCTCAAACTACTCGAA
GAATGTAAAGCCGGGAAT
GTGGACTTGATTTTAACCAAGTCAATCTCACGGTTTAGCAGAAACGCATTAG
ATTGCTTGGAACAGATCA
GGATGCTGAAGTCGCTGCCAAGTCCAGTTTATGTGTATTTTGAGAAAGAGAA
TATTCATACAAAAGATGA
GAAGAGTGAGCTGATGATTTCTATTTTTGGAAGTATCGCTCAGGAAGAGAGC
GTAAACATGGGAGAAGCC
ATGGCTTGGGGAAAACGGAGATATGCTGAGAGAGGGATAGTAAACCCAAGT
GTTGCACCTTATGGATATA
GAACGGTCAGAAAAGGTGAATGGGAGGTGGTTGAAGAAGCTACGATCA
TTAGAAGAATTTATCGGAT
GCTCCTAAGTGGAAAGAGTATTCATGAAATCACAAAGGAGCTCTCCATGGAG
AAGATAAAGGGTCCTGGC
GGCAACGAGCAGTGGCATCTTCAAACCATTAGAAATATCTTGAGAAATGAAA
TCTATAGGGGTAACTACC
TTTATCAAAAGGCTTATATCAAGGACACGATCGAGAAGAAGGTGGTAATGAA
TCGAGGAGAACTGCCACA
GTATCTCATAGAGAATCATCATAAAGCCATTGTTGACAATGAGACCTGGGAA
AAGGTCCAGAAGGTACTA
GAAGCCAGAAGGGAAAAATATGAGAATAAAAAGTCCATAACTTATCCTGAA
GACAAAATGAAAAACGCTT
 
CTCTTGAAGATATTTTTACCTGTGGAGAATGTGGAAGTAAAATAGGCCATAG
AAGGAGCATCCAGAGCTC
TAATGAGATTCATTCCTGGATCTGCACAAAAGCCGCTAAGTCTTTCTTGGTGG
ACTCGTGTAAGTCCACA
AGCGTATATCAGAAGCACCTGGAGCTGCATTTTATGAAGACTCTTCTCGATAT
TAAAAAGCATCGTTCTT
TCAAAGATGAGGTGCTCACCTATATTCGAACCCAAGAAGTAGATGAAAAGGA
AGAGTGGAGAATCAAAGT
CATAGAGAAACGAATCAAAGATCTTAACAGAGAGCTTTATAATGCGGTAGAC
CAGGAGCTCAATAAAAAA
GGTCAGGACTCCAGGAAAGTTGATGAGCTCACAGAGAAAATTGTGGATCTTC
AAGAGGAATTAAAGGTGT
TTAGGGACCGAAAGGCAAAGGTTGAGGATCTTAAAGCTGAGCTTGAATGGTT
CCTAAAGAAGCTGGAAAC
CATTGATGACGCTCGAGTAAAAAGAAATGAAGGAATAGGCCACGGTGAAGA
GATCTACTTCAGAGAAGAT
ATTTTTGAAAGAATAGTAAGGAGTGCACAGCTTTATAGCGATGGAAGGATCG
TCTACGAACTAAGCCTCG
GGATCCAGTGGTTCATTGACTTTAAATACAGCGCATTTCAGAAGCTTCTTATA
AAGTGGAAGGATAAACA
AAGGGCAGAAGAAAAAGAGGCTTTTCTTGAGGGGCCGGAAGTTAAAGAGCT
GCTGGAATTTTGTAAGGAA
CCGAAGAGCTACTCTGATTTACATGCCTTCATGTGTGAGAGAAAAGAGGTGT
CTTATAGCTATTTCAGGA
AATTGGTGATAAGACCTTTGATGAAGAAAGGAAAGCTGAAGTTCACCATACC
AGAAGATGTTATGAATAG
GCATCAGAGATACACATCAATCTAA
**SEQ ID NO: 65**
   NAME: gi|564135645|gb|ETA81829.1| recombinase [Youngiibacter fragilis 232.1]
   SEQUENCE: 
MKDNDKRMWVQTLWNPINERHKSPLDSPEPGIKVAAYCRVSMKEEEQLRSLEN
QVHHYTHFIKSKPNWRF
VGVYYDDGISAAMASGRRGFQRIIRHAEEGKVDLILTKNISRFSRNSKELLDIINQ
LKAIGVGIYFEKEN
IDTSREYNKFLLSTYAALAQEEIETISNSTMWGYEKRFLKGIPKFNRLYGYKVIHA
GDDSQLIVLEDEAK
IVRMMYEQYLQGKTFTDIARALTEAGVKTAKGKDVWIGGMIKHILSNVTYTGN
KLTRELKRDLFTNKVNS
GERDQVFIGNTHEPIISNDIFNLVQKKLEANTKERKPSEKREKNHMSGRLLCGRC
GYSFTIIHNRASHHF
KCSPKIMGVCDSELYRDADIREMMMRAMYIKYDFTDEDIVLKLLKELQVINQND
HFEFHRLKFITEIEIV
KRQQAISDRYSAISIEKMEEEYRTFESKIAKIEDDRYIRIDAVEWLKKNKTLDSFIA
QVTTKILRAWVSE
MTVYTRDDFLVQWIDGTQTEIGSCEHHLVKDRNSKSYESGEETSRRAKFEVNHIS
ETTEGQGELDLLSKS
ASSNNEDSNQPENNSTGKEELELNLNSNAEIIKIEPGQRDYIMKNLHKSLSANMM
MQNASVHTASINKPR
LKTAAYCRISTDSEEQKVSLKTQVAYYTYLILKDPQYEYAGIYADEGISGRSMKN
RTEFLKLLEECKAGN
VDLILTKSISRFSRNALDCLEQIRMLKSLPSPVYVYFEKENIHTKDEKSELMISIFGS
IAQEESVNMGEA
MAWGKRRYAERGIVNPSVAPYGYRTVRKGEWEVVEEEATIIRRIYRMLLSGKSI
HEITKELSMEKIKGPG
GNEQWHLQTIRNILRNEIYRGNYLYQKAYIKDTIEKKVVMNRGELPQYLIENHH
KAIVDNETWEKVQKVL
EARREKYENKKSITYPEDKMKNASLEDIFTCGECGSKIGHRRSIQSSNEIHSWICT
KAAKSFLVDSCKST
SVYQKHLELHFMKTLLDIKKHRSFKDEVLTYIRTQEVDEKEEWRIKVIEKRIKDL
NRELYNAVDQELNKK
 
GQDSRKVDELTEKIVDLQEELKVFRDRKAKVEDLKAELEWFLKKLETIDDARVK
RNEGIGHGEEIYFRED
IFERIVRSAQLYSDGRIVYELSLGIQWFIDFKYSAFQKLLIKWKDKQRAEEKEAFL
EGPEVKELLEFCKE
PKSYSDLHAFMCERKEVSYSYFRKLVIRPLMKKGKLKFTIPEDVMNRHQRYTSI
**SEQ ID NO: 66**
   NAME: gi|571264543:16423-16770 Clostridium difficile transposon Tn6218, strain Ox42 Transposase
   SEQUENCE: 
TTAGTCTTCAAAAGGTTTTGGACTAAATTACTCTCGTAGTCAGGTCCAAGTG
TTTCTTCAGATTTTTTT
TTCAACCAATCCACCTGCATGGTGAGCTGGCCAACTTTTTCGCATATTCAGC
TTTTTCCTTGCGTTCTA
AAGCGAGTTTTCTTTCAGATTATCCTCTCGTGTGTCATTAAAACCACGGAT
GCTTTATCGAGGAACTC
CTTCTTCCAGTTGCGGAGAAGATTCGGCTGAATATTGTTTCGGTTGCGATTG
TATTTAAGTCTTTTTCT
CCTTTGAGCAGTTCAATCACTAATTCTGATTTGAATTTGGCAGAGAAATTTCT
TCTTGTTCGAGACAT
**SEQ ID NO: 67**
   NAME: gi|571264559|emb|CDF47133.1| transposase [Peptoclostridium difficile]
   SEQUENCE: 
MSRTRRNFSAKFKSELVIELLKGEKDLNTIATENNIQPNLLRNWKKEFLDKASVV
FNDTREDNLKEKLAL
ERKEKAEYAKKVGQLTMQVDWLKKKSEETLGPDYESKFSPKPFED
**SEQ ID NO: 68**
   NAME: gb|CP009444.1|:1317724-1320543 Francisella philomiragia strain GA01-2801, complete genome Cpf1
   SEQUENCE: 
ATGAATCTATATAGTAATCTAACAAATAAATATAGTTTAAGTAAAACTCTAA
GATTTGAGTTAATTCCAC
 
AGGGTGAAACACTTGAAAATATAAAAGCAAGAGGTTTGATTTTAGATGATGA
GAAAAGAGCTAAAGACTA
TAAAAAAGCTAAACAAATCATTGATAAATATCATCAGTTTTTATAGAGGAG
ATATTAAGTTCGGTATGT
ATTAGCGAAGATTTATTACAAAACTATTCTGATGTTTATTTTAAACTTAAAAA
GAGTGATGATGATAATC
TACAAAAAGATTTAAAAGTGCAAAAGATACGATAAAGAAACACATATCTAG
ATATATAAATGACTCGGA
GAAATTTAAGAATTTGTTTAATCAAAATCTTATAGATGCTAAAAAAGGGCAA
GAGTCAGATTTAATTCTA
TGGCTAAAGCAATCTAAGGATAATGGCATAGAACTATTTAAAGCTAACAGTG
ATATCACAGACATAGATG
AGGCGTTAGAAATAATCAAATCTTTTAAAGGTTGGACAACTTATTTTAAGGGT
TTTCATGAAAATAGAAA
AAATGTCTATAGTAGTGATGATATCCCTACATCTATTATTTATAGAATAGTAG
ATGATAATTTGCCTAAA
TTTATAGAAAATAAAGCTAAGTATGAGAATTTAAAAGACAAAGCTCCAGAAG
CTATAAACTATGAACAAA
TTAAAAAAGATTTGGCAGAAGAGCTAACCTTTGATATTGACTACAAAACATC
TGAAGTTAATCAAAGAGT
TTTTTCACTTGATGAAGTTTTTGAGATAGCAAACTTTAATAATTATCTAAATC
AAAGTGGTATTACTAAA
TTTAATACTATTATTGGTGGTAAATTTGTTAATGGTGAAAATACAAAGAGAA
AAGGTATAAATGAATATA
TAAATCTATACTCACAGCAAATAAATGATAAAACACTTAAAAAATATAAAAT
GAGTGTTTTATTTAAGCA
AATTTTAAGTGATACAGAATCTAAATCTTTTGTAATTGATAAGTTAGAAGATG
ATAGTGATGTAGTTACA
ACGATGCAAAGTTTTTATGAGCAAATAGCAGCTTTTAAAACATTAGAAGAAA
AGTCTATTAAGGAAACAT
 
TATCTTTACTATTTGATGATTTAAAAGCTCAAAAACTTGATTTGAGTAAAATT
TATTTTAAAAATGATAA
ATCTCTTACTGATCTATCACAACAAGTTTTGATGATTATAGTGTTATTGGTAC
AGCGGTACTAGAATAT
ATAACTCAACAAGTAGCACCTAAAAATCTTGATAACCCTAGTAAGAAAGAGC
AAGATTTAATAGCCAAAA
AAACTGAAAAAGCAAAATACTTATCTCTAGAAACTATAAAGCTTGCCTTAGA
AGAATTTAATAAGTATAG
AGATATAGATAAACAGTGTAGGTTTGAAGAAATATTTGCAAGCTTTGCAGAT
ATTCCGGTGCTATTTGAT
GAAATAGCTCAAAACAAAAACAATTTGGCACAGATATCTATCAAATATCAAA
ATCAAGGTAAAAAGACC
TGCTTCAAACTAGTGCAGAAGTAGATGTTAAAGCTATCAAGGATCTTTTGGAT
CAAACTAATAATCTCTT
GCATAAACTAAAAATATTTCATATTACGCAATCAGAAGATAAGGCAAATATT
TTAGACAAGGATGAGCAT
TTTTATTTAGTATTTGATGAGTGCTACTTTGAGCTAGCGAATATAGTGGCTCTT
TATAACAAAATTAGAA
ACTATATAACTCAAAAGCCATATAGTGATGAGAAATTTAAGCTCAATTTTGA
GAACTCAACTTTAGCCAA
TGGTTGGGATAAAAATAAAGAGCCTGACAATACGGCAATTTTATTTATCAAA
GATGATAAATATTATCTG
GGTGTGATGAACAAGAAAAATAACAAAATATTTGATGATAAAGCTATCAAAG
AAAATAAAGGTGAAGGAT
ATAAGAAAGTTGTATATAAACTTTTACCCGGTGCAAATAAAATGTTACCTAA
GGTTTTCTTTTCTGCTAA
ATCTATAAATTTTTATAATCCTAGTGAAGATATACTTAGAATAAGAAACCACT
CAACACATACAAAAAAT
GGTAGTCCTCAAAAAGGATATGAAAAACTTGAGTTTAATATTGAAGATTGCC
GAAAATTTATAGATTTTT
 
ATAAACATTCTATAAGTAGGCATCCAGAGTGGAAAGATTTTGGATTTAGATTT
TCTGATACTAAAAAATA
CAACTCTATAGATGAATTTTATAGAGAAGTTGAAAATCAAGGCTACAAACTA
ACTTTTGAAAATATATCA
GAAAGCTATATTGATAGTTTAGTCGATGAAGGCAAATTATACCTATTCCAAAT
CTATAATAAAGATTTCT
CAGTATATAGTAAGGGTAAACCAAATTTACATACGCTATATTGGAAGGCGTT
GTTTGATGAGAGAAATCT
CCAAGATGTAGTATATAAATTAAATGGTGAAGCAGAACTCTTCTATCGTAAA
CAATCAATACCTAAGAAA
ATCACTCACCCAGCCAAAGAGGCAATAGCTAATAAAAACAAAGATAATCCTA
AAAAAGAGAGTATTTTTG
AATATGATTTAATCAAAGATAAACGCTTTACTGAAGATAAGTTTTTCTTTCAC
TGTCCTATTACAATCAA
TTTCAAATCTAGTGGAGCTAATAAGTTTAATGATGAAATCAATTTATTGCTAA
AAGAAAAAGCAAATGAT
GTTCATATCCTAAGTATAGATAGAGGAGAAAGACATTTAGCTTACTATACTTT
GGTAGATGGTAAAGGAA
ACATTATCTGTAAGAATTAA
**SEQ ID NO: 69**
   NAME: gi|754264888|gb|AJI57252.1| CRISPR-associated protein Cpfl, subtype PREFRAN [Francisella philomiragia]
   SEQUENCE: 
MKTNYHDKLAAIEKDRESARKDWKKINNIKEMKEGYLSQVVHEIAKLVIGYNAI
VVFEDLNFGFKRGRFK
VEKQVYQKLEKMLIEKLNYLVFKDNEFDKAGGVLRAYQLTAPFETFKKMGKQT
GIIYYVPADFTSKICPV
TGFVNQLYPKYESVSKSQEFFSKFDKICYNLDKGYFEFSFDYKNFGDKAAKGKW
TIASFGSRLINFRNSD
KNHNWDTREVYPTKELEKLLKDYSIEYGHGECIKAAIYAENDKKFFAKLTSILNS
ILQMRNSKTGTELDY
 
LISPVADVNGNFFDSRHAPKNMPQDADANGAYHIGLKGLMLLYRIKNNQDGKK
LNLVIKNEEYFEFVQNR
NKSSKI
**SEQ ID NO: 70**
   NAME: gi|438609|gb|L21188.1|HIV1NY5A Human immunodeficiency virus type 1 integrase gene, 3' end
   SEQUENCE: 
TTCCTGGACGGTATCGATAAAGCTCAGGAAGAACACGAAAAATACCACTCTA
ACTGGCGCGCCATGGCTT
CTGACTTCAACCTGCCGCCGGTTGTTGCCAAGGAAATCGTGGCTTCTTGCGAC
AAATGCCAATTGAAAGG
TGAAGCTATGCATGGTCAGGTCGACTGCTCTCCAGGTATCTGGCAGCTGGACT
GCACTCATCTCGAGGGT
AAAGTTATCCTGGTTGCTGTTCACGTGGCTTCCGGATACATCGAAGCTGAAGT
TATCCCGGCTGAAACCG
GTCAGGAAACTGCTTACTTCCTGCTTAAGCTGGCCGGCCGTTGGCCGGTTAAA
ACTGTTCACACTGACAA
CGGTTCTAACTTCACTAGTACTACTGTTAAAGCTGCATGCTGGTGGGCCGGCA
TCAAACAGGAGTTCGGG
ATCCCGTACAACCCGCAGTCTCAGGGCGTTATCGAATCTATGAACAAAGAGC
TCAAAAAAATCATTGGCC
AGGTACGTGATCAGGCTGAGCACCTGAAAACCGCGGTGCAGATGGCTGTTTT
CATCCACAACTTCAAACG
TAAAGGTGGTATCGGTGGTTACAGCGCTGGTGAACGTATCGTTGACATCATC
GCTACTGATATCCAGACT
AAAGAACTGCAGAAACAGATCACTAAAATCCAGAACTTCCGTGTATACTACC
GTGACTCTAGAGACCCGG
TTTGGAAAGGTCCTGCTAAACTCCTGTGGAAGGGTGAAGGTGCTGTTGTTATC
CAGGACAACTCTGACAT
CAAAGTGGTACCGCGTCGTAAAGCTAAAATCATTCGCGACTACGGCAAACAG
ATGGCTGGTGACGACTGC
 GTTGCTAGCCGTCAGGACGAAGACTAAAAGCTTCAGGC
**SEQ ID NO: 71**
   NAME: gi|438610|gb|AAC37875.1| integrase, partial [Human immunodeficiency virus 1]
   SEQUENCE: 
FLDGIDKAQEEHEKYHSNWRAMASDFNLPPVVAKEIVASCDKCQLKGEAMHGQ
VDCSPGIWQLDCTHLEG
KVILVAVHVASGYIEAEVIPAETGQETAYFLLKLAGRWPVKTVHTDNGSNFTSTT
VKAACWWAGIKQEFG
IPYNPQSQGVIESMNKELKKIIGQVRDQAEHLKTAVQMAVFIHNFKRKGGIGGYS
AGERIVDIIATDIQT
KELQKQITKIQNFRVYYRDSRDPVWKGPAKLLWKGEGAVVIQDNSDIKVVPRRK
AKIIRDYGKQMAGDDC
VASRQDED
**SEQ ID NO: 72**
   NAME: gi|545612232|ref]WP_021736722.1| type V CRISPR-associated protein Cpfl
   [Acidaminococcus sp. BV3L6]
   SEQUENCE: 
MTQFEGFTNLYQVSKTLRFELIPQGKTLKHIQEQGFIEEDKARNDHYKELKPIIDRI
YKTYADQCLQLVQ
LDWENLSAAIDSYRKEKTEETRNALIEEQATYRNAIHDYFIGRTDNLTDAINKRH
AEIYKGLFKAELFNG
KVLKQLGTVTTTEHENALLRSFDKFTTYFSGFYENRKNVFSAEDISTAIPHRIVQD
NFPKFKENCHIFTR
LITAVPSLREHFENVKKAIGIFVSTSIEEVFSFPFYNQLLTQTQIDLYNQLLGGISRE
AGTEKIKGLNEV
LNLAIQKNDETAHIIASLPHRFIPLFKQILSDRNTLSFILEEFKSDEEVIQSFCKYKTL
LRNENVLETAE
ALFNELNSIDLTHIFISHKKLETISSALCDHWDTLRNALYERRISELTGKITKSAKE
KVQRSLKHEDINL
QEIISAAGKELSEAFKQKTSEILSHAHAALDQPLPTTLKKQEEKEILKSQLDSLLGL
YHLLDWFAVDESN
 
EVDPEFSARLTGIKLEMEPSLSFYNKARNYATKKPYSVEKFKLNFQMPTLASGW
DVNKEKNNGAILFVKN
GLYYLGIMPKQKGRYKALSFEPTEKTSEGFDKMYYDYFPDAAKMIPKCSTQLKA
VTAHFQTHTTPILLSN
NFIEPLEITKEIYDLNNPEKEPKKFQTAYAKKTGDQKGYREALCKWIDFTRDFLS
KYTKTTSIDLSSLRP
SSQYKDLGEYYAELNPLLYHISFQRIAEKEIMDAVETGKLYLFQIYNKDFAKGHH
GKPNLHTLYWTGLFS
PENLAKTSIKLNGQAELFYRPKSRMKRMAHRLGEKMLNKKLKDQKTPIPDTLYQ
ELYDYVNHRLSHDLSD
EARALLPNVITKEVSHEIIKDRRFTSDKFFFHVPITLNYQAANSPSKFNQRVNAYL
KEHPETPIIGIDRG
ERNLIYITVIDSTGKILEQRSLNTIQQFDYQKKLDNREKERVAARQAWSVVGTIK
DLKQGYLSQVIHEIV
DLMIHYQAVVVLENLNFGFKSKRTGIAEKAVYQQFEKMLIDKLNCLVLKDYPAE
KVGGVLNPYQLTDQFT
SFAKMGTQSGFLFYVPAPYTSKIDPLTGFVDPFVWKTIKNHESRKHFLEGFDFLH
YDVKTGDFILHFKMN
RNLSFQRGLPGFMPAWDIVFEKNETQFDAKGTPFIAGKRIVPVIENHRFTGRYRD
LYPANELIALLEEKG
IVFRDGSNILPKLLENDDSHAIDTMVALIRSVLQMRNSNAATGEDYINSPVRDLN
GVCFDSRFQNPEWPM
DADANGAYHIALKGQLLLNHLKESKDLKLQNGISNQDWLAYIQELRN
**SEQ ID NO: 73**
   NAME: gi|769142322|ref|WP_044919442.1| type V CRISPR-associated protein Cpfl
   [Lachnospiraceae bacterium MA2020]
   SEQUENCE: 
MYYESLTKQYPVSKTIRNELIPIGKTLDNIRQNNILESDVKRKQNYEHVKGILDEY
HKQLINEALDNCTL
PSLKIAAEIYLKNQKEVSDREDFNKTQDLLRKEVVEKLKAHENFTKIGKKDILDL
LEKLPSISEDDYNAL
 
ESFRNFYTYFTSYNKVRENLYSDKEKSSTVAYRLINENFPKFLDNVKSYRFVKTA
GILADGLGEEEQDSL
FIVETFNKTLTQDGIDTYNSQVGKINSSINLYNQKNQKANGFRKIPKMKMLYKQI
LSDREESFIDEFQSD
EVLIDNVESYGSVLIESLKSSKVSAFFDALRESKGKNVYVKNDLAKTAMSNIVFE
NWRTFDDLLNQEYDL
ANENKKKDDKYFEKRQKELKKNKSYSLEHLCNLSEDSCNLIENYIHQISDDIENIII
NNETFLRIVINEH
DRSRKLAKNRKAVKAIKDFLDSIKVLERELKLINSSGQELEKDLIVYSAHEELLVE
LKQVDSLYNMTRNY
LTKKPFSTEKVKLNFNRSTLLNGWDRNKETDNLGVLLLKDGKYYLGIMNTSAN
KAFVNPPVAKTEKVFKK
VDYKLLPVPNQMLPKVFFAKSNIDFYNPSSEIYSNYKKGTHKKGNMFSLEDCHN
LIDFFKESISKHEDWS
KFGFKFSDTASYNDISEFYREVEKQGYKLTYTDIDETYINDLIERNELYLFQIYNK
DFSMYSKGKLNLHT
LYFMMLFDQRNIDDVVYKLNGEAEVFYRPASISEDELIIHKAGEEIKNKNPNRAR
TKETSTFSYDIVKDK
RYSKDKFTLHIPITMNFGVDEVKRFNDAVNSAIRIDENVNVIGIDRGERNLLYVV
VIDSKGNILEQISLN
SIINKEYDIETDYHALLDEREGGRDKARKDWNTVENIRDLKAGYLSQVVNVVAK
LVLKYNAIICLEDLNF
GFKRGRQKVEKQVYQKFEKMLIDKLNYLVIDKSREQTSPKELGGALNALQLTSK
FKSFKELGKQSGVIYY
VPAYLTSKIDPTTGFANLFYMKCENVEKSKRFFDGFDFIRFNALENVFEFGFDYR
SFTQRACGINSKWTV
CTNGERIIKYRNPDKNNMFDEKVVVVTDEMKNLFEQYKIPYEDGRNVKDMIISN
EEAEFYRRLYRLLQQT
LQMRNSTSDGTRDYIISPVKNKREAYFNSELSDGSVPKDADANGAYNIARKGLW
VLEQIRQKSEGEKINL
AMTNAEWLEYAQTHLL
**SEQ ID NO: 74**
   NAME: gi|489130501|ref|WP_003040289.11 type V CRISPR-associated protein Cpfl
   [Francisella tularensis]
   SEQUENCE: 
MSIYQEFVNKYSLSKTLRFELIPQGKTLENIKARGLILDDEKRAKDYKKAKQIIDK
YHQFFIEEILSSVC
ISEDLLQNYSDVYFKLKKSDDDNLQKDFKSAKDTIKKQISEYIKDSEKFKNLFNQ
NLIDAKKGQESDLIL
WLKQSKDNGIELFKANSDITDIDEALEIIKSFKGWTTYFKGFHENRKNVYSSNDIP
TSIIYRIVDDNLPK
FLENKAKYESLKDKAPEAINYEQIKKDLAEELTFDIDYKTSEVNQRVFSLDEVFEI
ANFNNYLNQSGITK
FNTIIGGKFVNGENTKRKGINEYINLYSQQINDKTLKKYKMSVLFKQILSDTESKS
FVIDKLEDDSDVVT
TMQSFYEQIAAFKTVEEKSIKETLSLLFDDLKAQKLDLSKIYFKNDKSLTDLSQQV
FDDYSVIGTAVLEY
ITQQIAPKNLDNPSKKEQELIAKKTEKAKYLSLETIKLALEEFNKHRDIDKQCRFE
EILANFAAIPMIFD
EIAQNKDNLAQISIKYQNQGKKDLLQASAEDDVKAIKDLLDQTNNLLHKLKIFHI
SQSEDKANILDKDEH
FYLVFEECYFELANIVPLYNKIRNYITQKPYSDEKFKLNFENSTLANGWDKNKEP
DNTAILFIKDDKYYL
GVMNKKNNKIFDDKAIKENKGEGYKKIVYKLLPGANKMLPKVFFSAKSIKFYNP
SEDILRIRNHSTHTKN
GSPQKGYEKFEFNIEDCRKFIDFYKQSISKHPEWKDFGFRFSDTQRYNSIDEFYRE
VENQGYKLTFENIS
ESYIDSVVNQGKLYLFQIYNKDFSAYSKGRPNLHTLYWKALFDERNLQDVVYKL
NGEAELFYRKQSIPKK
ITHPAKEAIANKNKDNPKKESVFEYDLIKDKRFTEDKFFFHCPITINFKSSGANKF
NDEINLLLKEKAND
 
VHILSIDRGERHLAYYTLVDGKGNIIKQDTFNIIGNDRMKTNYHDKLAAIEKDRD
SARKDWKKINNIKEM
KEGYLSQVVHEIAKLVIEYNAIVVFEDLNFGFKRGRFKVEKQVYQKLEKMLIEKL
NYLVFKDNEFDKTGG
VLRAYQLTAPFETFKKMGKQTGIIYYVPAGFTSKICPVTGFVNQLYPKYESVSKS
QEFFSKFDKICYNLD
KGYFEFSFDYKNFGDKAAKGKWTIASFGSRLINFRNSDKNHNWDTREVYPTKEL
EKLLKDYSIEYGHGEC
IKAAICGESDKKFFAKLTSVLNTILQMRNSKTGTELDYLISPVADVNGNFFDSRQ
APKNMPQDADANGAY
HIGLKGLMLLGRIKNNQEGKKLNLVIKNEEYFEFVQNRNN
**SEQ ID NO: 75**
   NAME: gi|502240446|ref|WP_012739647.1| type V CRISPR-associated protein Cpfl
   [[Eubacterium] eligens]
   SEQUENCE: 
MNGNRSIVYREFVGVIPVAKTLRNELRPVGHTQEHIIQNGLIQEDELRQEKSTELK
NIMDDYYREYIDKS
LSGVTDLDFTLLFELMNLVQSSPSKDNKKALEKEQSKMREQICTHLQSDSNYKNI
FNAKLLKEILPDFIK
NYNQYDVKDKAGKLETLALFNGFSTYFTDFFEKRKNVFTKEAVSTSIAYRIVHE
NSLIFLANMTSYKKIS
EKALDEIEVIEKNNQDKMGDWELNQIFNPDFYNMVLIQSGIDFYNEICGVVNAH
MNLYCQQTKNNYNLFK
MRKLHKQILAYTSTSFEVPKMFEDDMSVYNAVNAFIDETEKGNIIGKLKDIVNKY
DELDEKRIYISKDFY
ETLSCFMSGNWNLITGCVENFYDENIHAKGKSKEEKVKKAVKEDKYKSINDVND
LVEKYIDEKERNEFKN
SNAKQYIREISNIITDTETAHLEYDDHISLIESEEKADEMKKRLDMYMNMYHWA
KAFIVDEVLDRDEMFY
SDIDDIYNILENIVPLYNRVRNYVTQKPYNSKKIKLNFQSPTLANGWSQSKEFDN
NAIILIRDNKYYLAI
 
FNAKNKPDKKIIQGNSDKKNDNDYKKMVYNLLPGANKMLPKVFLSKKGIETFK
PSDYIISGYNAHKHIKT
SENFDISFCRDLIDYFKNSIEKHAEWRKYEFKFSATDSYSDISEFYREVEMQGYRI
DWTYISEADINKLD
EEGKIYLFQIYNKDFAENSTGKENLHTMYFKNIFSEENLKDIIIKLNGQAELFYRR
ASVKNPVKHKKDSV
LVNKTYKNQLDNGDVVRIPIPDDIYNEIYKMYNGYIKESDLSEAAKEYLDKVEV
RTAQKDIVKDYRYTVD
KYFIHTPITINYKVTARNNVNDMVVKYIAQNDDIHVIGIDRGERNLIYISVIDSHG
NIVKQKSYNILNNY
DYKKKLVEKEKTREYARKNWKSIGNIKELKEGYISGVVHEIAMLIVEYNAIIAME
DLNYGFKRGRFKVER
QVYQKFESMLINKLNYFASKEKSVDEPGGLLKGYQLTYVPDNIKNLGKQCGVIF
YVPAAFTSKIDPSTGF
ISAFNFKSISTNASRKQFFMQFDEIRYCAEKDMFSFGFDYNNFDTYNITMGKTQW
TVYTNGERLQSEFNN
ARRTGKTKSINLTETIKLLLEDNEINYADGHDIRIDMEKMDEDKKSEFFAQLLSLY
KLTVQMRNSYTEAE
EQENGISYDKIISPVINDEGEFFDSDNYKESDDKECKMPKDADANGAYCIALKGL
YEVLKIKSEWTEDGF
DRNCLKLPHAEWLDFIQNKRYE
**SEQ ID NO: 76**
   NAME: gi|537834683|ref|WP_020988726.1| type V CRISPR-associated protein Cpfl
   [Leptospira inadai]
   SEQUENCE: 
MEDYSGFVNIYSIQKTLRFELKPVGKTLEHIEKKGFLKKDKIRAEDYKAVKKIIDK
YHRAYIEEVFDSVL
HQKKKKDKTRFSTQFIKEIKEFSELYYKTEKNIPDKERLEALSEKLRKMLVGAFK
GEFSEEVAEKYKNLF
SKELIRNEIEKFCETDEERKQVSNFKSFTTYFTGFHSNRQNIYSDEKKSTAIGYRII
HQNLPKFLDNLKI
 
IESIQRRFKDFPWSDLKKNLKKIDKNIKLTEYFSIDGFVNVLNQKGIDAYNTILGG
KSEESGEKIQGLNE
YINLYRQKNNIDRKNLPNVKILFKQILGDRETKSFIPEAFPDDQSVLNSITEFAKYL
KLDKKKKSIIAEL
KKFLSSFNRYELDGIYLANDNSLASISTFLFDDWSFIKKSVSFKYDESVGDPKKKI
KSPLKYEKEKEKWL
KQKYYTISFLNDAIESYSKSQDEKRVKIRLEAYFAEFKSKDDAKKQFDLLERIEEA
YAIVEPLLGAEYPR
DRNLKADKKEVGKIKDFLDSIKSLQFFLKPLLSAEIFDEKDLGFYNQLEGYYEEID
SIGHLYNKVRNYLT
GKIYSKEKFKLNFENSTLLKGWDENREVANLCVIFREDQKYYLGVMDKENNTIL
SDIPKVKPNELFYEKM
VYKLIPTPHMQLPRIIFSSDNLSIYNPSKSILKIREAKSFKEGKNFKLKDCHKFIDFY
KESISKNEDWSR
FDFKFSKTSSYENISEFYREVERQGYNLDFKKVSKFYIDSLVEDGKLYLFQIYNKD
FSIFSKGKPNLHTI
YFRSLFSKENLKDVCLKLNGEAEMFFRKKSINYDEKKKREGHHPELFEKLKYPIL
KDKRYSEDKFQFHLP
ISLNFKSKERLNFNLKVNEFLKRNKDINIIGIDRGERNLLYLVMINQKGEILKQTLL
DSMQSGKGRPEIN
YKEKLQEKEIERDKARKSWGTVENIKELKEGYLSIVIHQISKLMVENNAIVVLED
LNIGFKRGRQKVERQ
VYQKFEKMLIDKLNFLVFKENKPTEPGGVLKAYQLTDEFQSFEKLSKQTGFLFY
VPSWNTSKIDPRTGFI
DFLHPAYENIEKAKQWINKFDSIRFNSKMDWFEFTADTRKFSENLMLGKNRVW
VICTTNVERYFTSKTAN
SSIQYNSIQITEKLKELFVDIPFSNGQDLKPEILRKNDAVFFKSLLFYIKTTLSLRQN
NGKKGEEEKDFI
LSPVVDSKGRFFNSLEASDDEPKDADANGAYHIALKGLMNLLVLNETKEENLSR
PKWKIKNKDWLEFVWE
RNR
**SEQ ID NO: 77**
   NAME: gi|739008549|ref|WP_036890108.1| type V CRISPR-associated protein Cpfl
   [Porphyromonas crevioricanis]
   SEQUENCE: 
MDSLKDFTNLYPVSKTLRFELKPVGKTLENIEKAGILKEDEHRAESYRRVKKIIDT
YHKVFIDSSLENMA
KMGIENEIKAMLQSFCELYKKDHRTEGEDKALDKIRAVLRGLIVGAFTGVCGRR
ENTVQNEKYESLFKEK
LIKEILPDFVLSTEAESLPFSVEEATRSLKEFDSFTSYFAGFYENRKNIYSTKPQSTA
IAYRLIHENLPK
FIDNILVFQKIKEPIAKELEHIRADFSAGGYIKKDERLEDIFSLNYYIHVLSQAGIEK
YNALIGKIVTEG
DGEMKGLNEHINLYNQQRGREDRLPLFRPLYKQILSDREQLSYLPESFEKDEELL
RALKEFYDHIAEDIL
GRTQQLMTSISEYDLSRIYVRNDSQLTDISKKMLGDWNAIYMARERAYDHEQAP
KRITAKYERDRIKALK
GEESISLANLNSCIAFLDNVRDCRVDTYLSTLGQKEGPHGLSNLVENVFASYHEA
EQLLSFPYPEENNLI
QDKDNVVLIKNLLDNISDLQRFLKPLWGMGDEPDKDERFYGEYNYIRGALDQVI
PLYNKVRNYLTRKPYS
TRKVKLNFGNSQLLSGWDRNKEKDNSCVILRKGQNFYLAIMNNRHKRSFENKM
LPEYKEGEPYFEKMDYK
FLPDPNKMLPKVFLSKKGIEIYKPSPKLLEQYGHGTHKKGDTFSMDDLHELIDFF
KHSIEAHEDWKQFGF
KFSDTATYENVSSFYREVEDQGYKLSFRKVSESYVVSLIDQGKLYLFQIYNKDFS
PCSKGTPNLHTLYWR
MLFDERNLADVIYKLDGKAEIFFREKSLKNDHPTHPAGKPIKKKSRQKKGEESLF
EYDLVKDRRYTMDKF
QFHVPITMNFKCSAGSKVNDMVNAHIREAKDMHVIGIDRGERNLLYICVIDSRGT
ILDQISLNTINDIDY
 
HDLLESRDKDRQQEHRNWQTIEGIKELKQGYLSQAVHRIAELMVAYKAVVALE
DLNMGFKRGRQKVESSV
YQQFEKQLIDKLNYLVDKKKRPEDIGGLLRAYQFTAPFKSFKEMGKQNGFLFYIP
AWNTSNIDPTTGFVN
LFHVQYENVDKAKSFFQKFDSISYNPKKDWFEFAFDYKNFTKKAEGSRSMWILC
THGSRIKNFRNSQKNG
QWDSEEFALTEAFKSLFVRYEIDYTADLKTAIVDEKQKDFFVDLLKLFKLTVQM
RNSWKEKDLDYLISPV
AGADGRFFDTREGNKSLPKDADANGAYNIALKGLWALRQIRQTSEGGKLKLAIS
NKEWLQFVQERSYEKD
**SEQ ID NO: 78**
   NAME: gi|517171043|ref|WP_018359861.1| type V CRISPR-associated protein Cpfl
   [Porphyromonas macacae]
   SEQUENCE: 
MKTQHFFEDFTSLYSLSKTIRFELKPIGKTLENIKKNGLIRRDEQRLDDYEKLKKV
IDEYHEDFIANILS
SFSFSEEILQSYIQNLSESEARAKIEKTMRDTLAKAFSEDERYKSIFKKELVKKDIP
VWCPAYKSLCKKF
DNFTTSLVPFHENRKNLYTSNEITASIPYRIVHVNLPKFIQNIEALCELQKKMGAD
LYLEMMENLRNVWP
SFVKTPDDLCNLKTYNHLMVQSSISEYNRFVGGYSTEDGTKHQGINEWINIYRQR
NKEMRLPGLVFLHKQ
ILAKVDSSSFISDTLENDDQVFCVLRQFRKLFWNTVSSKEDDAASLKDLFCGLSG
YDPEAIYVSDAHLAT
ISKNIFDRWNYISDAIRRKTEVLMPRKKESVERYAEKISKQIKKRQSYSLAELDDL
LAHYSEESLPAGFS
LLSYFTSLGGQKYLVSDGEVILYEEGSNIWDEVLIAFRDLQVILDKDFTEKKLGK
DEEAVSVIKKALDSA
LRLRKFFDLLSGTGAEIRRDSSFYALYTDRMDKLKGLLKMYDKVRNYLTKKPYS
IEKFKLHFDNPSLLSG
 
WDKNKELNNLSVIFRQNGYYYLGIMTPKGKNLFKTLPKLGAEEMFYEKMEYKQ
IAEPMLMLPKVFFPKKT
KPAFAPDQSVVDIYNKKTFKTGQKGFNKKDLYRLIDFYKEALTVHEWKLFNFSF
SPTEQYRNIGEFFDEV
REQAYKVSMVNVPASYIDEAVENGKLYLFQIYNKDFSPYSKGIPNLHTLYWKAL
FSEQNQSRVYKLCGGG
ELFYRKASLHMQDTTVHPKGISIHKKNLNKKGETSLFNYDLVKDKRFTEDKFFF
HVPISINYKNKKITNV
NQMVRDYIAQNDDLQIIGIDRGERNLLYISRIDTRGNLLEQFSLNVIESDKGDLRT
DYQKILGDREQERL
RRRQEWKSIESIKDLKDGYMSQVVHKICNMVVEHKAIVVLENLNLSFMKGRKK
VEKSVYEKFERMLVDKL
NYLVVDKKNLSNEPGGLYAAYQLTNPLFSFEELHRYPQSGILFFVDPWNTSLTDP
STGFVNLLGRINYTN
VGDARKFFDRFNAIRYDGKGNILFDLDLSRFDVRVETQRKLWTLTTFGSRIAKSK
KSGKWMVERIENLSL
CFLELFEQFNIGYRVEKDLKKAILSQDRKEFYVRLIYLFNLMMQIRNSDGEEDYIL
SPALNEKNLQFDSR
LIEAKDLPVDADANGAYNVARKGLMVVQRIKRGDHESIHRIGRAQWLRYVQEGI
VE
**SEQ ID NO: 79**
   NAME: Integrase protein sequence found on the Uniprot site. DNA sequence was obtained from GenBank.
   SEQUENCE: 
TTTTTAGATGGAATAGATAAGGCCCAAGATGAACATGAGAAATATCACAGTA
ATTGGAGAGCAATGGCTAGTGATTTTAACCTGCCACCTGTAGTAGCAAAAGA
AATAGTAGCCAGCTGTGATAAATGTCAGCTAAAAGGAGAAGCCATGCATGGA
CAAGTAGACTGTAGTCCAGGAATATGGCAACTAGATTGTACACATTTAGAAG
GAAAAGTTATCCTGGTAGCAGTTCATGTAGCCAGTGGATATATAGAAGCAGA
AGTTATTCCAGCAGAAACAGGGCAGGAAACAGCATATTTTCTTTTAAAATTA
GCAGGAAGATGGCCAGTAAAAACAATACATACTGACAATGGCAGCAATTTCA
 
CCGGTGCTACGGTTAGGGCCGCCTGTTGGTGGGCGGGAATCAAGCAGGAATT
TGGAATTCCCTACAATCCCCAAAGTCAAGGAGTAGTAGAATCTATGAATAAA
GAATTAAAGAAAATTATAGGACAGGTAAGAGATCAGGCTGAACATCTTAAG
ACAGCAGTACAAATGGCAGTATTCATCCACAATTTTAAAAGAAAAGGGGGA
TTGGGGGGTACAGTGCAGGGGAAAGAATAGTAGACATAATAGCAACAGACA
TACAAACTAAAGAATTACAAAAACAAATTACAAAAATTCAAAATTTTCGGGT
TTATTACAGGGACAGCAGAAATCCACTTTGGAAAGGACCAGCAAAGCTCCTC
TGGAAAGGTGAAGGGGCAGTAGTAATACAAGATAATAGTGACATAAAAGTA
GTGCCAAGAAGAAAAGCAAAGATCATTAGGGATTATGGAAAACAGATGGCA
GGTGATGATTGTGTGGCAAGTAGACAGGATGAGGATTAG
**SEQ ID NO: 80**
   NAME: sp|P04585|1148-1435
   SEQUENCE: 
FLDGIDKAQDEHEKYHSNWRAMASDFNLPPVVAKEIVASCDKCQLKGEAMHGQ
VDCSPGIWQLDCTHLEGKVILVAVHVASGYIEAEVIPAETGQETAYFLLKLAGR
WPVKTIHTDNGSNFTGATVRAACWWAGIKQEFGIPYNPQSQGVVESMNKELKKI
IGQVRDQAEHLKTAVQMAVFIHNFKRKGGIGGYSAGERIVDIIATDIQTKELQKQI
TKIQNFRVYYRDSRNPLWKGPAKLLWKGEGAVVIQDNSDIKVVPRRKAKIIRDY
GKQMAGDDCVASRQDED
**SEQ ID NO: 81**
   a protein domain that characterizes zinc finger proteins
   CX(2-4)CX(12)HX(3-5)H (X(2-4) means XX or XXX or XXXX for example)
**SEQ ID NO: 82**
   >gi|1616606|emb|X97044.1| Mouse mammary tumor virus 5' LTR DNA 
ATGCCGCGCCTGCAGCAGAAATGGTTGAACTCCCGAGAGTGTCCTACACTTA
GGGGAGAAGCAGCCAAGG
GGTTGTTTCCCACCCAGAACGACCCATCTGCGCACACACGGATGAGCCCGTC
AAACAAAGACATATTCAT
TCTCTGCTGCAAACTTGGCATAGCTCTGCTTTGCCTGGGGCTATTGGGGGAAG
TTGCGGTTCATGCTCGC
 
AGGGCTCTCACCCTTGACTCTTTTAATAGCTCTTCTGTGCAAGATTACAATCT
AAACAATTCGGAGAACT
CGACCTTCCTCCTGAGGCAAGGACCACAGCCAACTTCCTCTTACAAGCCGCA
TCGATTTAGTCCTTCAGA
AATAGAAATAAGAATGCTTGCTAAAAATTATATTTTACCAATGAGACCAAT
CCAATAGGTCGATTATTA
ATTACTATGTTAAGAAATGAATCATTATCTTTTAGTACTATTTTTACTCAAATT
CAGAAGTTAGAAATGG
GAATAGAAAATAGAAAGAGACGCTCAGCCTCAGTTGAAGAACAGGTGCAAG
GACTAAGGGCCTCAGGCCT
AGAAGTAAAAAGGGGGAAGAGGAGTGCGCTTGTCAAAATAGGAGACAGGTG
GTGGCAACCAGGAACTTAT
AGGGGACCTTACATCTACAGACCAACAGACGCCCCCTTACCGTATACAGGAA
GATATGACCTAAATTTTG
ATAGGTGGGTCACAGTCAATGGCTATAAAGTGTTATACAGATCCCTCCCCTTT
CGTGAAAGGCTCGCCAG
AGCTAGACCTCCTTGGTGCGTGTTGTCTCAGGAAGAAAAAGACGACATGAAA
CAACAGGTACATGATTAT
ATTTATCTAGGAACAGGAATGAACTTTTGGAGATATTATACCAAGGAGGGGG
CAGTGGCTAGACTATTAG
AACACATTTCTGCAGATACTAATAGCATGAGTTATTATGATTAGCCTTTATTG
GCCCAATCTTGTGGTTC
CCAGGGTTCAAGTAGGTTCATGGTCACAAACTGTTCTTAAAAACAAGGATGT
GAGACAAGTGGTTTCCTG
GCTTGGTTTGGTATCAAATGTTTTGATCTGAGCTCTGAGTGTTCTGTTTTCCTA
TGTTCTTTTGGAATCT
ATCCAAGTCTTATGTAAATGCTTATGTAAACCAAAGTATAAAAGAGTGCTGA
TTTTTTGAGTAAACTTGC
AACAGTCCTAACATTCACCTCTCGTGTGTTTGTGTCTGTTCGCCATCCCGTCTC
CGCTCGTCACTTATCC
 
TTCACTTTCCAGAGGGTCCCCCGCAGACCCCGGTGACCCTCAGGTTGGCCG
ACTGCGGCA
**SEQ ID NO: 83**
   >gi|1403387|emb|X98457.1| Mouse mammary tumor virus 3' LTR 
ATGCCGCGCCTGCAGCAGAAATGGTTGAACTCCCGAGAGTGTCCTACACTTA
GGAGAGAAGCAGCCAAGG
GGTTGTTTCCCACCAAGGACGACCCGTCTGCGTGCACGCGGATGAGCCCATC
AGACAAAGACATACTCAT
TCTCTGCTGCAAACTTGGCATAGCTCTGCTTTGCCTGGGGCTATTGGGGGAAG
TTGCGGTTCGTGCTCGC
AGGGCTCTCACCCTTGATTCTTTTAATAACTCTTCTGTGCAAGATTACAATCT
AAACGATTCGGAGAACT
CGACCTTCCTCCTGGGGCAAGGACCACAGCCAACTTCCTCTTACAAGCCACA
CCGACTTTGTCCTTCAGA
AATAGAAATAAGAATGCTTGCTAAAAATTATATTTTACCAATGAGACCAAT
CCAATAGGTCGATTATTA
ATCATGATGTTTAGAAATGAATCTTTGTCTTTTAGCACTATATTTACTCAAATT
CAAAGGTTAGAAATGG
GAATAGAAAATAGAAAGAGACGCTCAACCTCAGTTGAAGAACAGGTGCAAG
GACTAAGGGCCTCAGGCCT
AGAAGTAAAAAGGGGAAAGAGGAGTGCGCTTGTCAAAATAGGAGACAGGTG
GTGGCAACCAGGGACTTAT
AGGGGACCTTACATCTACAGACCAACAGACGCCCCGCTACCATATACAGGAA
GATACGATTTAAATTTTG
ATAGGTGGGTCACAGTCAACGGCTATAAAGTGTTATACAGATCCCTCCCCCTT
CGTGAAAGACTCGCCAG
GGCTAGACCTCCTTGGTGTGTGTTAACTCAGGAAGAAAAAGACGACATGAAA
CAACAGGTACATGATTAT
ATTTATCTAGGAACAGGAATGAACTTCTGGGGAAAGATATTTGACTACACCG
AAGAGGGAGCTATAGCAA
 
AAATTATATATAATATGAAATATACTCATGGGGTCGCATTGGCTTCGATCCC
TTTTGAAACATTTATAA
ATACAATTAGGTCTACCTTGCGGTTCCCAAGGTTTAAGTAAGTTCAGGGTCAC
AAACTGTTCTTAAAACA
AGGATGTGAGACAAGTGGTTTCCTGACTTGGT
**SEQ ID NO: 84**
   >gi|119662099|emb|AM076881.1| Human immunodeficiency virus 1 proviral 5' LTR, TAR element and U3, U5 and R repeat regions, clone PG232.14 
GGCAAGAAATCCTTGATTTGTGGGTCTACTACACACAAGGCTTCTTCCCTGAT
TGGCAAAACTACACACC
GGGACCAGGGGTCAGATATCCACTGACCTTTGGATGGTGCTACAAGCTAGTG
CCAGTTGACCCAAAGGAA
GTAGAAGAGGCTAACCAAAGAGAAGACAACTGTTTGCTACACCCTATGAGCC
TGCATGGAATAGAGGACG
AAGACAGAGAAGTATTAAAGTGGCAGTTTGACAGCAGCCTAGCACGCAGAC
ACATGGCCCGCGAGCTACA
TCCAGAGTATTACAAAGACTGCTGACACAGAAAGACTTTCCGCTAGGACTT
TCCACTGAGGCGTTCCAG
GGGGAGTGGTCTAGGCAGGACTAGGAGTGGCCAACCCTCAGATGCTGCATAT
AAGCAGCTGCTTTTCGCC
TGTACTAGGTCTCTCTAGGTGGACCAGATCTGAGCCTAGGCGCTCTCTGGCTA
TCTAAGGAACCCACTGC
TTAAGCCTCAATAAAGCTTGCCTTGAGTGCTCTAAGTAGTGTGTGCCCGTCTG
TTGTGTGACTCTAGTAA
CTAGAGATCCCTCAGACCAACTTTAGTAGTGTAAAAATCTCTAGCAGTGGC
GCCCGAACAGGGACCCGA
AAGTGAAAGCAGGACCAGAGGAGATCTCTCGACGCAGGACTCGGCTTGCTGA
AAGTGCACTCGGCAAGAG
GCGAGAGCAGCGGCGACTGGTGAGTACGCCGAATTTTATTTGACTAGCGGA
GGCTAGAAGGAGAGAGAT
A
**SEQ ID NO: 85**
   >gi|1072081|gb|U37267.1|HIV1U37267 Human immunodeficiency virus type 1 3' LTR region 
ATGGGTGGCAAGTGGTCAGAAAGTAGTGTGGTTAGAAGGCATGTACCTTTAA
GACAAGGCAGCTATAGAT
CTTAGCCGCTTTTTAAAAGAAAAGGGGGGACTGGAAGGGCTAATTCACTCAC
AGAGAAGATCAGTTGAAC
CAGAAGAAGATAGAAGAGGCCATGAAGAAAACAACAGATTGTTCCGTT
TGTTCCGTTGGGGACTTTC
CAGGAGACGTGGCCTGAGTGATAAGCCGCTGGGGACTTTCCGAAGAGGCGTG
ACGGGACTTTCCAAGGCG
ACGTGGCCTGGGCGGGACTGGGGAGTGGCGAGCCCTCAGATGCTGCATATAA
GCAGCTGCTTTCTGCCTG
TACTGGGTCTCTCTGGTTAGACCAGATCTGAGCCTGGGAGCTCTCTGGCTAAC
TAGGGAACCCACTGCTT
AAGCCTCAATAAAGCTTGCCTTGAGTGCTTCAAGTAGTGTGTGCCCGTCTGTT
GTGTGACTCTGGTATCT
AGA
**THERE ARE NO SEQ ID NOS: 86-99**
**SEQ ID NO: 100**
   Oligo for insertion of neo into a cell's genome (using full sequences of 5' and 3' HIV LTRs 
GACAAGACATCCTTGATTTGTGGGTCTATAACACACAAGGCTTCTTCCCTGAT
TGGCAAAACTACACACC
GGGACCAGGGACCAGATACCCACTGACCTTTGGATGGTGCTTCAAGCTAGTG
CCAGTTGACCCAAGGAA
GTAGAAGAGGCCAATACAGGGGAAAACAACTGTTTGCTCCACCCTATGAGCC
AGCATGGAATGGAAGATG
ACCATAGAGAAGTATTAAAGTGGAAGTTTGACAGTATGCTAGCACGCAGACA
CCTGGCCCGCGAGCTACA
 
TCCGGAGTACTACAAAAACTGCTGACATGGAGGGACTTTCCGCTGGGACTTT
CCATTGGGGCGTTCCAGG
AGGTGTGGTCTGGGCGGACAAGGGAGTGGTCAACCCTCAGATGCTGCATAT
AAGCAGCTGCTTTTCGCT
TGTACTGGGTCTCTTTAGGTAGACCAGATCTGAGCCTGGGAGCTCTCTGGCTA
CCTGAGGAACCCACTGC
TTAAGCCTCAATAAAGCTTGCCTTGAGTGCTCTAAGTAGTGTGTGCCCGTCTG
TTGTGTGACTCTGGTAA
CTAGAGATCCCTCAGACCCTTTTGGTAGTGTGGAAAATCTCTAGCAGATGATT
GAACAAGATGGATTGCAC
GCAGGTTCTCCGGCCGCTTGGGTGGAGAGGCTATTCGGCTATGACTGGGCAC
AACATGGGTGGCAAGTGGTCAG
AAAGTAGTGTGGTTAGAAGGCATGTACCTTTAAGACAAGGCAGCTATAG
ATCTTAGCCGCTTTTTAAAAGAAAAG
GGGGGACTGGAAGGGCTAATTCACTCACAGAAGATCAGTTGAACCAG
AAGAAGATAGAAGAGGCCATGAAG
AAGAAAACAACAGATTGTTCCGTTTGTTCCGTTGGGGACTTTCCAGGAG
ACGTGGCCTGAGTGATAAGCCGCTGGG
GACTTTCCGAAGAGGCGTGACGGGACTTTCCAAGGCGACGTGGCCTGGG
CGGGACTGGGGAGTGGCGAGCCCTC
AGATGCTGCATATAAGCAGCTGCTTTCTGCCTGTACTGGGTCTCTCTGGT
TAGACCAGATCTGAGCCTGGGAGCTCT
CTGGCTAACTAGGGAACCCACTGCTTAAGCCTCAATAAAGCTTGCCTTG
AGTGCTTCAAGTAGTGTGTGCCCGTCTG
TTGTGTGACTCTGGTATCTAGA
 First 5'LTR is underlined, plain text is neo, and 3'LTR is **bolded** (1179 bp)
**SEQ ID NO: 101**
   An abbreviated version of 5'LTR and 3'LTR with neo sequence within (224 bp) First 5'LTR is underlined, plain text is neo, and 3'LTR is **bolded** 
GACAAGACATCCTTGATTTGTGGGTCTATAACACACAAGGCTTCTTCCCTGAT
TGGCAAAACTACACACCATGATTGAACAAGATGGATTGCAC
 
GCAGGTTCTCCGGCCGCTTGGGTGGAGAGGCTATTCGGCTATGACTGGGCAC
AACTTAAGCCTCAATAAAGCTTGCCTTGAGTGCTTCAAGTAGTGTGTGCC
CGTCTG
TTGTGTGACTCTGGTATCTAGA
**Regarding SEQ ID NO: 72**
   Genbank Protein ID: WP_021736722.1
   NCBI protein GI from NR Database or local GI (for proteins originated from WGS database): 545612232
   Contig ID in WGS database: AWUR01000016.1
   Contig description: Acidaminococcus sp. BV3L6 contig00028, whole genome shotgun sequence
   Protein completeness: Complete
   Proteins analyzed experimentally: 8
   Non-redundant set: nr
   Organism: Acidaminococcus_sp_BV3L6
   Taxonomy:
      Bacteria, Firmicutes,Negativicutes,Selenomonadales, Acidaminococcaceae, Acidaminococ cus,Acidaminococcus sp. BV3L6
**Regarding SEQ ID NO: 73**
   Genbank Protein ID: WP_044919442.1
   NCBI protein GI from NR Database or local GI (for proteins originated from WGS database): 769142322
   Contig ID in WGS database: JQKK01000008.1
   Contig description: Lachnospiraceae bacterium MA2020
   T348DRAFT_scaffold00007.7_C, whole genome shotgun sequence
   Protein completeness: Complete
   Proteins analyzed experimentally: 9
   Non-redundant set: nr
   Organism: Lachnospiraceae_bacterium_MA2020
   Taxonomy: Bacteria,Firmicutes,Clostridia,Clostridiales,Lachnospiraceae,unclassified Lachnospiraceae,Lachnospiraceae bacterium MA2020
   **Additional nucleic acid sequences and protein sequences that can be used in the disclosed compositions and methods - CPF 1 alignment.** SEQ ID NOS: 86-92; in order from the top to the bottom of the chart. 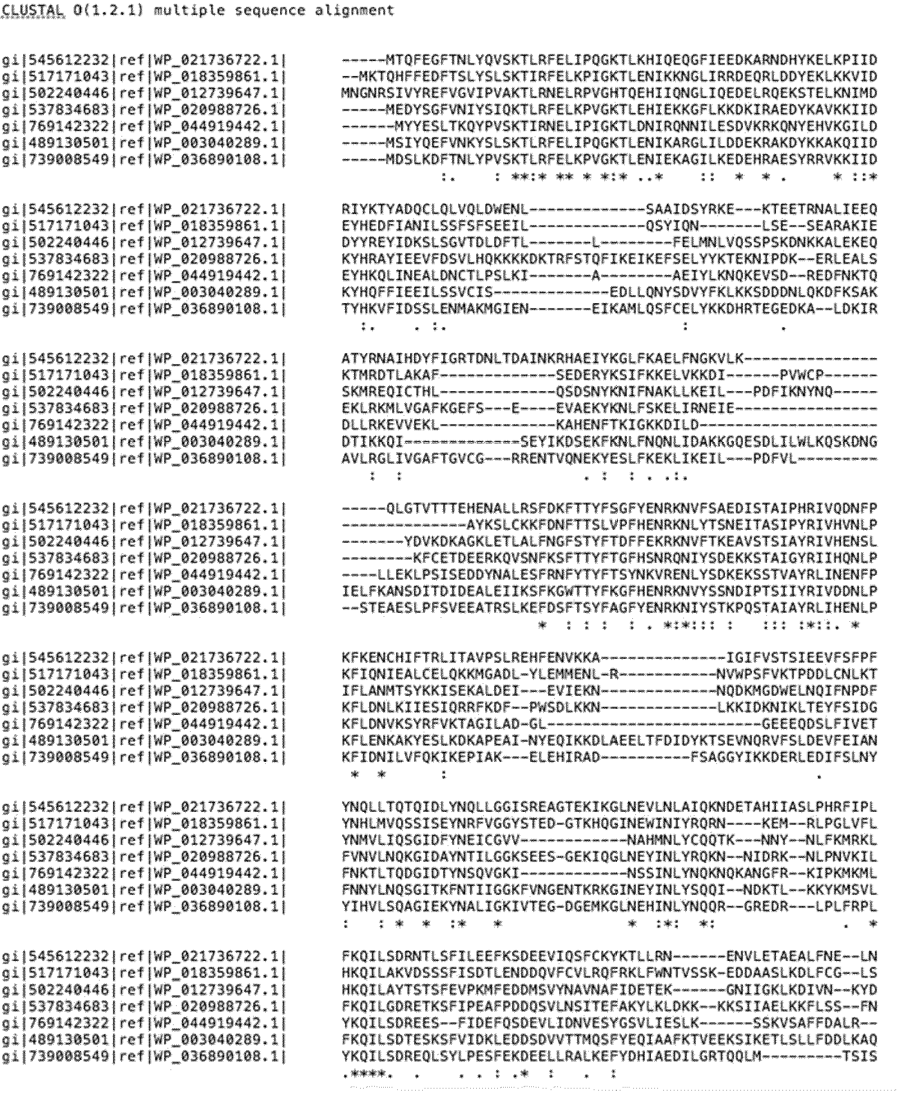 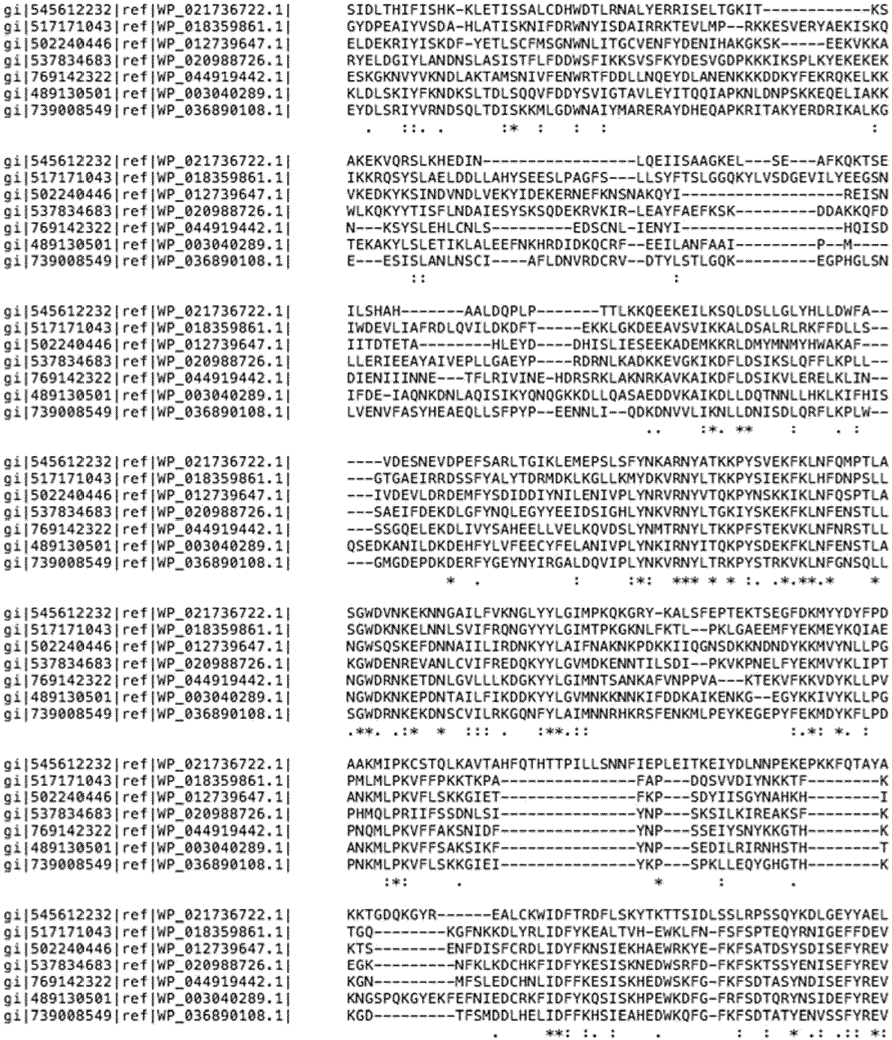 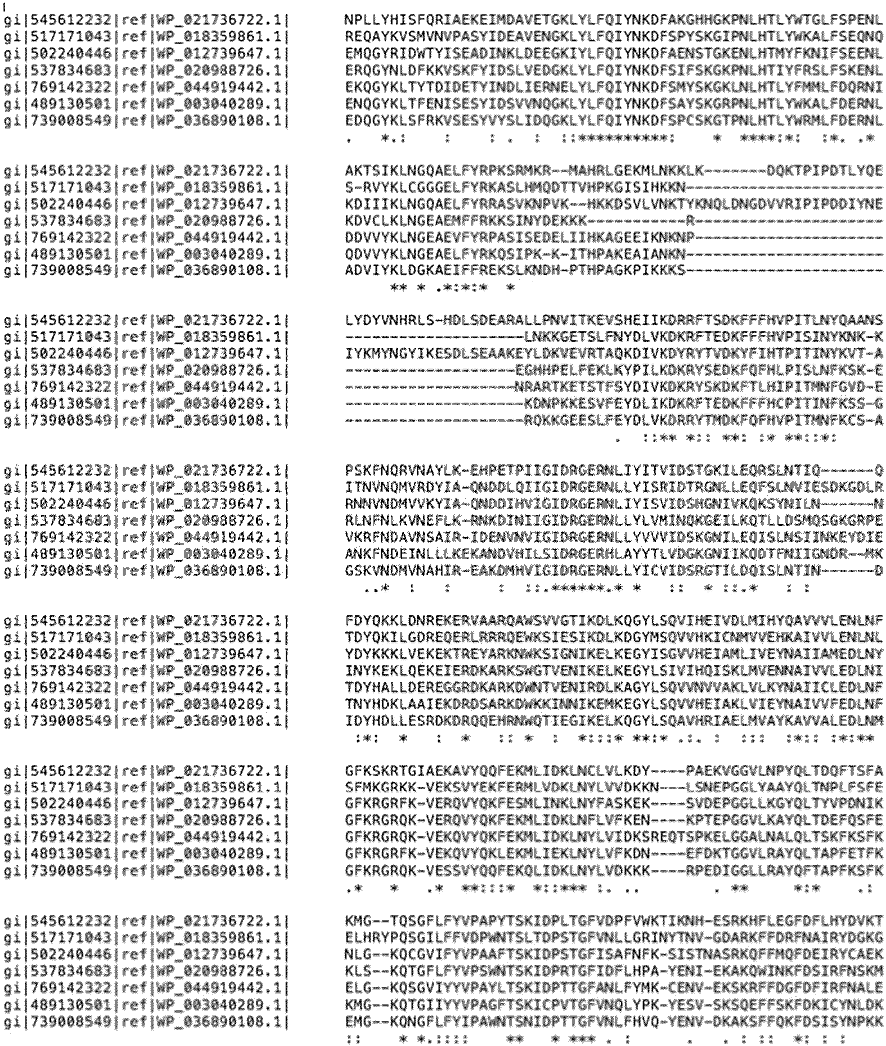 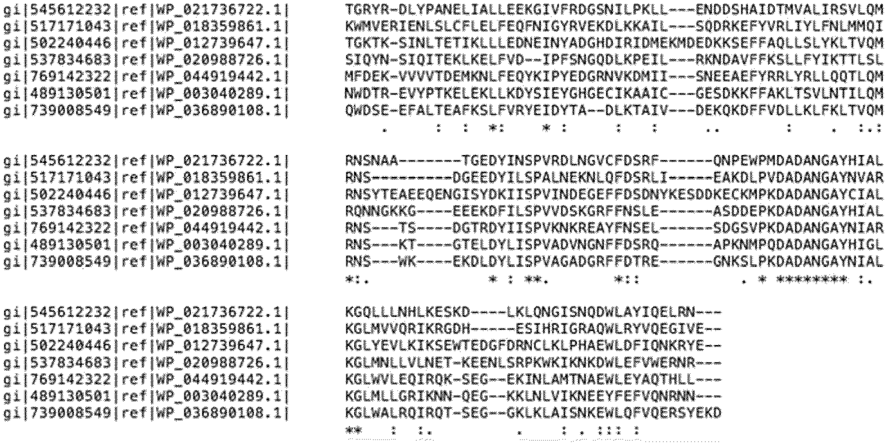
   **Additional nucleic acid sequences and protein sequences that can be used in the disclosed compositions and methods - Cfp1 human cleaving proteins alignment. SEQ** ID NO: 86 (first row) and SEQ ID NO: 90 (second row). 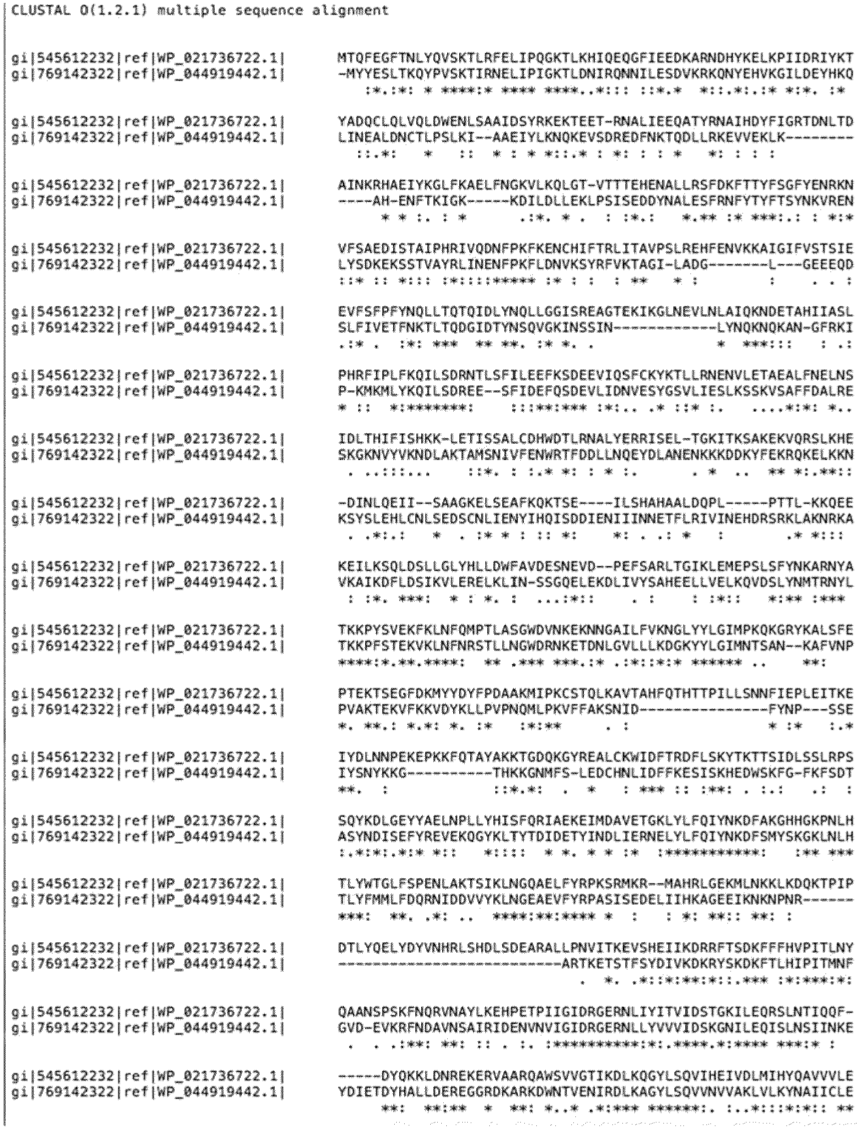 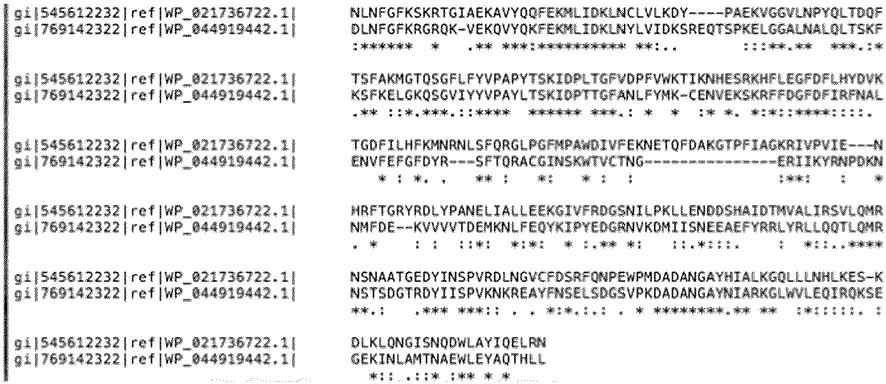
   **Additional nucleic acid sequences and protein sequences that can be used in the disclosed compositions and methods.** Table taken from Haft, D., et al. PLoS Computational Biology, November 2005, Vol. 1, Issue 6, pp. 474-483. SEQ ID NOS: 200-253; in order from the top to the bottom of the chart. 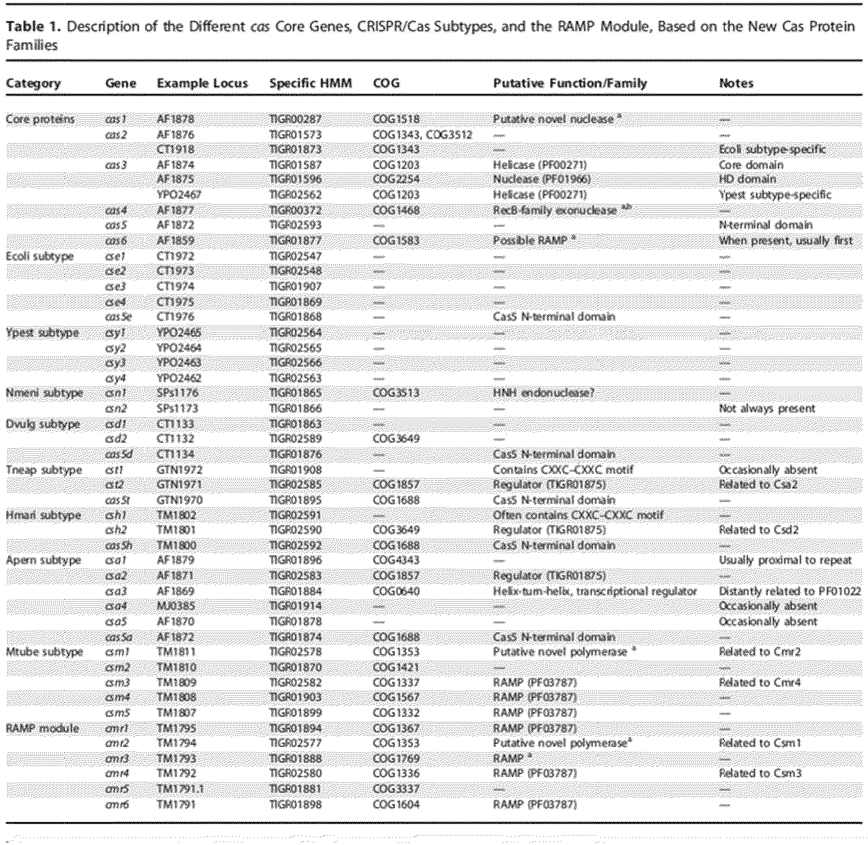 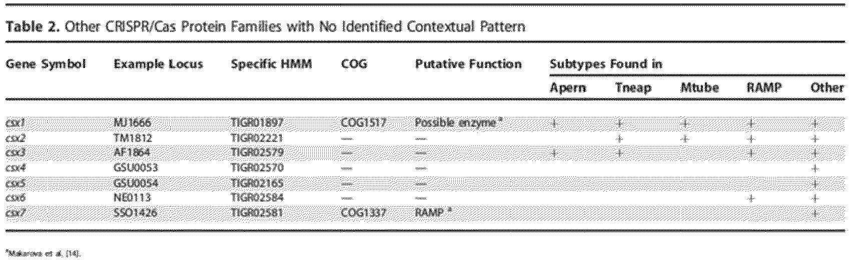
   Editing target sequences and PAMs for Nrf2 (exon 2): Used for sgRNA design 1-3
**SEQ ID NO: 254**
   GCGACGGAAAGAGTATGAGC TGG
**SEQ ID NO: 255**
   TATTTGACTTCAGTCAGCGA CGG
**SEQ ID NO: 256**
   TGGAGGCAAGATATAGATCT TGG
   Primer Key for Detection of Integration at Nrf2 Target
   Primer Set 1:
      **SEQ ID NO: 257**
         Primer 1:5'-GTGTTAATTTCAAACATCAGCAGC-3',
      **SEQ ID NO: 258**
         Primer 2: 5'- GACAAGACATCCTTGATTTG-3'
   Primer Set 2:
      **SEQ ID NO: 259**
         Primer 1:5'-GAGGTTGACTGTGTAAATG-3',
      **SEQ ID NO: 260**
         Primer 2: 5'- GATACCAGAGTCACACAACAG-3'
   Primer Set 3:
      **SEQ ID NO: 261**
         Primer 1: 5'-TCTACATTAATTCTCTTGTGC-3',
      **SEQ ID NO: 262**
         Primer 2:5'- GATACCAGAGTCACACAACAG-3'

      Accession number for human CXCR4
      Uniprot P61073
      Ensembl gene ID: ENSG00000121966
      Editing target sequence and PAM for CXCR4 (Exon 2): Used for sgRNA design1
**SEQ ID NO: 263**
   GGGCAATGGATTGGTCATCC TGG
   Primer Key for Detection of Integration at CXCR4 Target
   Primer Set 1:
      **SEQ ID NO: 264**
         Primer 1: 5'- TCTACATTAATTCTCTTGTGC-3',
      **SEQ ID NO: 265**
         Primer 2: 5'- GACAAGACATCCTTGATTTG-3'
   Primer Set 2:
      **SEQ ID NO: 266**
         Primer 1: 5'- TCTACATTAATTCTCTTGTGC-3',
      **SEQ ID NO: 267**
         Primer 2: 5'- GATACCAGAGTCACACAACAG -3'
   Primer Set 3:
      **SEQ ID NO: 268**
         Primer 1: 5'- GAGGTTGACTGTGTAAATG -3',
      **SEQ ID NO: 269**
         Primer 2: 5'- GACAAGACATCCTTGATTTG-3'
   Primer Set 4:
      **SEQ ID NO: 270**
         Primer 1: 5'- GAGGTTGACTGTGTAAATG -3',
      **SEQ ID NO: 271**
         Primer 2: 5'- GATACCAGAGTCACACAACAG -3'
         Avi-tagged Cas9 for biotinylation
         Sequence of the avi-tag used for Cas9 biotinylation
         Amino acid seqeunce:
            **SEQ ID NO: 272**
            G G D L E G S G L N D I F E A Q K I E W H E *
         Nucleic acid sequence:
            **SEQ ID NO: 273**

GGCGGCGACCTCGAGGGTAGCGGTCTGAACGATATTTTTGAAGCGCAGAAAA
TTGAATGGCATGAATAA

## Claims

1. A system for insertion of a donor nucleic acid into genomic DNA, comprising:
(a) a fusion protein comprising:
i) a first protein comprising a catalytically inactive Cas9 protein;
ii) a second protein comprising a retroviral integrase, and
iii) a linker linking the first protein to the second protein;
(b) a guide RNA (gRNA); and
(c) a DNA vector comprising: the donor DNA, a first retroviral long terminal repeat (rLTR) and a second rLTR, wherein the donor DNA is located between the first rLTR and second rLTR.

2. The system of claim 1, wherein the first protein is encoded by a polynucleotide having at least 80%, at least 85%, at least 90%, at least 95%, or at least 99% sequence identity to SEQ ID NO:56; or comprises an amino acid sequence having at least 80%, at least 85%, at least 90%, at least 95%, or at least 99% sequence identity to SEQ ID NO: 52.

3. The system of claim 1 or 2, wherein the first protein comprises the amino acid sequence of SEQ ID NO:52.

4. The system of any one of claims 1-3, wherein the second protein is encoded by a polynucleotide having at least 80%, at least 85%, at least 90%, at least 95%, or at least 99% sequence identity to any one of SEQ ID NOs: 15, 17, 47, 62, or 70; and/or comprises at least 85%, at least 90%, at least 95%, or at least 99% sequence identity to any one of SEQ ID NOs: 16, 18, 48, 63, or 71.

5. The system of any one of claims 1-4, wherein the retroviral integrase is an HIV1 integrase or a lentiviral integrase.

6. The system of any one of claims 1-5, wherein the retroviral integrase is an HIV1 integrase.

7. The system of any one of claims 1-6, wherein the second protein comprises the amino acid sequence of SEQ ID NO:71.

8. The system of any one of claims 1-7, wherein the linker is 4-8 amino acids in length.

9. The system of any one of claims 1-6, wherein the gRNA targets a target DNA sequence having a length from 16 to 24 base pairs.

10. The system of any one of claims 1-6, wherein the first or second rLTR comprises a nucleotide sequence selected from SEQ ID NO: 59 and 60.

11. An *in vitro* method of inserting a DNA sequence into genomic DNA, comprising:
a) identifying a target sequence in the genomic DNA;
b) designing a fusion protein and guide RNA (gRNA) as defined in any one of claims 1 to 10 to bind to the target sequence in the genomic DNA;
c) designing a DNA sequence of interest to incorporate into the genomic DNA; and
d) providing the fusion protein, the gRNA, and the DNA sequence of interest to a cell by techniques that allow for entry of the fusion protein, gRNA and DNA sequence of interest into the cell; wherein the DNA sequence of interest becomes integrated at the target sequence in the genomic DNA.

12. An *in vitro* method of inhibiting gene transcription in a cell, comprising:
a) identifying an ATG start codon in a gene;
b) designing a fusion protein system with a fusion protein as defined in any one of claims 1 to 10 and a guide RNA (gRNA) to bind to a target sequence immediately after the ATG start codon of the gene;
c) designing a DNA sequence of interest that is one or more consecutive stop codons; and
d) providing the fusion protein, the gRNA, and the DNA sequence of interest to a cell by techniques that allow for entry of the fusion protein, gRNA and DNA sequence of interest into the cell; wherein the DNA sequence of interest becomes integrated at the target sequence in the genomic DNA; and wherein transcription of the gene is inhibited.

## Patentansprüche

1. System zum Einsetzen einer Spendernukleinsäure in genomische DNA, umfassend:
(a) ein Fusionsprotein, umfassend:
i) ein erstes Protein, das ein katalytisch inaktives Cas9-Protein umfasst;
ii) ein zweites Protein, das eine retrovirale Integrase umfasst, und
iii) einen Linker, der das erste Protein mit dem zweiten Protein verbindet;
(b) eine Leit-RNA (gRNA); und
(c) einen DNA-Vektor, umfassend: die Spender-DNA, eine erste retrovirale lange terminale Wiederholung (rLTR) und eine zweite rLTR, wobei sich die Spender-DNA zwischen der ersten rLTR und der zweiten rLTR befindet.

2. System nach Anspruch 1, wobei das erste Protein durch ein Polynukleotid kodiert wird, das wenigstens 80 %, wenigstens 85 %, wenigstens 90 %, wenigstens 95 % oder wenigstens 99 % Sequenzidentität mit SEQ ID NO: 56 aufweist; oder eine Aminosäuresequenz mit wenigstens 80 %, wenigstens 85 %, wenigstens 90 %, wenigstens 95 %, oder wenigstens 99 % Sequenzidentität mit SEQ ID NO: 52 umfasst.

3. System nach Anspruch 1 oder 2, wobei das erste Protein die Aminosäuresequenz von SEQ ID NO: 52 umfasst.

4. System nach einem der Ansprüche 1-3, wobei das zweite Protein durch ein Polynukleotid kodiert wird, das wenigstens 80 %, wenigstens 85 %, wenigstens 90 %, wenigstens 95 % oder wenigstens 99 % Sequenzidentität mit SEQ ID NO: 15, 17, 47, 62 oder 70 aufweist; und/oder wenigstens 80 %, wenigstens 85 %, wenigstens 90 %, wenigstens 95 %, oder wenigstens 99 % Sequenzidentität mit einer von SEQ ID NO: 16, 18, 48, 63 oder 71 umfasst.

5. System nach einem der Ansprüche 1-4, wobei die retrovirale Integrase eine HIV1-Integrase oder eine lentivirale Integrase ist.

6. System nach einem der Ansprüche 1-5, wobei die retrovirale Integrase eine HIV1-Integrase ist.

7. System nach einem der Ansprüche 1-6, wobei das zweite Protein die Aminosäuresequenz von SEQ ID NO: 71 umfasst.

8. System nach einem der Ansprüche 1-7, wobei der Linker 4-8 Aminosäuren lang ist.

9. System nach einem der Ansprüche 1-6, wobei die gRNA auf eine Ziel-DNA-Sequenz mit einer Länge von 16 bis 24 Basenpaaren abzielt.

10. System nach einem der Ansprüche 1-6, wobei die erste oder zweite rLTR eine Nukleotidsequenz umfasst, die aus SEQ ID NO: 59 und 60 ausgewählt ist.

11. In-vitro-Verfahren zum Einfügen einer DNA-Sequenz in genomische DNA, umfassend:
a) Identifizieren einer Zielsequenz in der genomischen DNA;
b) Gestalten eines Fusionsproteins und einer Leit-RNA (gRNA), wie in einem der Ansprüche 1 bis 10 definiert, um an die Zielsequenz in der genomischen DNA zu binden;
c) Gestalten einer DNA-Sequenz von Interesse zum Einbauen in die genomische DNA; und
d) Bereitstellen des Fusionsproteins, der gRNA und der DNA-Sequenz von Interesse an eine Zelle durch Techniken, die den Eintritt des Fusionsproteins, der gRNA und der DNA-Sequenz von Interesse in die Zelle ermöglichen; wobei die DNA-Sequenz von Interesse an der Zielsequenz in der genomischen DNA integriert wird.

12. *In*-*vitro-*Verfahren zum Hemmen der Gentranskription in einer Zelle, umfassend:
a) Identifizieren eines ATG-Startcodons in einem Gen;
b) Gestalten eines Fusionsproteinsystems mit einem Fusionsprotein, wie es in einem der Ansprüche 1 bis 10 definiert ist, und einer Leit-RNA (gRNA), um an eine Zielsequenz unmittelbar nach dem ATG-Startcodon des Gens zu binden;
c) Gestalten einer DNA-Sequenz von Interesse, die ein oder mehrere aufeinanderfolgende Stoppcodons ist; und
d) Bereitstellen des Fusionsproteins, der gRNA und der DNA-Sequenz von Interesse für eine Zelle durch Techniken, die den Eintritt des Fusionsproteins, der gRNA und der DNA-Sequenz von Interesse in die Zelle ermöglichen; wobei die DNA-Sequenz von Interesse an der Zielsequenz in der genomischen DNA integriert wird; und wobei die Transkription des Gens gehemmt wird.

## Revendications

1. Système pour insertion d'un acide nucléique donneur dans un ADN génomique, comprenant :
(a) une protéine de fusion comprenant :
i) une première protéine comprenant une protéine Cas9 catalytiquement inactive ;
ii) une seconde protéine comprenant une intégrase rétrovirale, et
iii) un lieur liant la première protéine à la seconde protéine ;
(b) un ARN guide (gARN) ; et
(c) un vecteur ADN comprenant : l'ADN donneur, une première répétition terminale longue rétrovirale (rLTR) et une seconde rLTR, dans lequel l'ADN donneur est situé entre les première rLTR et seconde rLTR.

2. Système de la revendication 1, dans lequel la première protéine est codée par un polynucléotide ayant au moins 80 %, au moins 85 %, au moins 90 %, au moins 95 %, ou au moins 99 % d'identité de séquence avec SEQ ID n° : 56 ; ou comprend une séquence d'acides aminés ayant au moins 80 %, au moins 85 %, au moins 90 %, au moins 95 %, ou au moins 99 % d'identité de séquence avec SEQ ID n° : 52.

3. Système de la revendication 1 ou 2, dans lequel la première protéine comprend la séquence d'acides aminés de SEQ ID n° : 52.

4. Système de l'une quelconque des revendications 1 à 3, dans lequel la seconde protéine est codée par un polynucléotide ayant au moins 80 %, au moins 85 %, au moins 90 %, au moins 95 %, ou au moins 99 % d'identité de séquence avec l'un quelconque de SEQ ID n° : 15, 17, 47, 62, ou 70 ; et/ou comprend au moins 85 %, au moins 90 %, au moins 95 %, ou au moins 99 % d'identité de séquence avec l'un quelconque de SEQ ID n° : 16, 18, 48, 63, ou 71.

5. Système de l'une quelconque des revendications 1 à 4, dans lequel l'intégrase rétrovirale est une intégrase de VIH1 ou une intégrase lentivirale.

6. Système de l'une quelconque des revendications 1 à 5, dans lequel l'intégrase rétrovirale est une intégrase de VIH1.

7. Système de l'une quelconque des revendications 1 à 6, dans lequel la seconde protéine comprend la séquence d'acides aminés de SEQ ID n° : 71.

8. Système de l'une quelconque des revendications 1 à 7, dans lequel la longueur du lieur est de 4 à 8 acides aminés.

9. Système de l'une quelconque des revendications 1 à 6, dans lequel le gARN cible une séquence ADN cible ayant une longueur allant de 16 à 24 paires de bases.

10. Système de l'une quelconque des revendications 1 à 6, dans lequel la première ou seconde rLTR comprend une séquence nucléotidique sélectionnée parmi SEQ ID n° : 59 et 60.

11. Procédé *in vitro* d'insertion d'une séquence ADN dans un ADN génomique, comprenant :
a) l'identification d'une séquence cible dans l'ADN génomique ;
b) la désignation d'une protéine de fusion et d'un ARN guide (gARN) tels que définis dans l'une quelconque des revendications 1 à 10 à lier à la séquence cible dans l'ADN génomique ;
c) la désignation d'une séquence ADN d'intérêt à incorporer dans l'ADN génomique ; et
d) la fourniture de la protéine de fusion, du gARN, et de la séquence ADN d'intérêt à une cellule par l'intermédiaire de techniques qui permettent l'entrée de la protéine de fusion, du gARN, et de la séquence ADN d'intérêt dans la cellule ; dans lequel la séquence ADN d'intérêt devient intégrée au niveau de la séquence cible dans l'ADN génomique.

12. Procédé *in vitro* d'inhibition de transcription de gène dans une cellule, comprenant :
a) l'identification d'un codon d'initiation ATG dans un gène ;
b) la désignation d'un système protéine de fusion avec une protéine de fusion telle que définie dans l'une quelconque des revendications 1 à 10 et un ARN guide (gARN) à lier à une séquence cible immédiatement après le codon d'initiation ATG du gène ;
c) la désignation d'une séquence ADN d'intérêt qui est un ou plusieurs codons d'arrêt consécutifs ; et
d) la fourniture de la protéine de fusion, du gARN, et de la séquence ADN d'intérêt à une cellule par l'intermédiaire de techniques qui permettent l'entrée de la protéine de fusion, du gARN, et de la séquence ADN d'intérêt dans la cellule ; dans lequel la séquence ADN d'intérêt devient intégrée au niveau de la séquence cible dans l'ADN génomique ; et dans lequel la transcription du gène est inhibée.
